# EUROPÄISCHE PATENTSCHRIFT

(11) **EP 3 806 786 B1**
(45) Veröffentlichungstag und Bekanntmachung des Hinweises auf die Patenterteilung: **07.06.2023**
(21) Anmeldenummer: 19731212.7
(22) Anmeldetag: 12.06.2019
(51) Int. Cl.: A61F 5/56, A61C 7/08, A61C 7/36, A61C 19/045

(54) **UNTERKIEFERPROTRUSIONSEINRICHTUNG**
MANDIBULAR PROTRUSION DEVICE
DISPOSITIF DE PROTRUSION DE LA MÂCHOIRE INFÉRIEURE

(30) Priorität: 13.06.2018 DE 102018114103
(43) Veröffentlichungstag der Anmeldung: 21.04.2021
(73) Patentinhaber: Schlieper, Jörg, 24558 Henstedt-Ulzburg (DE)
(72) Erfinder: Schlieper, Jörg, 24558 Henstedt-Ulzburg (DE)
(74) Vertreter: Schmitt-Nilson Schraud Waibel Wohlfrom Patentanwälte Partnerschaft mbB
(86) Internationale Anmeldenummer: PCT/EP2019/065329
(87) Internationale Veröffentlichungsnummer: WO 2019/238744

(56) Entgegenhaltungen:
- WO-A1-00/01317
- DE-A1-102013 100 898
- DE-A1-102014 117 080
- DE-U1-202018 103 323

## Beschreibung

Die Erfindung betrifft eine Unterkieferprotrusionseinrichtung für eine therapeutische Unterkieferprotrusion bei einem zu therapierenden Patienten, sowie ein Verfahren zu Herstellung einer solchen Unterkieferprotrusionseinrichtung, wie in den Ansprüchen definiert.

Solche Unterkieferprotrusionseinrichtungen sind auch unter der Bezeichnung orofaciale Gebissschienen oder Unterkieferprotrusionsschienen, abgekürzt UPS, bekannt.

Mit einer derartigen Unterkieferprotrusionsschiene lässt sich bei einem diese tragenden Patienten eine Unterkiefervorverlagerung vorbestimmten Ausmaßes gegenüber dem Oberkiefer bewirken. Mit einer solchen Unterkiefervorverlagerung kann man dem primären Schnarchen und dem obstruktiven Schlafapnoesyndrom (OSAS) entgegenwirken.

Im Folgenden werden in der Anatomie und in der Zahnmedizin verwendete Begriffe zur Bezeichnung von Körperebenen, wie z. B. Sagittalebene und Medianebene, und Richtungen, wie beispielsweise caudal, cranial oder dorsal, lateral etc., benutzt, deren Bedeutung man nachsehen kann bei https://de.wikipedia.org/wiki/Anatomische_Lage-und_Richtungsbezeichnungen#Weitere_allgemeine_Lage-_und_Richtungsbezeichnungen.

Eine Zusammenstellung anatomischer Richtungsangaben findet man auch bei https://www.cobocards.com/pool/de/card/9loej1211/online-karteikarten-wasbedeuten-dorsal-ventral-kaudal-kranial-rostral-okzipital-frontal-temporal-late-/. Wie in der Anatomie und Zahnheilkunde üblich werden auch hier nachfolgend die Begriffe rechts und links immer aus der Sicht des Patienten betrachtet und nicht aus der Sicht eines auf den Patienten oder auf Patienten-Abbildungen oder -Darstellungen schauenden Betrachters.

Das Problem des primären Schnarchens, d. h. Schnarchens ohne relevante Atemaussetzer, und des obstruktiven Schlafapnoesyndroms, bei dem längere, wiederholte Atemaussetzer auftreten, ist mit seinen gesundheitsschädlichen Folgen für den menschlichen Körper und mit seinen störenden Auswirkungen auf die Mitmenschen hinlänglich bekannt.

Ursächlich für das Schnarchen und die Atemaussetzer ist meistens eine mehr oder weniger stark ausgeprägte Einengung im hinteren Rachenbereich zwischen dem weichen Gaumen, der Rachenhinterwand und der Zunge, bis hin zum vollständigen Verschluss. Das Schnarchgeräusch entsteht beim unvollständigen Verschluss während des Ein- und Ausatmens und wird meistens verursacht durch eine Vibration des weichen Gaumens bei zurückliegender oder zu großer Zunge während des Schlafes. Bei vollständigem Verschluss des hinteren Rachenbereichs kommt es zu einem Sistieren der Atmung, das heißt, Atemaussetzern, mit unterschiedlicher Frequenz und Dauer.

Als Abhilfe haben sich UPS in der medizinischen Therapie seit einigen Jahren bewährt. Mittels solcher UPS wird der Unterkiefer nach vorne geschoben in eine therapeutische Position bei ca. 70% des maximal möglichen Unterkiefervorschubs. Bedingt durch die Bauhöhe der Schienen zwischen den Kauflächen der sich gegenüber stehenden Zahnkronen wird der Unterkiefer auch zwangsläufig geöffnet (Bisssperrung). Durch den Vorschub (Protrusion) des Unterkiefers wird der hintere Rachenraum erweitert und das Absinken der Zunge in den hinteren Rachenraum, das dem Effekt der Rachenraumerweiterung entgegenwirken würde, verhindert. Derartige UPS finden ihren Halt über die Ober- und Unterkieferzähne, deren Kronen sie ganz oder teilweise körperlich einfassen und an denen sie über Eigenfriktion infolge von geeigneter Klemmpassung gegen Abzugskräfte, die durch den Vorschub und die Mundöffnungsbewegungen entstehen, stabilisiert werden.

Eine patientenindividuelle Anpassung solcher UPS unter physiologischen Gesichtspunkten ist aus zweierlei Hinsicht unverzichtbar:
Erstens um negative Wirkungen auf den menschlichen Körper, insbesondere auf die Kiefergelenke und die stomatognathe (Mund und Kiefer betreffende) Muskulatur, vermeiden zu können. Eine unphysiologische Belastung des stomathognathen Systems führt unweigerlich über die zumeist lebenslange Tragedauer derartiger Schienen zu unerwünschten Wirkungen.

Zweitens um die Verträglichkeit dieser Therapieform zu optimieren. Denn nur bei optimaler Verträglichkeit ist dem Patienten der tägliche und lebenslange Gebrauch der Gebissschiene zumutbar und nur, wenn die beim Schlafen eingesetzte Gebissschiene wirken kann, ist das Erreichen des gewünschten medizinischen Ergebnisses möglich. Am stärksten wird die Verträglichkeit durch den Tragekomfort beeinflusst. Der Tragekomfort der Gebissschiene ist wiederum umso größer, je mehr die Gebissschiene die Physiognomie des stomatognathen Systems berücksichtigt.

UPS werden üblicherweise patientenindividuell nach Abdrücken in zahntechnischen Fachlaboren angefertigt. Während der Herstellung der UPS ist nicht absehbar, welche Vorschubeinstellung des Unterkiefers beim Patienten bei minimaler Mundöffnung und damit Unterkieferöffnung zu einem maximalen therapeutischen Effekt bei gleichzeitig minimalen Nebenwirkungen führt. Während der UPS-Herstellung kann der Vorschub der UPS nur in Annäherung an einen therapeutisch optimalen Wert voreingestellt werden und erfordert im Laufe der Behandlung eine Titrierung, also eine stufenweise Anpassung des Vorschubs bis zum gewünschten Wirkungsergebnis der UPS.

Bekannt sind einteilige monoblockartige UPS und zweiteilige einstellbare UPS.

Bei einteiligen UPS sind die Oberkieferschiene und die Unterkieferschiene fest miteinander verbunden. Für eine Änderung der Vorschubeinstellung ist eine zeit- und kostenaufwendige labortechnische Umarbeitung mit Trennung der oberen und unteren Schienenbereiche erforderlich. Der Patient muss zum Einsetzen der einteiligen UPS in den geöffneten Mund einen Unterkiefervorschub entsprechend der therapeutischen Unterkieferprotrusion, für welche die einteilige UPS gestaltet ist, einnehmen, um die Zähne in die richtige relative Einbissstellung zur Schiene zu bringen. Dies ist besonders bei Patienten mit Koordinationsstörungen, wie z. B. bei M. Parkinson, deutlich erschwert bis unmöglich.

Bei zweiteiligen UPS-Systemen, bei welchen die Oberkieferschiene und die Unterkieferschiene als zwei separate Teile ausgebildet sind, deren Vorschub durch dazwischen wirkende Eingriffselemente variabel einstellbar ist, entfällt das Erfordernis einer Umarbeitung, um von einer anfänglichen Voreinstellung aus schrittweise zu einer therapeutisch optimalen Vorschubeinstellung zu gelangen. Zur Einstellung des therapeutischen Vorschubs ist eine Änderung der Einstellung der Eingriffselemente ausreichend.

Zweiteilige UPS-Systeme stellen heute international den medizinischen Standard für Unterkieferprotrusionsschienen dar, die in einer Langzeittherapie für eine lebenslange Anwendung geeignet sind. (DGZS Positionspapier 2009, www.dgzs.de http://www.dgzs.de/_downloads/dgzs-positionspapier-protrusionsschienen-beisbas-2006.pdf, mit dem Anmelder der vorliegenden Anmeldung als einem der Autoren; S3 Leitlinie 2017 der DGSM, www.dgsm.de, Seiten 100, 114 unter: http://www.dgsm.de/downloads/aktuelles/S3%20LL%20Nicht-erholsamer %20Schlaf%20Kap%20SBAS%2011818_20_s2_lssue_PrintPDF%202017.pdf, G.S3 Leitlinie der DGSM: Mayer et al., "Nicht erholsamer Schlaf / Schlafstörungen" Somnologie, Band 20, Sonderheft 2, Januar 2017, Seiten 114 - 115).

Im Folgenden ist mit Schiene, UPS oder UPS-System immer eine zweiteilige einstellbare Unterkieferprotrusionseinrichtung gemeint.

Ein weiterer Vorteil solcher zweiteiliger einstellbarer UPS-Systeme ist, dass der Patient bei entsprechender Konstruktion dieser UPS-Systeme nicht nur in die therapeutische Vorschubposition hinein geführt werden kann sondern auch aus dieser heraus den Mund öffnen kann, ohne dass sich die UPS von den Zähnen lockert oder löst, was ein Öffnen und Schließen des Mundes bei eingesetzter UPS möglich macht. Hierdurch wird der Tragekomfort deutlich erhöht und werden die Kiefergelenke entlastet. Dieser Vorteil der freien Mundbewegung kommt besonders bei Patienten mit Angst vor Atmungsnot bei eingesetzter UPS und bei Patienten mit Kiefergelenkstörungen zum Tragen. Auch sind mit solchen UPS-Systemen noch verständliches Sprechen und Flüssigkeitsaufnahme möglich.

Beispiele für zweiteilige einstellbare UPS-Systeme zeigen die US 4 901 737 A, die US 6 041 784 A, die US 4 551 095, die EP 0 128 744 B1 und die DE 102 16 242 C1.

Aus dem Patent DE 102 16 242 C1 des Anmelders ist eine zweiteilige einstellbare UPS mit Teleskopvorrichtung bekannt, bei welcher auf jeder Kieferseite zwischen einem hinteren Bereich der Oberkieferschiene und einem vorderen Bereich der Unterkieferschiene je eine Teleskopstange angeordnet ist, mittels welchen die therapeutische Protrusion der UPS einstellbar ist. Bei ausreichend weit geöffnetem Mund kann die UPS bei auseinandergezogenen Teleskopstangen zahngerecht eingesetzt werden, ohne dass ein bestimmter Unterkiefervorschub eingenommen werden müsste, wie im Fall des Einsetzens einer monoblockartigen einteiligen UPS. Beim anschließenden Schließen des Mundes werden die beiden Teleskopstangen zusammengeschoben bis zu einem die Zusammenschiebbarkeit begrenzenden Anschlag, der entsprechend dem gewünschten therapeutischen Unterkiefervorschub durch Veränderung der Anschlagposition einstellbar ist. Bei dieser UPS mit Teleskopvorrichtung ist neben einer leichten schrittweisen Einstellbarkeit des Unterkiefervorschubs bis zum Erreichen des für den jeweiligen Patienten optimalen Vorschubs die leichte Aufsetzbarkeit dieser UPS auf die Ober-und Unterkieferzähne - gegebenenfalls auf eine Zahnprothese - von Vorteil. Mit einer solchen UPS kann problemlos und einfach dem Umstand Rechnung getragen werden, dass meist nicht von vornherein bekannt ist, wann eine bestimmte Unterkiefereinstellung zu dem optimalen therapeutischen Erfolg führt.

Teleskopschienen gemäß DE 102 16 242 C1 haben sich zwar recht gut bewährt. Unter bestimmten Aspekten hat sich aber Verbesserungsbedarf herausgestellt. Bedingt durch die Führung der seitlichen Teleskope kann weder eine habituelle (entspannte) Unterkieferöffnung eingenommen noch von irgendeiner mit eingesetzter Schiene eingenommenen Unterkieferöffnungsposition zwanglos in den therapeutischen Vorschub hinein geglitten werden, was der physiologischen Kieferbewegung entgegen steht und Auslöser von unerwünschten Nebenwirkungen sein kann. Da die Unterkieferöffnungsbewegung diametral zur Bewegung der Teleskope verläuft, kommt es am vorderen Ende der Unterkieferschiene und am hinteren Ende der Oberkieferschiene zu Drehmomenten mit resultierender Hebelwirkung. Wird dieser Hebelwirkung nicht mit einer entsprechend ausgebildeten Eigenfriktion und Klemmpassung der UPS auf den Zähnen bzw. Prothesen entgegengewirkt, kommt es zu einer Lockerung oder einem Ablösen der Schiene von den Zähnen bzw. Prothesen. Je höher die Eigenfriktion oder Klemmpassung der Schiene jedoch ist, desto schwieriger ist es für den Patienten, die Schiene auf die Zähne aufzusetzen und sie von denen wieder herunter zu ziehen, wodurch es zu Beschädigungen der Zähne, des Zahnfleischs, gegebenenfalls der Prothesen kommen und bei dem Patienten ein Dyskomfort bzw. Angst ausgelöst werden kann.

Solche Drehmomente führen auch zu einer Beeinträchtigung der physiologischen Kiefergelenkbewegung während des Gleitens des Unterkiefers in die therapeutische Vorschubposition hinein und können zu Kiefergelenk- und Muskelschmerzen bzw. Veränderungen führen. Denn die Führungsbahnen der beiden Teleskope während der Gleitbewegung des Unterkiefers in die therapeutische Vorschubposition hinein entsprechen aufgrund der geometrischen Verhältnisse der Teleskope grundsätzlich nicht den Führungsbahnen der Dreh- und Gleitbewegung der beiden Kiefergelenke. Die Führungsbahnen des rechten und des linken Teleskops lassen sich somit nicht an die für die jeweiligen therapeutischen Vorschubpositionen typischen Bewegungsbahnen der beiden Kiefergelenke anpassen.

Ein weiterer Nachteil dieser Teleskopschiene ist, dass der Unterkiefer in den überwiegenden Fällen nur über eine mit mehr oder weniger Spannkraft eingesetzte gummielastische Verbindung zwischen dem Frontbereich der Unterkieferschiene und dem Frontbereich der Oberkieferschiene der UPS in der therapeutischen Vorschubposition gehalten werden kann, da der Kraftvektor der Teleskopschiene für den Unterkiefervorschub zu dem Kraftvektor für die Unterkieferöffnungsaktivität nicht gegenläufig ist oder ihm anderweitig durch z. B. Friktion entgegenwirkt. Diese frontalen gummielastischen Verbindungen reduzieren mehr oder weniger vollständig die Kraftvektoren der Teleskopschiene und der Unterkieferöffnungsaktivität des Patienten während des Schlafs und fördern somit den Halt der UPS auf den Zahnkronen. Diese frontale gummielastische Verbindung wirkt jedoch einer freien ungehinderten Mundbewegung entgegen, was sich einerseits positiv auf die Sicherung einer möglichst geringen Bissöffnung mit unverminderter Protrusion auswirkt, andererseits aber durch vom Patienten unabsichtlich während des Schlafs mit genügend Kraft ausgeführte Unterkieferöffnung zu einer Lockerung oder sogar Ablösung der UPS von den Zähnen bzw. Prothesen führen kann, der wiederum nur durch eine Erhöhung der Eigenfriktion und Klemmpassung der Schienen auf den Zähnen bzw. Prothesen entgegen gewirkt werden kann - mit allen oben beschriebenen Nachteilen für den Patienten.

Diese genannten Probleme der UPS mit Teleskopen verstärken sich zudem dann, wenn die Schienen der UPS auf mehr oder weniger gut fixierten Prothesen oder anderweitigem Zahnersatz aufliegen und führen in ausgeprägten Fällen sogar zur Unbrauchbarkeit dieser UPS.

Bei einer aus der EP 1 094 761 B1 bekannten zweiteiligen UPS erreicht man eine bestimmte Unterkieferprotrusionsposition mittels an beiden Kieferseiten angeordneten Eingriffselementepaaren, die je ein an der Oberkieferschiene angeordnetes oberkieferseitiges Eingriffselement und ein damit zusammenwirkendes, an der Unterkieferschiene angeordnetes unterkieferseitiges Eingriffselement aufweisen, wobei die beiden Eingriffselementepaare derart an den Schienen positioniert und derart geformt sind, dass sie, wenn die beiden Schienen auf die Zähne aufgesetzt sind, bei einer Unterkieferbewegung von einer weiten Unterkieferöffnung heraus in eine Mundschließstellung hinein in einen protrusionssteuernden gegenseitigen Eingriff miteinander gelangen. Die Protrusionsbewegung und Protrusionsposition wird durch die Form der Eingriffselemente bestimmt.

Mit den Eingriffselementen der zweiteiligen UPS gemäß EP 1 094 761 B1 werden oben beschriebene Nachteile der Teleskopschiene nicht überwunden und wird den physiologischen Gegebenheiten im Zusammenhang mit der Unterkieferbewegung und den Kiefergelenken nicht ausreichend Rechnung getragen.

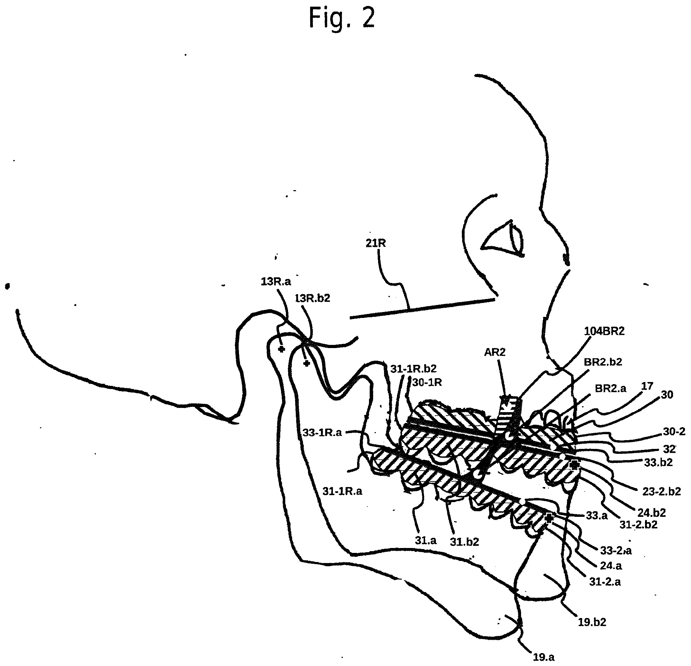

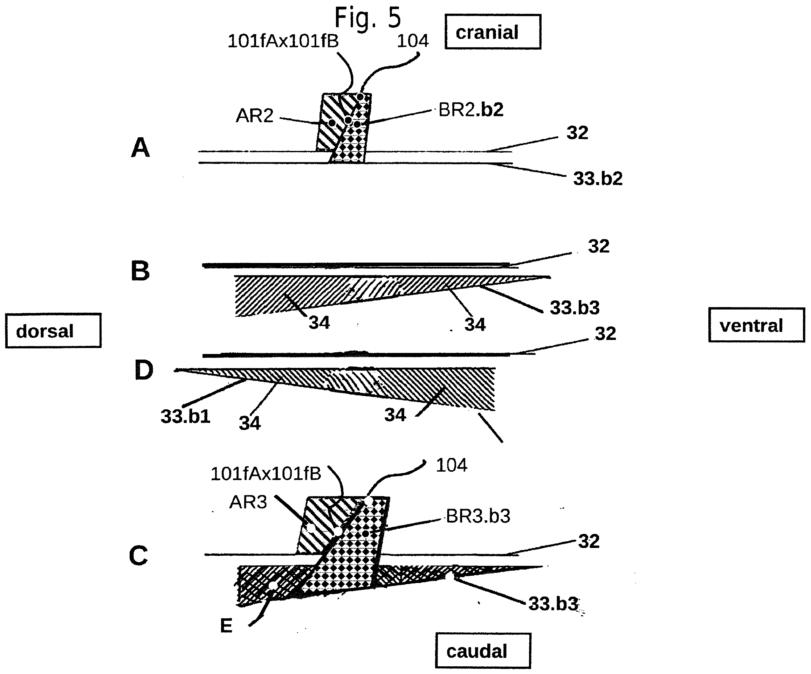

Für die Herstellung einer Schiene gemäß EP 1 094 761 B1 wird entsprechend dortigem Absatz [0032] lediglich ein Abdruck in habitueller (zwangloser) Unterkieferöffnung hergestellt, um die Länge der seitlichen Eingriffselemente zueinander, unter Beachtung der Strecke AB0-F in Fig. 5 der EP 1 094 761 B1, bestimmen und herstellen zu können. Aber bei der EP 1 094 761 B1 verläuft die durch die Kontaktflächenbahnen der Eingriffselemente gebildete Protrusionsführungsbahn der Eingriffselemente parallel zur Protrusionsgrenzbahn. Dies ist ersichtlich unter Anderem aus Spalte 5, Zeilen 41-46 in Verbindung mit Fig. 5, wonach die Eingriffsflächen der Eingriffselemente gemäß einer Linie C1C2 entsprechend dem Unterkieferöffnungsbogen A1A2 bei maximaler Protrusion entsprechend einer Teilkreisbahn gekrümmt sind. Oder, wie es in Spalte 6, Zeilen 54-57 ausgedrückt und auch in Anspruch 1 der EP 1 094 761 B1 beansprucht ist: Die Kontur der Eingriffsflächen verläuft parallel zur Krümmung der Protrusionsgrenzbahn. Die Protrusionsgrenzbahn A1A2 ist die Bahn, die die Unterkieferöffnung aus einer maximalen Protrusion heraus beschreibt (Spalte 5, Zeilen 1-2 und 41-42), wobei das Kiefergelenkkopfzentrum, das sich bei Schlussbiss in der Ausgangsposition Bx (Fig. 2b) befindet, bei maximaler Protrusion in einer maximalen ventralen Stellung in Position Ax steht (Spalte 5, Zeilen 1-2 und 45-46; Fig. 2b). Bei einer Schiene, wie sie in EP 1 094 761 B1 beansprucht ist, muss der Patient nach Einsetzen der Schiene auf die Zähne noch zwingend eine Protrusionsbewegung vollführen, um die Eingriffselemente in Eingriff miteinander bringen zu können. Sind sie in Eingriff gekommen, kann der Patient ausschließlich über die unphysiologische Protrusionsgrenzbahn nur in eine maximale Protrusion gelangen. Solche maximalen Protrusionen sollten aber vermieden werden um einerseits die Nebenwirkungen auf die Kiefergelenke, Muskulatur und Nerven zu vermeiden und weil diese maximale Protrusion auf die Öffnung der oberen Atmungswege einen adversen Effekt hat. Eine Ausführungsform der EP 1 094 761 B1 ermöglicht eine variable Positionsveränderung der Eingriffselemente an den Schienen zur Einstellung einer von dieser maximalen Protrusion abweichenden, also einer geringeren Protrusion. Der unphysiologischen Biomechanik wird hiermit jedoch nicht Abhilfe geschaffen. Die unverändert parallel zu der maximal möglichen Protrusion geformten Kontaktflächenbahnen der Eingriffselemente, also entsprechend der Protrusionsgrenzbahn geformt, entsprechen nicht den Protrusionsbahnen für geringere Protrusionspositionen des Unterkiefers.

Die UPS gemäß vorliegender Erfindung schafft hier Abhilfe, da der Patient durch deren nachstehend erläuterte und beanspruchte Gestaltung in einer zwanglosen Position, der habituellen Unterkieferöffnung, die Schiene in Eingriff nehmen kann und sein Unterkiefer durch eine anschließende physiologische Unterkieferschließbewegung (Protrusionsbewegung) in eine beliebige, nach physiologisch therapeutischen Gesichtspunkten gewählte Protrusionsposition geführt werden kann. Es können also je nach therapeutischem Bedarf unterschiedliche, zuvor bestimmte therapeutische Protrusionspositionen mit unterschiedlichen Protrusionsführungsbahnen erreicht werden.

In der habituellen Unterkieferöffnung befindet sich der Unterkiefer in einer habituellen, im Wesentlichen weder einer Protrusion noch einer Retrusion unterliegenden Unterkieferöffnungsposition, in die er aus der Schlussbissposition, also aus einer Position mit maximalem Ober- und Unterkieferzahnkontakt, gelangt ist.

Diese Bewegung aus z. B. der habituellen Unterkieferöffnung in die therapeutische Protrusion hinein, der eine Rotations- und Translationsbewegung des Kiefergelenkkopfzentrums zugrunde liegt, werden im Folgenden zur sprachlich kürzeren Darstellbarkeit auch als therapeutische Protrusionsbewegung und die entsprechende Bewegungsbahn als therapeutische Protrusionsbahn bezeichnet, wobei hier darauf hingewiesen sei, dass der therapeutische Effekt in der damit erreichten Protrusion liegt und nicht in der Bewegung in die therapeutische Protrusion hinein. Diese therapeutische Protrusionsbahn findet bei der EP 1 094 761 B1, bei welcher die Führungsbahnen der Eingriffselemente parallel zu der bei maximaler Protrusion auftretenden Protrusionsgrenzbahn geformt sind, keine Berücksichtigung.

WO 00/01317 A1 offenbart eine Unterkieferprotrusionseinrichtung für eine therapeutische Unterkieferprotrusion bei einem zu therapierenden Patienten. Eine an zwei Schienenkörpern angeordnete, den therapeutischen Unterkiefervorschub bewirkende Vorschubeinrichtung weist in den beiden Seitenbereichen des Unterkieferschienenkörpers angeordnete vordere Eingriffselemente und in den beiden Seitenbereichen des Oberkieferschienenkörpers angeordnete hinteren Eingriffselemente auf, die auf jeder Kieferseite ein durch Mundschließbewegung in einen protrusionssteuernden Eingriff miteinander bringbares Eingriffselementepaar bilden, mit protrusionssteuernden Führungsbahnen an den vorderen Eingriffselementen und an den hinteren Eingriffselementen.

DE 10 2014 117 080 A1 offenbart ein Verfahren zum Ermitteln einer Therapieposition für eine Protrusionsschiene sowie ein Verfahren zur Herstellung einer solchen Protrusionsschiene. Das Verfahren zum Ermitteln einer Therapieposition umfasst die Verfahrensschritte Bereitstellen von patientenindividuellen Daten des Kiefergelenkapparates des Patienten und Ermitteln einer Therapieposition unter Berücksichtigung dieser Daten.

DE 10 2013 100 898 A1 zeigt eine Unterkieferprotrusionseinrichtung für eine therapeutische Unterkieferprotrusion mit unterem und oberem Einsatz, wobei jeder Einsatz dazu eingerichtet ist, an jeweiligen oberen und unteren Zähnen zurückgehalten zu werden, wobei der untere Einsatz zwei Flansche aufweist, die sich beim Tragen zum oberen Einsatz hin erstrecken. Der obere Einsatz hat entsprechende flache Bereiche auf jeder Seite, die sich in die äußere (bezogen auf die Zähne) Ebene des Einsatzes schneiden. Die Flansche liegen somit an derartigen flachen Bereichen an der äußeren Seite des oberen Einsatzes an, drücken den unteren Einsatz und daher die unteren Zähne und die Zunge nach vorne.

Die vorliegende Erfindung betrifft eine Unterkieferprotrusionseinrichtung und ein Verfahren zu deren Herstellung gemäß den beigefügten Patentansprüchen. Ausführungsformen sind in der Unteransprüchen angegeben.

Der Erfindung liegt die Erkenntnis zugrunde, dass es für unterschiedliche Positionen entlang der Unterkiefererstreckung, die für eine therapeutische Anordnung der Eingriffselemente gewählt werden können, zu verschieden verlaufenden therapeutischen Protrusionsbahnen mit unterschiedlichen Bahnkrümmungen kommt, die je nach Protrusionsgrad flacher oder steiler verlaufen und auf jeden Fall von der Protrusionsbahn bei maximaler Protrusion (wie der Protrusionsgrenzbahn in der EP 1 094 761 B1) erheblich abweichen. Durch Abstimmung der Form der Führungsbahnen der Eingriffselemente auf eine therapeutische Protrusionsbahn, die von dem Ort entlang der Unterkiefererstreckung, an dem die Führungsbahnen der Eingriffselemente liegen sollen, abhängt und patientenindividuell messbar ist, kann den physiologischen Gegebenheiten der Kiefergelenke des jeweiligen Patienten Rechnung getragen werden. Durch diese patientenindividuelle Anpassung lässt sich die Verträglichkeit der resultierenden UPS deutlich verbessern.

Die Führungsbahnen können durch Führungsflächen und/oder Führungskanten der Eingriffselemente gebildet sein.

Zusätzlich zu sagittalen Unterkieferbewegungen beim Öffnen und Schließen des Mundes können auch seitliche Unterkieferbewegungen ausgeführt werden. Zu solchen Seitbewegungen gehören Mediotrusions- und Laterotrusionsbewegungen des Unterkiefers. Mediotrusionsbewegungen bewegen eine der beiden Unterkieferseiten relativ zur zugehörigen Oberkieferseite in Richtung Mundmitte. Laterotrusionsbewegungen bewegen eine der beiden Unterkieferseiten relativ zur zugehörigen Oberkieferseite von der Mundmitte weg gerichtet zur Seite hin. Eine Mediotrusionsbewegung auf einer Kieferseite führt zwangsweise zu einer Laterotrusionsbewegung auf der anderen Kieferseite. Die Mediotrusion und die Laterotrusion verlaufen je auf physiologischen Bewegungsbahnen, die nicht linear verlaufen sondern gekrümmt sind, und zwar mit unterschiedlich starker Krümmung und unterschiedlicher Bewegungsrichtung.

Diesen physiologischen Gegebenheiten trägt ein weiterer Aspekt der Erfindung Rechnung, wonach bei einer Unterkieferprotrusionseinrichtung der eingangs angegebenen Art die Eingriffselemente mit Seitbewegungen des Unterkiefers führenden gekrümmten Transversalbahnen versehen sind, die entsprechend physiologischen Seitbewegungsbahnen des Unterkiefers gekrümmt sind, die an der Position der Führungsbahnen der Eingriffselemente bei einer Seitbewegung des Unterkiefers aus der therapeutischen Protrusionsposition heraus patientenindividuell messbar sind.

Diese Bewegung von der therapeutischen Protrusionsposition in eine Seitbewegung hinein und wieder zurück wird im Folgenden - wieder zur sprachlich kürzeren Darstellbarkeit - auch therapeutische Protrusionsseitbewegung genannt, und ihre Bewegungsbahn wird auch therapeutische Protrusionsseitbewegungsbahn genannt. Auch hier sei erwähnt, dass damit nicht eine therapeutische Wirkung einer Seitbewegung gemeint sein soll sondern eine Seitbewegung aus der therapeutischen Protrusion heraus.

Diesem Aspekt der Erfindung liegt einerseits die Erkenntnis zugrunde, dass es auch im Schlaf zu Seitbewegungen des Unterkiefers kommt, auch bei eingesetzter UPS, sofern diese solche seitlichen Bewegungen nicht behindert.

Und dem liegt andererseits die weitere Erkenntnis zugrunde, dass derartige Seitbewegungen des Unterkiefers blockiert werden, wenn die Eingriffselemente einer UPS gemäß EP 1 094 761 B1 gestaltet sind. Denn die Führungsbahnen von deren Eingriffselementen sind zwar in einer sagittalen Ebene entsprechend der Protrusionsgrenzbahn gekrümmt, geben somit eine Bewegung in der sagittalen Ebene frei, verlaufen aber in Transversalrichtung flach (nicht gekrümmt). Seitbewegungen des Unterkiefers verlaufen jedoch, wie zuvor erwähnt, auf gekrümmten Bewegungsbahnen. Seitbewegungen des Unterkiefers, die nur auf den physiologisch vorgegebenen gekrümmten Bewegungsbahnen möglich sind, werden daher bei gemäß EP 1 094 761 B1 in Transversalrichtung flach gestalteten Eingriffselementen blockiert.

Für eine optimale Seitbewegungsführung des Unterkiefers sind die transversalen Führungsbahnen der Eingriffselemente in einem ersten Führungsbereich entsprechend der Mediotrusionsbahn und in einem zweiten Führungsbereich entsprechend der Laterotrusionsbahn, die patientenindividuell am Ort der Führungsbahnen der Eingriffselemente messbar sind, gestaltet.

Hinsichtlich der jeweiligen Lage dieser ersten und zweiten Führungsbereiche sind die Eingriffselemente auf der einen Kieferseite spiegelbildlich zu den Eingriffselementen auf der anderen Kieferseite ausgebildet.

Besonders bevorzugt ist eine Ausführungsform einer Unterkieferprotrusionseinrichtung, bei welcher die beiden oben angegebenen Erfindungsaspekte in Kombination verwirklicht sind, also die die Führungsbahnen aufweisenden Führungsflächen der Eingriffselemente sowohl entsprechend der sagittalen therapeutischen Protrusionsbahn als auch entsprechend der therapeutischen Mediotrusionsbahn und der therapeutischen Laterotrusionsbahn, die am Ort der Führungsbahnen der Eingriffselemente patientenindividuell messbar sind, gestaltet sind.

Bei einer vorteilhaften Weiterbildung der Erfindung sind die Eingriffselemente an den Schienen in lösbarer Weise befestigt, sodass bei einer Änderung der anfangs gewählten Protrusion zum Zweck der Annäherung an eine therapeutisch optimale Protrusion und entsprechender Änderung der Führungsflächen der Eingriffselemente ansonsten die bereits gefertigte UPS weiter verwendet werden kann.

Bei geschlossenem Unterkiefer ohne Protrusion liegen die oberen und die unteren Molaren flächig aufeinander. Bewegt man nun den Unterkiefer in zunehmende Protrusion, entsteht ein zu den hinteren Molaren hin zunehmender keilförmiger Spalt zwischen den Ober- und Unterkieferzähnen. Entsprechend entsteht bei auf die Zähne aufgesetzter UPS ein entsprechender Spalt zwischen der Oberkieferschiene und der Unterkieferschiene, mit entsprechend ungleichmäßiger Kraftverteilung über die Molarenreihen bei Krafteinwirkungen auf die UPS. Dadurch werden unphysiologische Belastungen und Fehlstellungen der Eingriffselemente, somit auch ihrer Führungsflächen, zueinander hervorgerufen.

Dem wird bei einer vorteilhaften Ausführungsform der Erfindung dadurch abgeholfen, dass mindestens der Unterkieferschiene keilförmige Distanzplättchen zugeordnet werden, die entsprechend dem keilförmigen Spalt zwischen Unterkieferschiene und Oberkieferschiene, der sich bei dem jeweiligen Patienten bei der therapeutischen Protrusion messen lässt, gestaltet werden. Damit lassen sich die genannten physiologischen Belastungen und Fehlstellungen der Eingriffselemente zueinander beheben.

Das rechte und das linke Kiefergelenk liegen je außerhalb der Medianebene, der durch den Inzisalpunkt gehenden Sagittalebene. Entsprechend liegen die Protrusionsbewegungsbahnen der beiden Kiefergelenke nicht auf der mittigen Medianebene sondern je auf verschiedenen außermittigen Sagittalebenen. Dem kann man mit einer sogenannten vollfunktionellen oder mit einer sogenannten halbfunktionellen Methode bei der Gestaltung der Führungsflächen der Eingriffselemente Rechnung tragen. Bei der vollfunktionellen Methode werden Messungen am Ort der Führungsflächen der Eingriffselemente zugrunde gelegt. Bei der halbfunktionellen Methode wird auf Messungen an zwei ortsfern der Eingriffselemente liegenden Unterkieferpunkten, vorzugsweise auf Messungen am Inzisalpunkt und am Kiefergelenkkopfzentrum derselben Seite (d. h. der Seite der zu berechnenden Führungsflächen), zurückgegriffen.

Der Inzisalpunkt ist definiert als der Berührungspunkt der Schneidekanten der beiden unteren mittleren Schneidezähne. Er liegt 1 mm unterhalb deren Schneidekanten.

Bei der Methode der halbfunktionellen Gestaltung der Führungsflächen der Eingriffselemente kann aus den therapeutischen Protrusionsbahnen, nämlich aus der während der therapeutischen Protrusionsbewegung einerseits auf mesialer Seite des Eingriffselementepaares am Unterkiefer, vorzugsweise am Inzisalpunkt, patientenindividuell messbaren Bewegungsbahn und aus der an mindestens einem ausgewählten distal der Eingriffelemente gelegenen Unterkieferpunkt, vorzugsweise im Kiefergelenkkopfzentrum derselben Seite, patientenindividuell messbaren Kiefergelenkkopfzentrumbewegungsbahn errechneten Protrusionsbahn gestaltet werden oder die während der therapeutischen Protrusionsbewegung zwei auf mesialer oder zwei auf distaler Seite des Eingriffselementepaares am Unterkiefer messbaren Bewegungsbahnen gestaltet werden.

Hierfür wird vorzugsweise für einzelne zusammengehörende Punktepaare dieser beiden Bewegungsbahnen jeweils eine erste Strecke zwischen dem in der medianen Sagittalebene liegenden Inzisalpunkt und dem auf die mediane Sagittalebene projizierten Kiefergelenkkopfzentrum ermittelt. Der Bezug zur Bewegungsbahn eines z. B. cranialen Oberkantenpunktes des vorderen Eingriffselementes wird hergestellt über den Winkel zwischen einer ersten Strecke, die zwischen dem Inzisalpunkt und dem Kiefergelenkkopfzentrumpunkt verläuft, und einer zweiten Strecke, die zwischen dem Inzisalpunkt und diesem Oberkantenpunkt verläuft, und über den Winkel zwischen der ersten Strecke und einer dritten Strecke, die zwischen dem Mittelpunkt des jeweiligen Kiefergelenkkopfzentrums und diesem Oberkantenpunkt 104 verläuft.

Die halbfunktionelle Methode arbeitet somit mit einer indirekten Ermittlung der therapeutischen Protrusionsbahn am Ort der Führungsflächen der Eingriffselemente aus den Bewegungsbahnen von vorzugsweise Inzisalpunkt und jeweiligem Kiefergelenkkopfzentrum (halbfunktionelle endwertige seitenindividuelle Methode). Die Führungsflächen der beiden Eingriffselemente können lediglich spiegelbildlich hergestellt zu werden, wenn aus den errechneten therapeutischen Protrusionsbahnen der rechten und linken Seite ein Mittelwert errechnet wird (halbfunktionelle mittelwertige Methode) oder wenn beide Eingriffselementepaare nach den Werten nur einer Seite ausgeformt werden (halbfunktionelle endwertige rechts- oder linksseitige Methode).

Entsprechendes gilt, wenn bei der halbfunktionellen Methode die Messung an einem auf mesialer Seite der Eingriffselemente am Unterkiefer gelegenen Ort bzw. an einem distal der Eingriffelemente gelegenen Unterkieferpunkt vorgenommen wird.

Bei der der vollfunktionellen Methode zur Gestaltung der Führungsflächen der Eingriffselemente werden die therapeutischen Protrusionsbahnen nicht durch Errechnung aus der Protrusionsbahn am z. B. Inzisalpunkt und der Protrusionsbahn am z. B. Kiefergelenkkopfzentrum erhalten sondern durch Messungen der Protrusionsbahnen am Ort der Führungsflächen der Eingriffselemente (vollfunktionelle endwertige seitenindividuelle Methode).

Die Führungsflächen der beiden Eingriffselemente können auch bei dieser Methode lediglich spiegelbildlich hergestellt werden, wenn aus den gemessenen therapeutischen Protrusionsbahnen der rechten und linken Seite ein Mittelwert errechnet wird (vollfunktionelle mittelwertige Methode) oder wenn beide Eingriffselementepaare nach den Werten nur einer Seite ausgeformt werden (vollfunktionelle endwertige rechts oder linksseitige Methode)..

Zwischen den beiden Kiefergelenken besteht eine physiologische Seitenasymmetrie insofern, als die Bewegungen der beiden Kiefergelenke nicht seitensymmetrisch sind sondern in seitenasymmetrischer Weise von einer in einer mittigen Sagittalebene (Medianebene) betrachteten Bewegungsbahn nicht nur mehr oder weniger stark abweichen sondern auch unterschiedlich nach links und/oder rechts abweichen. Dem kann man durch kieferseitenindividuelle Messungen Rechnung tragen, unter Einsatz der Methode der vollfunktionellen Gestaltung der Führungsflächen der Eingriffselemente.

Dem trägt eine vorteilhafte Ausführungsform der Erfindung Rechnung, bei welcher für die Gestaltung der protrusionssteuernden Führungsbahnen des Eingriffselementepaars auf der rechten Schienenseite und der protrusionssteuernden Führungsbahnen des Eingriffselementepaars auf der linken Schienenseite je nicht nur patientenindividuelle sondern auch kieferseitenindividuelle Messungen an der Position der Führungsbahnen des rechten bzw. linken Eingriffselementepaars zugrunde gelegt werden. Die therapeutischen Protrusionsbahnen werden also für die rechte und für die linke Kieferseite getrennt gestaltet.

Zur leichteren Übersichtlichkeit über die hier verwendeten Begrifflichkeiten folgt eine Zusammenfassung der verschiedenen Methoden zur Ermittlung von patientenindividuellen therapeutischen Protrusionsbahnen am Ort der Eingriffselemente:
- Vollfunktionelle endwertige links- oder rechtsseitige Methode:
   Beide Eingriffselementepaare werden nach der Messung am Ort der Eingriffselemente entweder der linken oder rechten Kieferseite spiegelsymmetrisch ausgeformt.
- Vollfunktionelle endwertige seitenentsprechende / seitenindividuelle Methode:
   Die Eingriffselementepaare jeder Kieferseite werden individuell entsprechend den Messungen am Ort der jeweiligen Eingriffselemente dieser Seite ausgeformt.
- Vollfunktionelle mittelwertige Methode:
   Aus den Messungen am Ort der Eingriffselemente auf beiden Kieferseiten wird der Mittelwert gebildet und werden beide Eingriffselementepaare entsprechend diesem Mittelwert spiegelbildlich ausgeformt.
- Halbfunktionelle endwertige links- oder rechtsseitige Methode:
   Beide Eingriffselementepaare werden nach Messungen an zwei Unterkieferpunkten ortsfern der Eingriffselemente und nach den daraus errechneten Werten am Ort der Eingriffselemente einer Kieferseite spiegelsymmetrisch ausgeformt.
- Halbfunktionelle endwertige seitenentsprechende / seitenindividuelle Methode:
   Die Eingriffselementepaare jeder Kieferseite werden seitenindividuell entsprechend zwei Messungen ortsfern der Eingriffselemente und nach den daraus errechneten Werten am Ort der jeweiligen Eingriffselemente der jeweiligen Kieferseite ausgeformt.
- Halbfunktionelle mittelwertige Methode:
   Aus den zwei Messungen ortsfern der Eingriffselemente und nach den daraus errechneten Werten am Ort der Eingriffselemente auf beiden Kieferseiten wird der Mittelwert gebildet und werden beide Eingriffselementepaare dem Mittelwert entsprechend spiegelbildlich ausgeformt.

Bisher sind physiologisch normale Bewegungsbahnen der Kiefergelenkkopfzentren betrachtet worden. Es gibt aber auch Patienten, bei denen diese Bewegungsbahnen infolge einer Schädigung, z. B. der zwischen Kiefergelenkkopfzentrum und Kiefergelenkbahn befindlichen Kiefergelenkscheibe, an einer entsprechenden Stelle ihres Bahnverlaufs eine Abweichung vom physiologisch normalen Verlauf zeigen. Wird bei der patientenindividuellen Messung der Bewegungsbahn eines Kiefergelenkkopfzentrums eine solche Abweichung festgestellt, kann es vorteilhaft sein, diese bei der Gestaltung der Führungsflächen der Eingriffselemente nicht zu berücksichtigen sondern den physiologisch abnormalen Verlaufsbereich durch einen physiologisch korrekten Verlaufsbereich zu überbrücken. Bei einer mittels der Führungsbahnen der Eingriffselemente gesteuerten Führung des Unterkiefers aus der habituellen Unterkieferöffnung heraus in die therapeutische Protrusion wird die Auswirkung der Schadstelle dann überbrückt und das geschädigte Kiefergelenk geschont. Da meist nur eine Schädigung auf einer Kiefergelenkseite vorliegt oder unterschiedlich starke oder unterschiedlich gelagerte Schädigungen auf den beiden Kiefergelenkseiten, empfiehlt es sich im Fall solcher Schädigungen besonders, die Führungsflächen der beiden Einstellelementpaare entsprechend kieferseitenindividuellen Messungen zu gestalten.

Wenn vorliegend einerseits von Führung und andererseits von Steuerung die Rede ist, ist damit praktisch das Gleiche gemeint. Die sich gegenüberliegenden Führungsflächen des jeweils betrachteten Eingriffselementes führen sich bei einer Relativbewegung zwischen diesen beiden Eingriffselementen gegenseitig und steuern damit einerseits ihre Relativposition zu einander und steuern damit andererseits, über die mit den Eingriffselementen verbundenen beiden Schienen der UPS, auch eine Bewegung des Unterkiefers relativ zum Oberkiefer. Umgekehrt kann man sagen, dass durch die Führungsflächen der Eingriffselemente deren Relativposition zueinander gesteuert wird und als Folge die Bewegung der Eingriffselemente relativ zueinander und dadurch letztendlich die Unterkieferprotrusion geführt wird.

Weitere Aspekte und vorteilhafte Weiterbildungen der Erfindung, sowohl unter vorrichtungsmäßigen als auch unter verfahrensmäßigen Gesichtspunkten, sind den Patentansprüchen entnehmbar.

Weitere Gesichtspunkte zweiteiliger UPS herkömmlicher Art, die anhand von Zeichnungen leichter verständlich gemacht werden können, und erfindungsgemäßer Art werden nun anhand von Zeichnungen näher erläutert. In diesen zeigen je in schematisierter Darstellung:
- Fig. 1: Eine Seitenansicht eines menschlichen Schädels ohne UPS in einer Sagittalebene, in der das rechte Kiefergelenk liegt.
- Fig. 2: Die in Fig. 1 gezeigte Seitenansicht mit eingesetzter UPS und deren Eingriffselementen einerseits in habitueller (zwangloser) Unterkieferöffnung und andererseits bei geschlossenem Kiefer in therapeutischer Protrusion.
- Fig. 3 I: Eine gegenüberstellende Betrachtung von unterschiedlichen therapeutischen Protrusionsbahnen und den daraus resultierenden unterschiedlichen ventralen und dorsalen Kiefergelenkkopfzentrumspositionen.

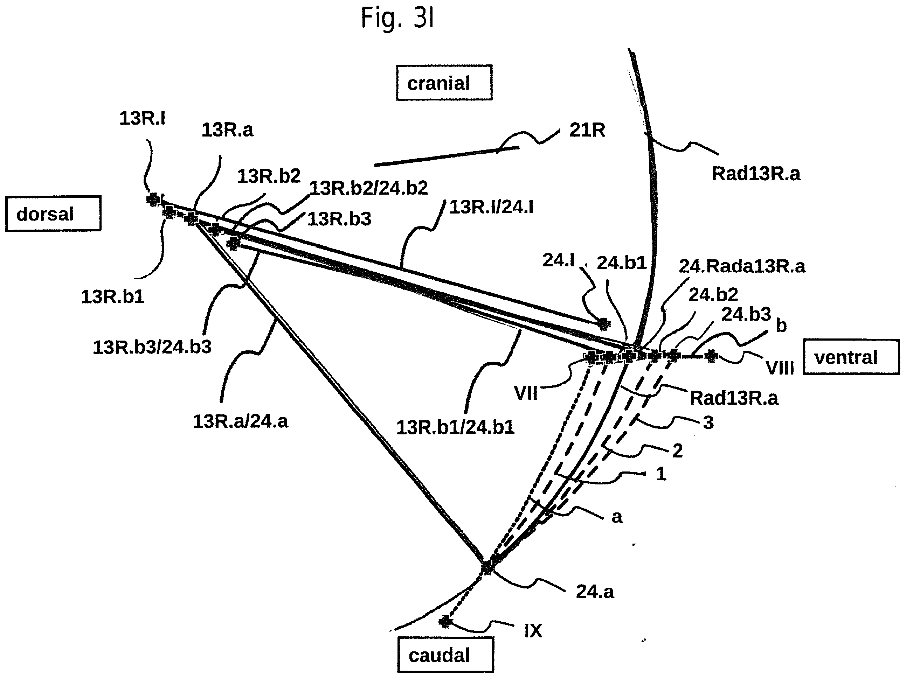

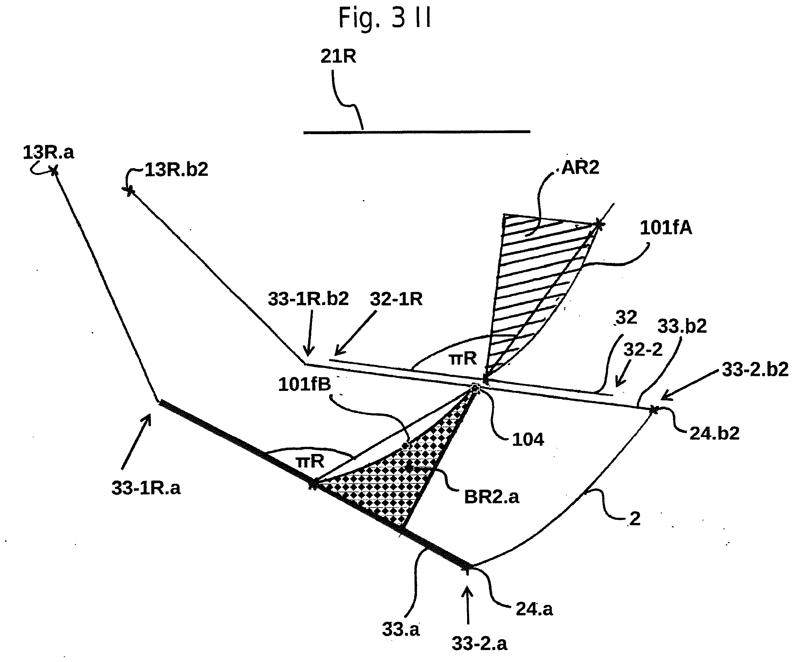

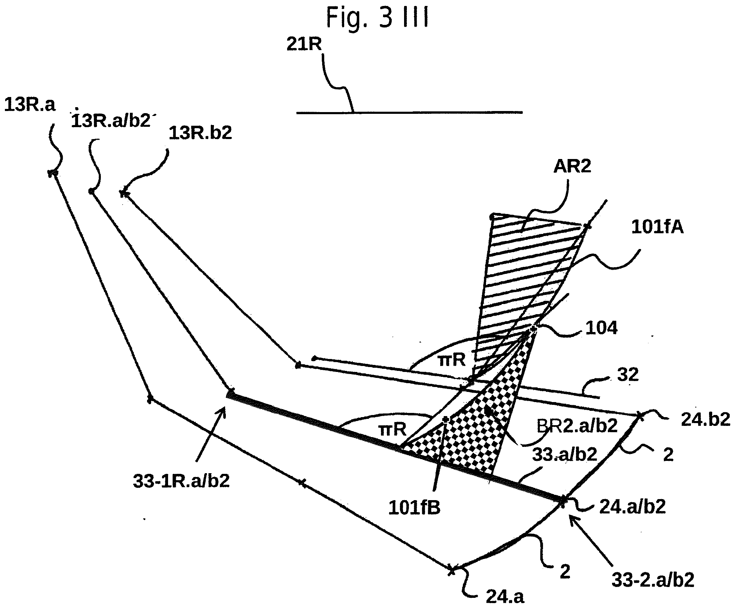

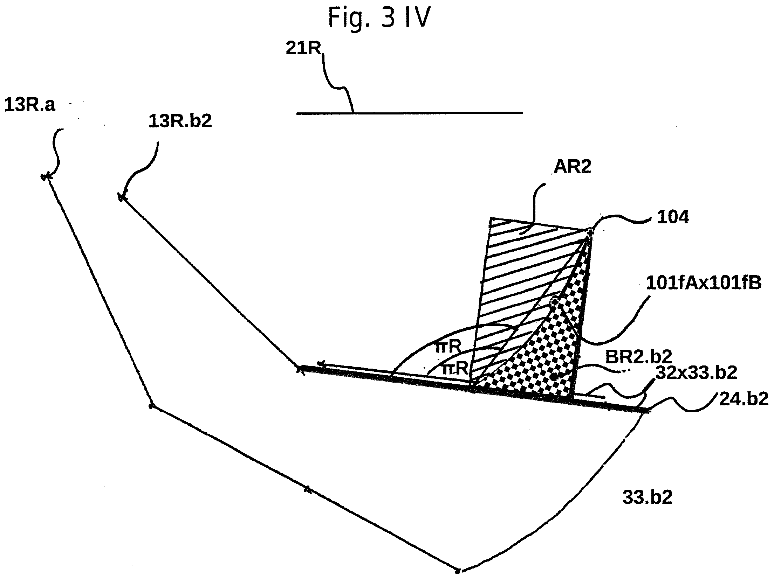

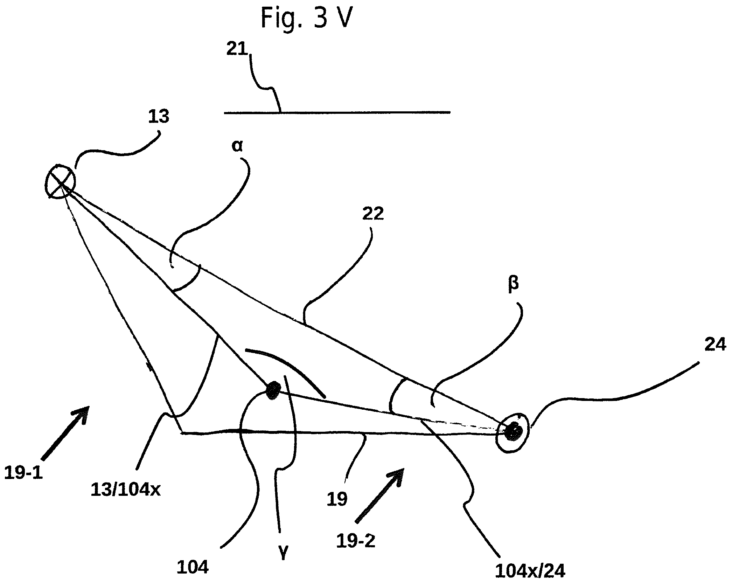

- Fig. 3 II-IV: Extrem schematisierte Darstellungen der Unterkieferschienenbasis, der Oberkieferschienenbasis und der Eingriffselemente in Positionen bei unterschiedlicher Unterkieferöffnung und Protrusion mit resultierenden ventralen Kiefergelenkkopfzentrumpositionen.
- Fig. 3 II bis 3 V: zeigen folgende Konstellationen:
- Fig. 3 II: gemäß Fig. 2 in habitueller Unterkieferöffnung ohne gegenseitigen Eingriff der Eingriffselemente und mit dorsal liegendem Kiefergelenckopfzentrum bei habitueller Unterkieferöffnung.
- Fig. 3 III: bei teilweisem gegenseitigen Eingriff der Eingriffselemente bei einer teilweisen Unterkieferöffnung auf einer therapeutischen Protrusionsbahn, die auf halber Wegstrecke zwischen den in Fig. 3 II und 3 IV gezeigten Unterkieferstellungen liegt, und bei einer Position des Kiefergelenkkopfzentrums zwischen dessen in Fig. 3 II und Fig. 3 IV gezeigten Positionen.
- Fig. 3 IV: gemäß Fig. 2 in therapeutischer Protrusion bei einem Aufeinanderliegen von Oberkieferschiene und Unterkieferschiene in therapeutischer Protrusion, vollständigem gegenseitigen Eingriff der Eingriffselemente und ventral liegendem Kiefergelenkkopfzentrum.
- Fig. 3 V: Eine geometrische Darstellung im Zusammenhang mit der Bestimmung von Führungsbahnen der Eingriffselemente aus Bahnmessungen ortsfern von den Eingriffselementen beispielhaft am Inzisalpunkt und am Kiefergelenkkopfzentrum nach der halbfunktionellen Methode.

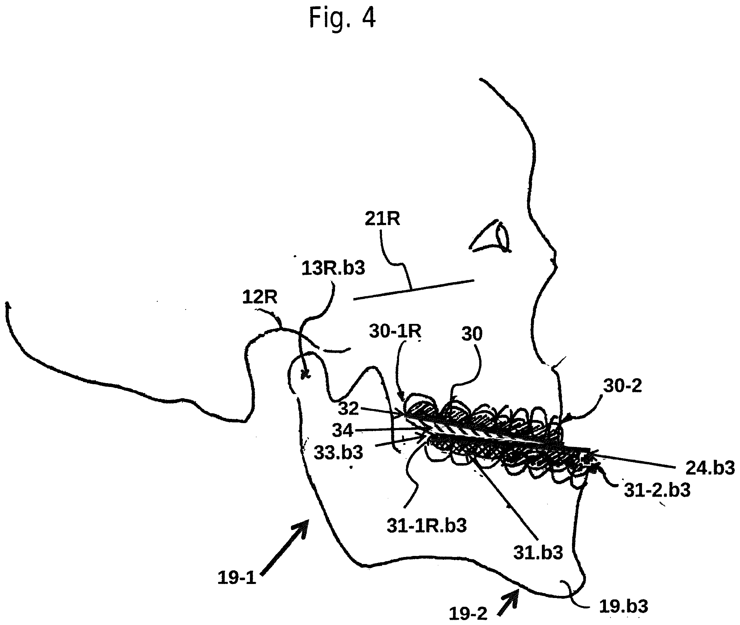

- Fig. 4: Die in Fig. 2 gezeigte Seitenansicht mit eingesetzter UPS bei geschlossenem Unterkiefer jedoch mit vergrößerter Unterkieferprotrusion und dabei entstehendem keilförmigen Spalt zwischen Oberkieferschiene und Unterkieferschiene.
- Fig. 5: Ein Schienenpaar mit Oberkieferschiene und Unterkieferschiene: Darstellung A mit einem oberkieferseitigen hinteren Eingriffselement und einem unterkieferseitigen vorderen Eingriffselement ohne keilförmigen Spalt; Darstellung B mit keilförmigem Spalt ohne Eingriffselemente bei einer Positionenenkonstellation, bei welcher sich ein keilförmiger Spalt mit Dickenzunahme in dorsaler/distaler Richtung ergibt; Darstellung D eine Positionenenkonstellation, bei welcher sich ein keilförmiger Spalt mit Dickenzunahme in mesialer Richtung ergibt. Darstellung C mit Eingriffselementen und keilförmigem Spalt, mit einem den Spalt kompensierenden Distanzplättchen und mit einem an die dadurch veränderte therapeutische Protrusion und die daraus resultierende neue therapeutische Protrusionsbahn angepassten Eingriffselementepaar;

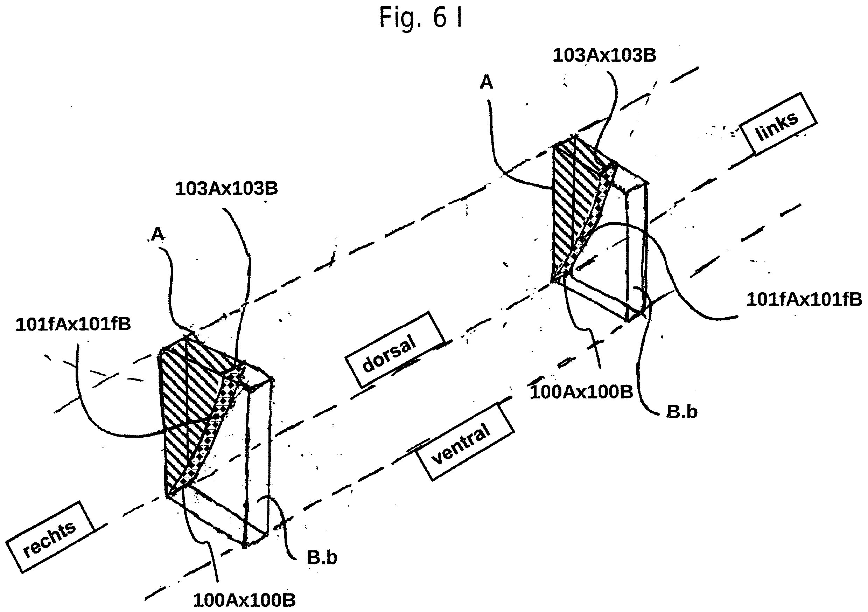

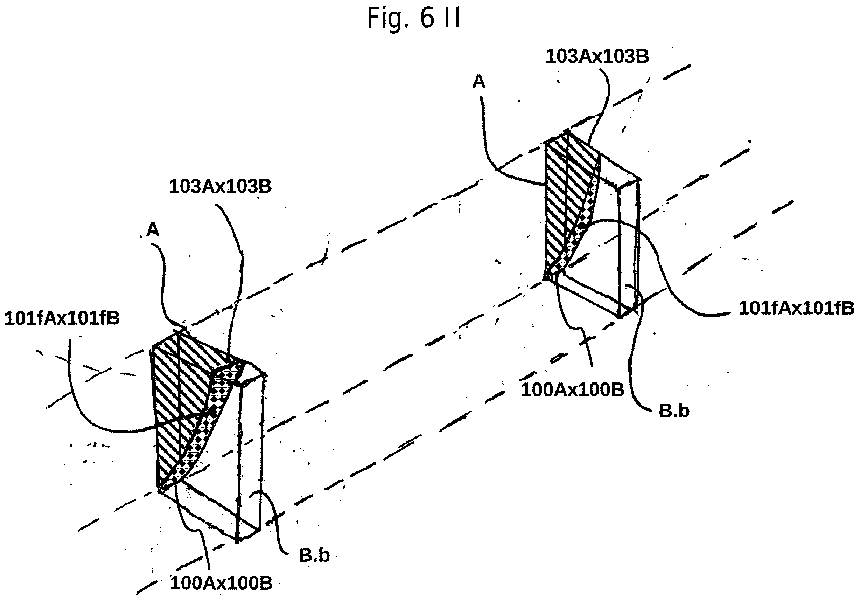

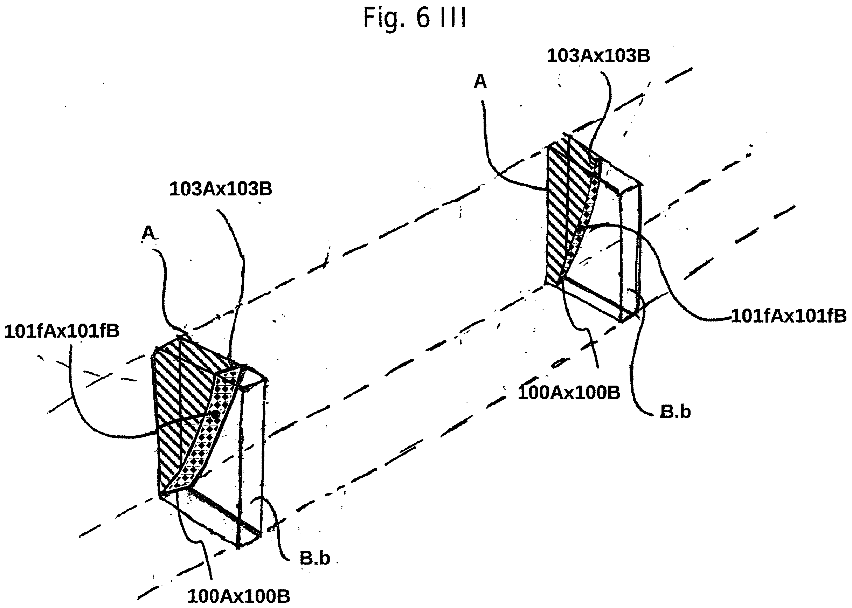

- Fig. 6 I - 6 III: Ein rechtes und ein linkes Eingriffselementepaar, dessen je zusammenwirkende Protrusionsführungsflächen sich beim Erreichen der therapeutischen Protrusion, entsprechend der Darstellung in Fig. 3 IV, in vollständigem gegenseitigen Eingriff befinden, dargestellt ohne Schienen, wobei die Protrusionsführungsflächen in den Varianten der Fig. 6 I - 6 III unterschiedliche Anstellwinkel zu der in einer Frontalebene liegenden Transversalrichtung der Eingriffselemente einnehmen, und zwar folgendermaßen:
- Fig. 6 I: Anstellwinkel null über die gesamte craniocaudale Erstreckung der Protrusionsführungsflächen;
- Fig. 6 II: der Anstellwinkel der Protrusionsführungsflächen geht von ungleich null am cranialen Ende zu null am caudalen Ende über;
- Fig. 6 III: Anstellwinkel ungleich null über die gesamte craniocaudale Erstreckung der Protrusionsführungsflächen.

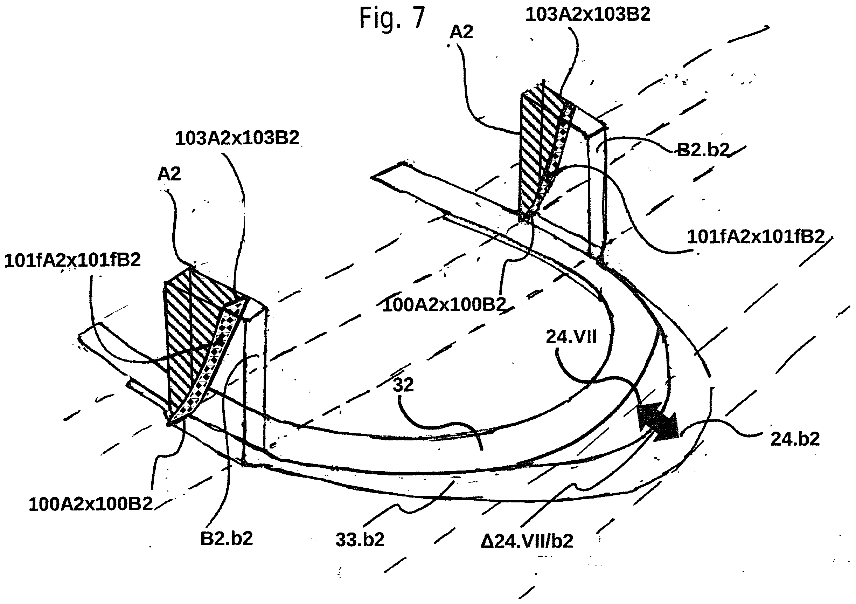

- Fig. 7: Die Eingriffselementepaare gemäß Fig. 6 II mit zugehörigen Schienen.

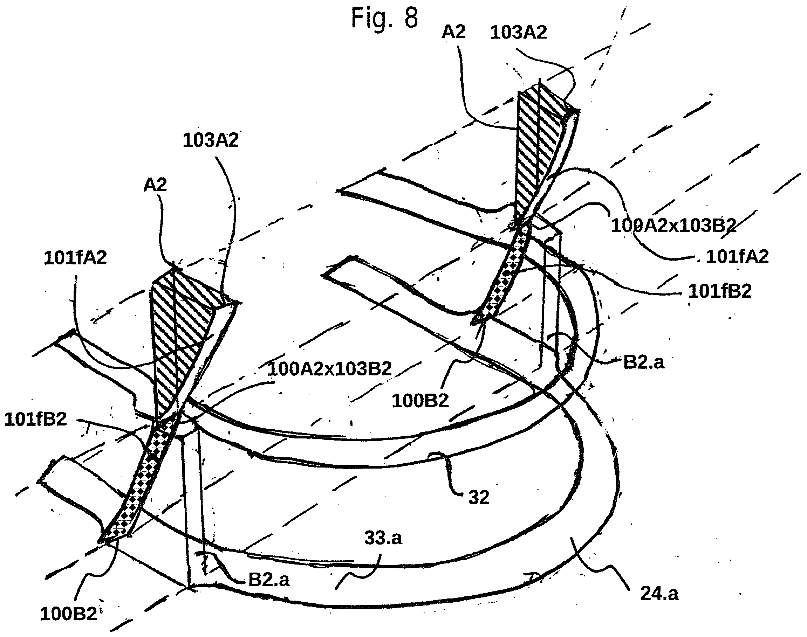

- Fig. 8: Die Anordnung gemäß Fig. 7, jedoch mit den Eingriffselementen in anfänglicher gegenseitiger Berührung bei habitueller Unterkieferöffnung, entsprechend Fig. 3 II.

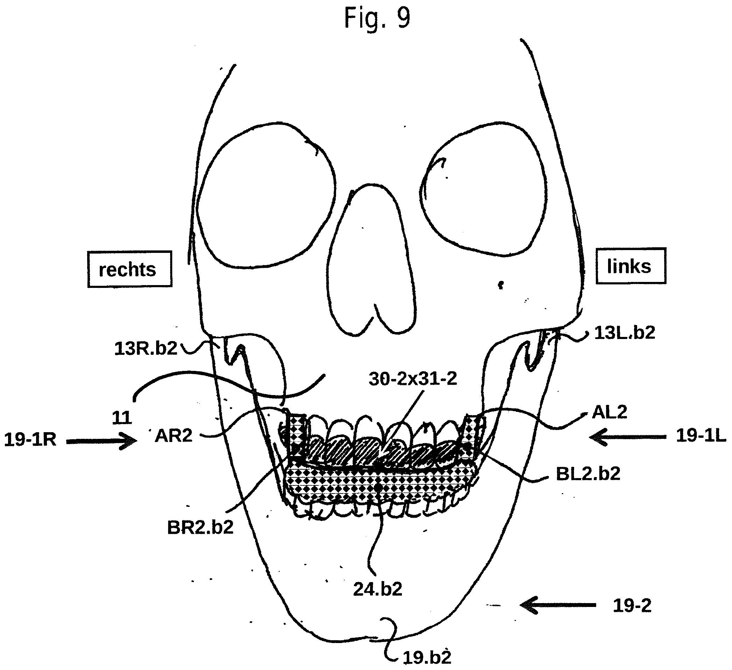

- Fig. 9: Eine Frontansicht eines Schädels mit eingesetzter UPS ohne Seitverschiebung des Unterkiefers.

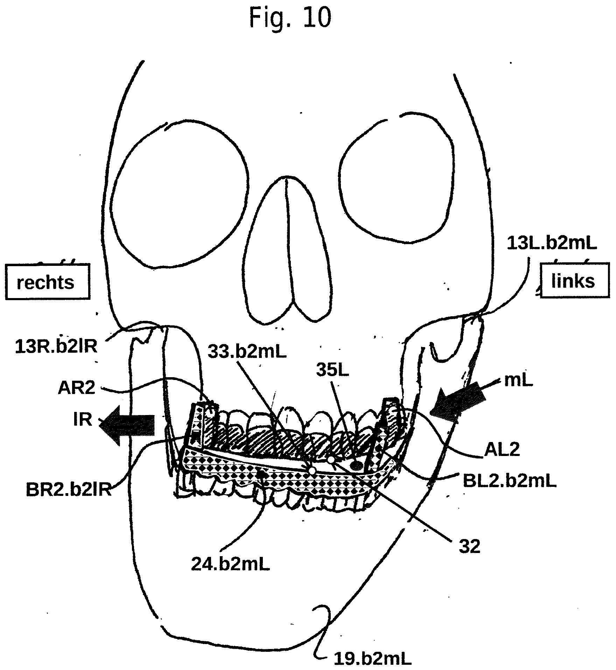

- Fig. 10: Eine Frontansicht gemäß Fig. 9 mit eingesetzter UPS, jedoch mit Seitverschiebung des Unterkiefers nach rechts (vom Patienten aus gesehen).

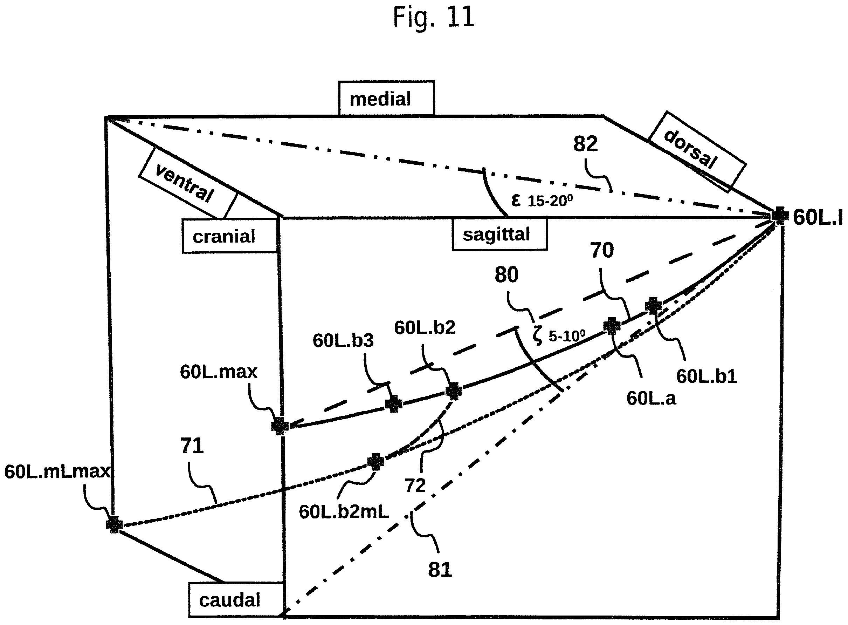

- Fig. 11: Kiefergelenkkopfzentrumbahnen bei Unterkieferprotrusion; darin bedeuten: durchgezogene Bahn 70: Unterkiefergelenkkopfzentrumbahn bei Unterkieferprotrusion in der sagittalen Ebene ohne Kieferseitbewegung; punktierte Bahn 71: Sagittale Unterkieferprotrusion mit gleichzeitiger Mediotrusionseitbewegung; gestrichelte Bahn 72: Mediotrusionsseitbewegung aus therapeutischer Unterkieferprotrusion (70 % der maximalen Unterkieferprotrusion) heraus; strichpunktierte Strecken 80 und 81: zwischen den Anfangs- und Endpunkten der Bahnen 70 bzw. 71 verlaufende Strecken, wobei die Strecke 80 wie die Bahn 70 in der durch den Ruhepunkt 60L.I des Kiefergelenkkopfzentrums gehenden Sagittalebene liegt, während die Strecke 81 eine Projektion der Bahn 71 auf diese Sagittalebene darstellt; strichdoppelpunktierte Bahn 82: zwischen den Endpunkten der Bahn 71 verlaufende Strecke 82, projiziert auf die durch den Ruhepunkt 60L.I des Kiefergelenkkopfzentrums gehende Horizontalebene; sowie der zwischen den Strecken 80 und 81 auftretende Winkel ζ bzw. der zwischen der Strecke 82 und der Sagittalebene auftretende Winkel ε.

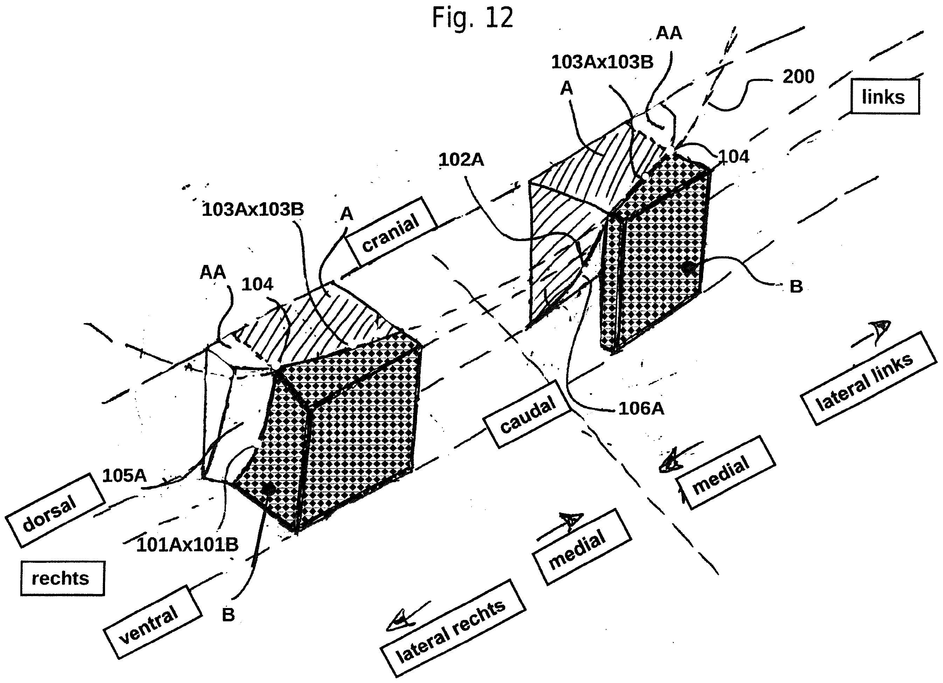

- Fig. 12: Ein rechtes und ein linkes Eingriffselementepaar bei der in Fig. 9 und in Fig. 2 in therapeutischer Protrusion gezeigten Unterkieferstellung, je mit Protrusionsführungsbahnen und je zusätzlich mit Mediotrusionsführungsbahnen und mit Laterotrusionsführungsbahnen, bei vollständigem gegenseitigen Protrusionseingriff und ohne Seitbewegung des Unterkiefers, dargestellt ohne Schienen.

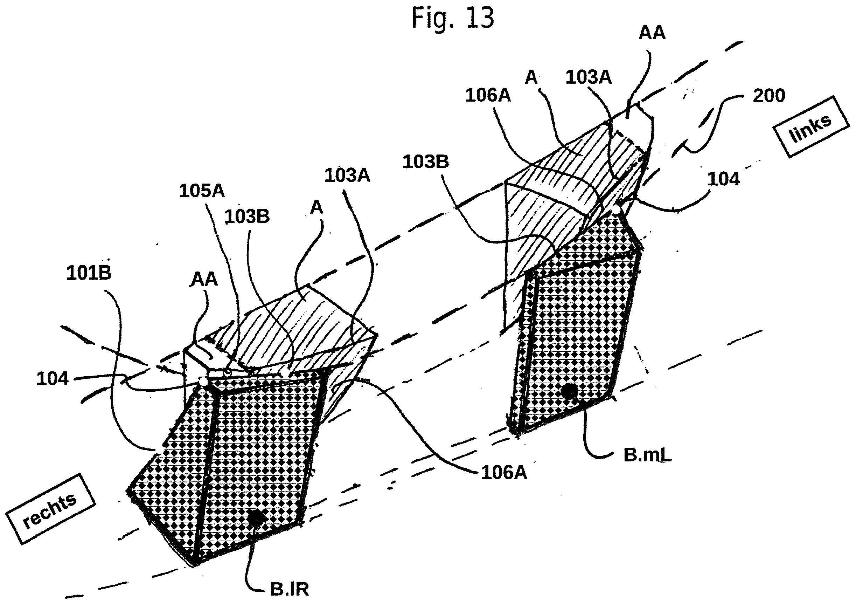

- Fig. 13: Die Eingriffselementepaare gemäß Fig. 12 mit vollständigem gegenseitigen Protrusionseingriff, jedoch nach einer zu Mediotrusion des linken Eingriffselementepaares und zu Laterotrusion des rechten Eingriffselementepaares führenden Seitbewegung des Unterkiefers in die in Fig. 10 gezeigte Unterkieferstellung, dargestellt ohne Schienen.

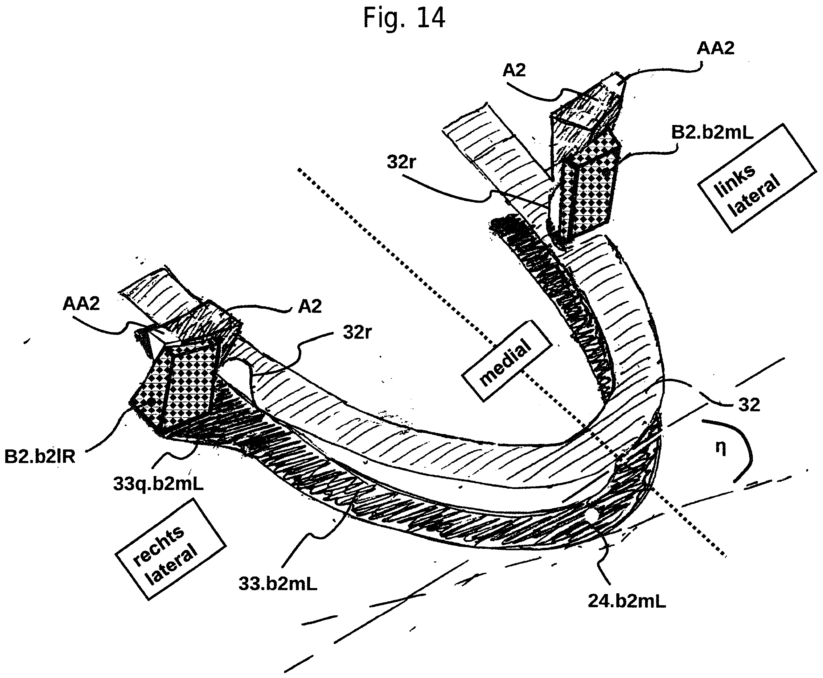

- Fig. 14: Die Eingriffselementepaare in ihren Positionen gemäß Fig. 13, dargestellt mit den zugehörigen Schienen.

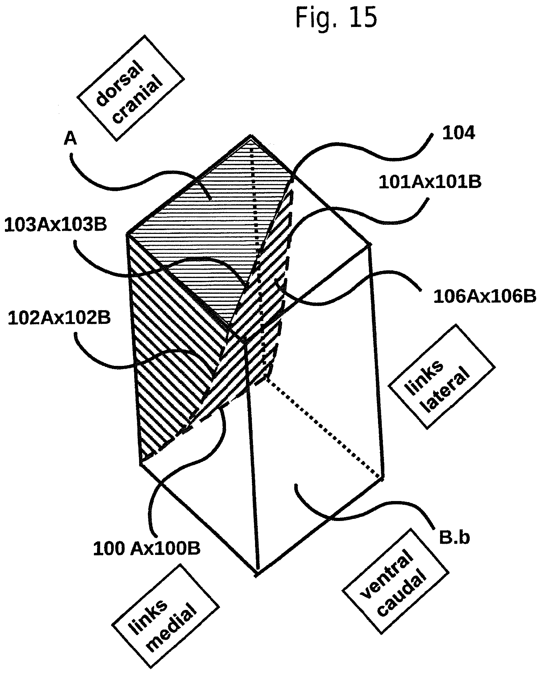

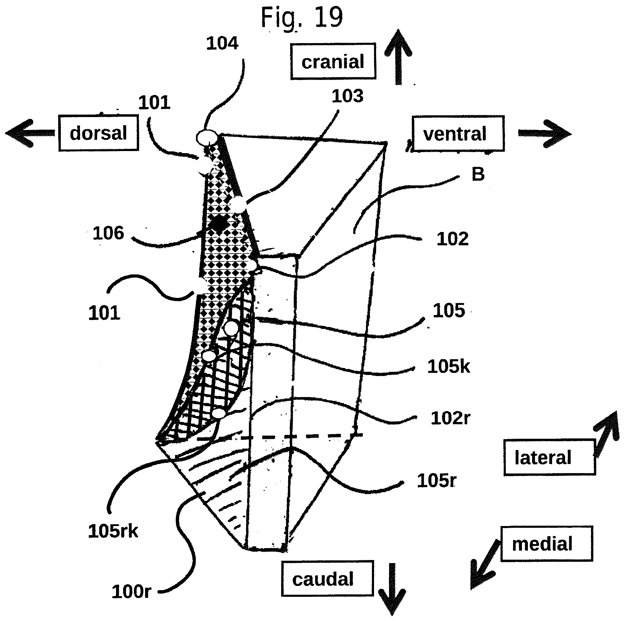

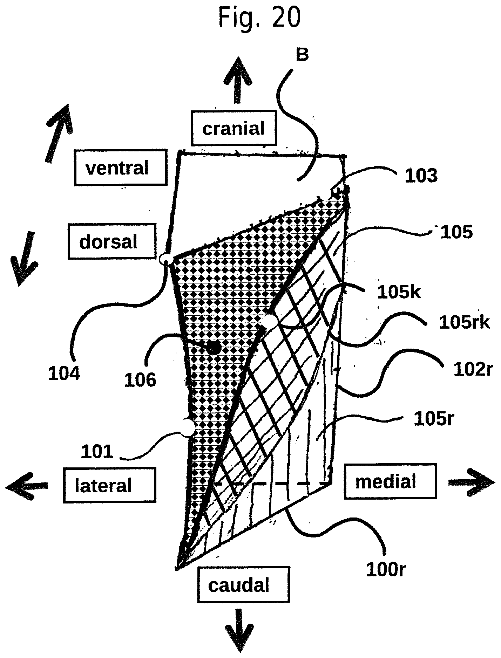

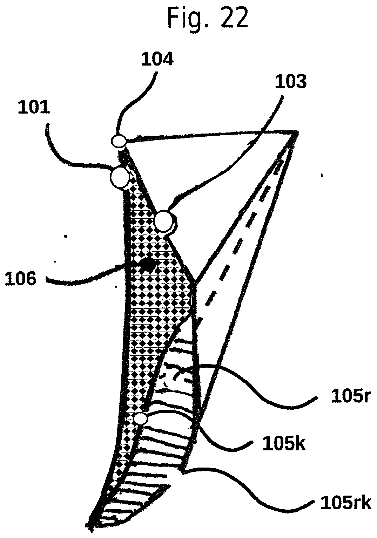

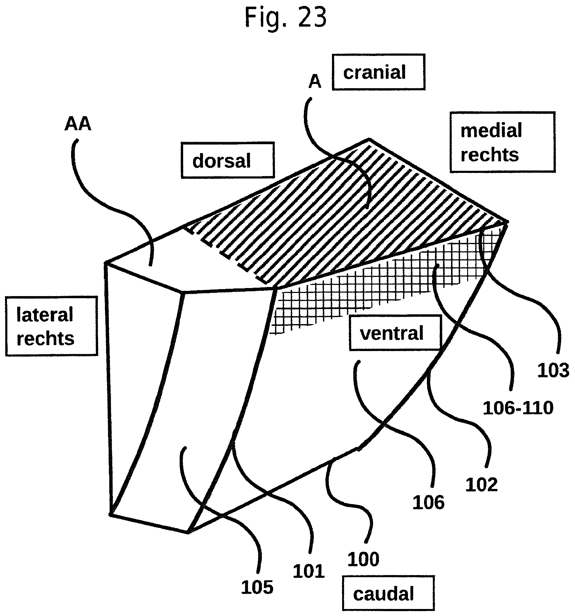

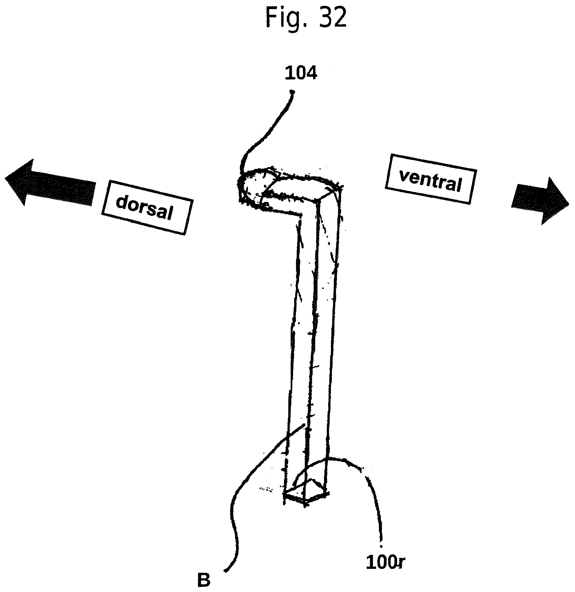

- Fig. 15 - 24, 31, 32: Anhand dieser Figuren erfolgt eine nähere Erläuterung zur Gestaltung der Protrusions-, Mediotrusions- und Laterotrusionsführungsbahnen der Eingriffselemente. Dargestellt sind jeweils die linksseitigen Eingriffselemente von medioventrocranial aus gesehen (Fig. 15 - 18, 21), von mediocranial aus gesehen (Fig.19, 22), von dorsocranial aus gesehen (Fig. 20) und die rechtsseitigen Eingriffselemente von lateroventrocranial aus gesehen (Fig. 23-24, 31) und des seitensymmetrischen/seitengleichen vorderen Eingriffselementes von lateroventrocranial aus gesehen (Fig. 32) .
- Fig. 15: Eine aus medioventrocranialer Sicht vorgenommene Perspektivdarstellung eines separat dargestellten linken Eingriffselementepaars mit vollflächig aneinanderliegenden, zusammen einen Kubus bilden Eingriffselementen.

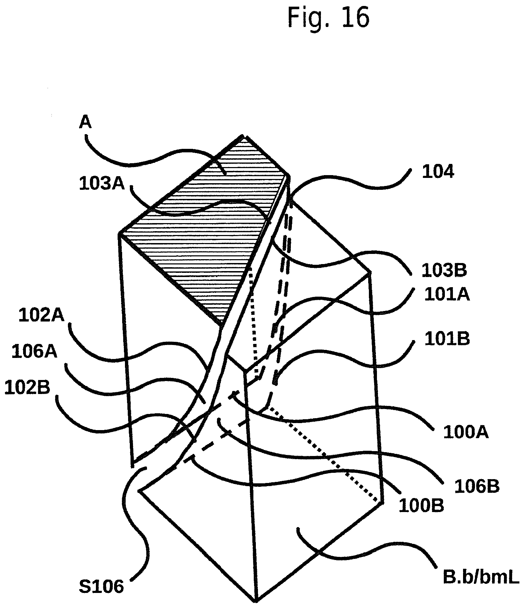

- Fig. 16: Das Eingriffselementpaar gemäß Fig. 15 nach caudal und dorsal gerichteter Verschiebung des vorderen Eingriffselementes - unter Wahrung der Parallelität von ventraler und dorsaler Fläche des Eingriffselementepaars - mit einer Verschiebung eines cranialen Oberpunktes 104 des vorderen Eingriffselementes entlang einer protrusionssteuernden Führungskante 101A des hinteren Eingriffselementes unter Verlassen des vollflächigen Kontakts der beiden Eingriffselemente.

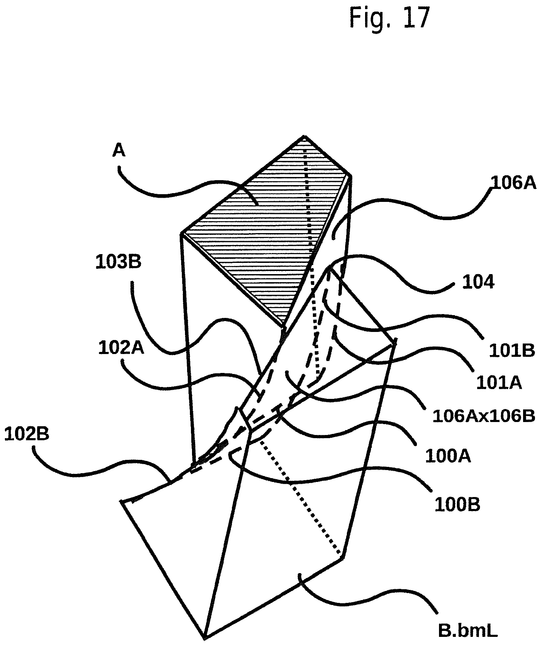

- Fig. 17: Das Eingriffselementpaar gemäß Fig 16, wobei das vordere Eingriffselement nach medial verschoben und durch eine Drehkippbewegung im Uhrzeigersinn (von medioventrocranial gesehen) in einen flächigen Kontakt der Führungsflächen der beiden Eingriffselemente gebracht wird.

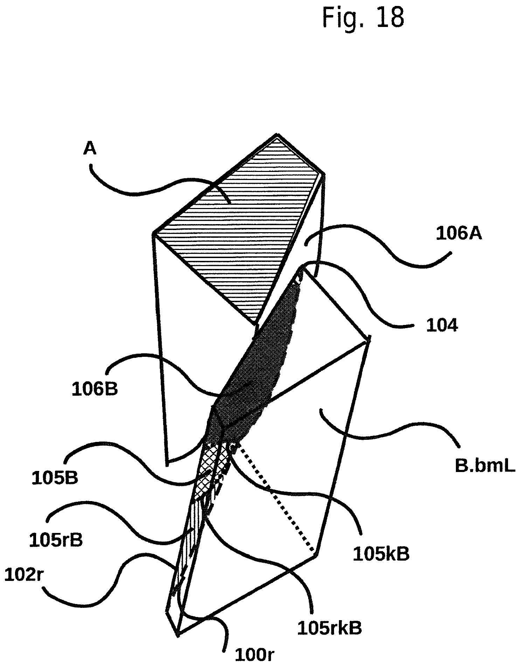

- Fig. 18: Das linke Eingriffselementpaar gemäß Fig. 17 und Unterkieferstellung nach einer Seitbewegung entsprechend Fig. 10, bei welchem die dorsale Führungsfläche des vorderen Eingriffselements eine gegenüber Fig. 17 unveränderte dorsolaterocraniale mediotrusionssteuernde Führungsfläche und eine daran nach mediocaudal angrenzende gegenüber Fig. 17 reduzierte Ausformung zur Schaffung einer laterotrusionssteuernden Fläche und Kante und einer daran nach mediocaudal angrenzenden vermehrt reduzierten nicht steuernden Fläche aufweist.
- Fig. 19: Das vordere Eingriffselement der Fig. 18 in Einzeldarstellung aus mediocranialer Sicht.
- Fig. 20: Das vordere Eingriffselement gemäß Fig. 19 mit einer medio- und laterotrusionssteuernden Fläche und einer durch die Reduzierung erhaltenen, nicht steuernden Fläche, zum Betrachter gedreht aus dorsocranialer Sicht.

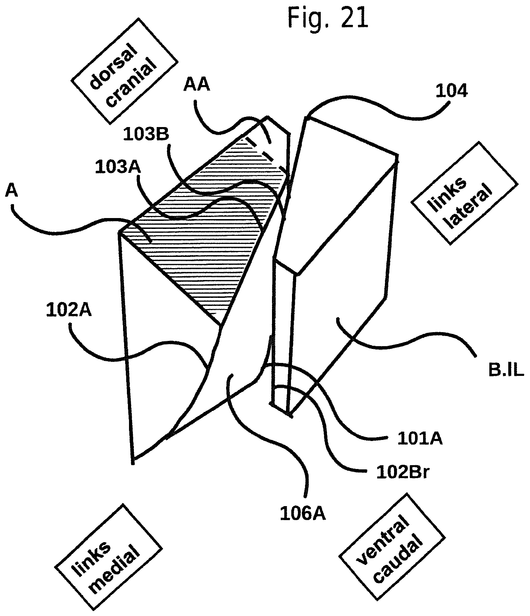

- Fig. 21: Das linke Eingriffselementepaar gemäß Fig. 18, jedoch mit einer laterotrusionssteuernden Fläche am hinteren Eingriffselement, in einer Laterotrusionstellung des vorderen Eingriffselements, und zwar spiegelbildlich asymmetrisch zum rechten Eingriffselement in Fig. 10.
- Fig. 22: Das vordere Eingriffselement gemäß Fig. 19 aus gleicher Sicht bei Reduzierung des Eingriffselementkörpers unter Erhaltung der steuernden Flächen und Kanten.
- Fig. 23: Das rechte hintere Eingriffselement gemäß Fig. 12, 13 und 14 in Perspektivdarstellung mit den steuernden Flächen und Kanten sowie mit Retentionselementen.

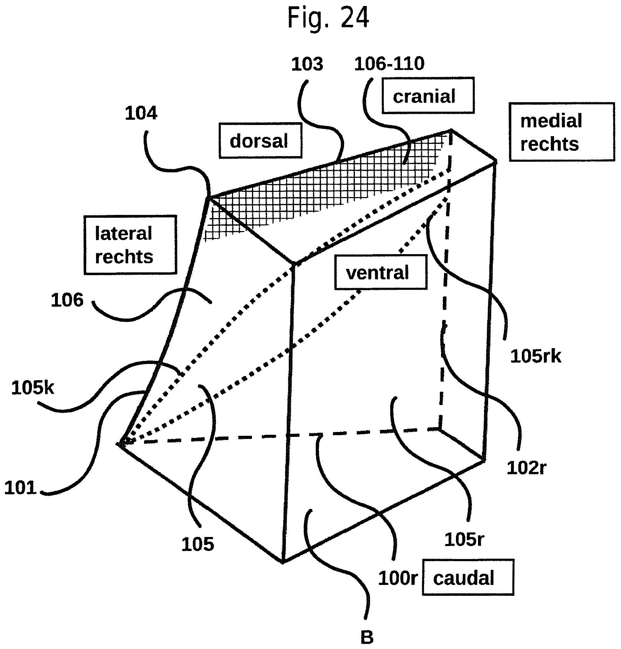

- Fig. 24: Das rechte vordere Eingriffselement gemäß Fig. 12, 13 und 14 in Perspektivdurchsichtdarstellung mit den steuernden Flächen und Kanten sowie mit Retentionselementen.

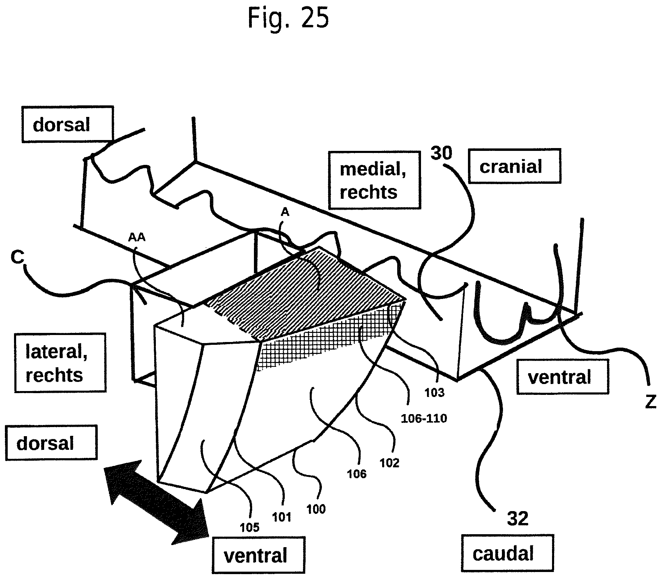

- Fig. 25: Das rechte hintere Eingriffselement gemäß Fig. 23 ohne Durchsichtzeichnung, mit der Oberkieferschiene über einen in sagittaler Pfeilrichtung einstellbaren Mechanismus (nicht dargestellt) verbunden.

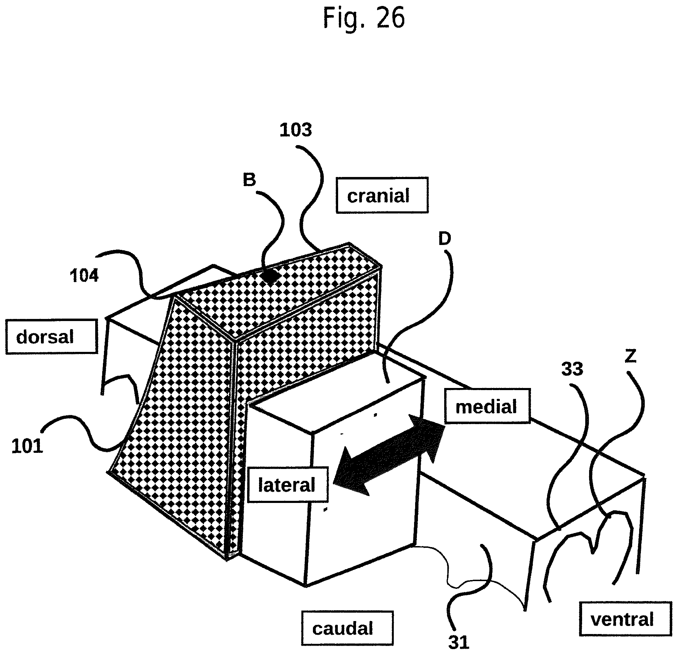

- Fig. 26: Das rechte vordere Eingriffselement gemäß Fig. 24 ohne Durchsichtzeichnung, mit der Unterkieferschiene über einen in transversaler Pfeilrichtung einstellbaren Mechanismus (nicht dargestellt) verbunden.

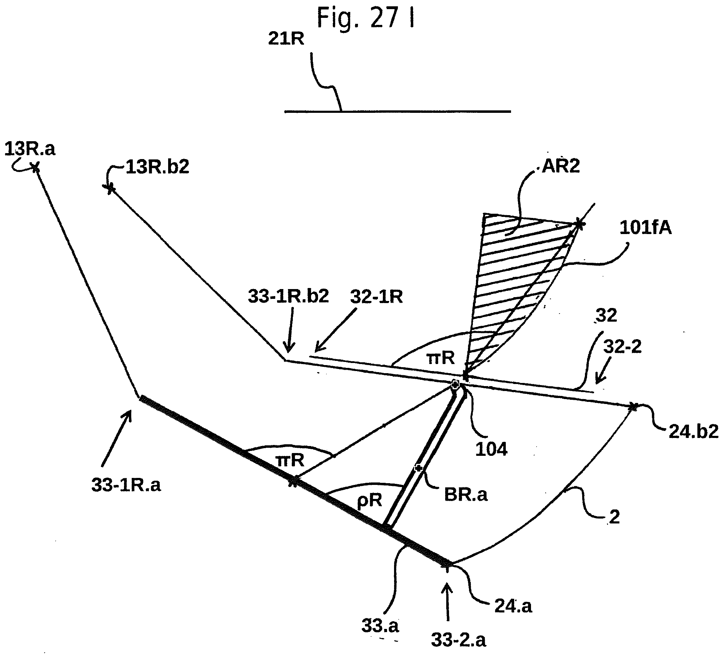

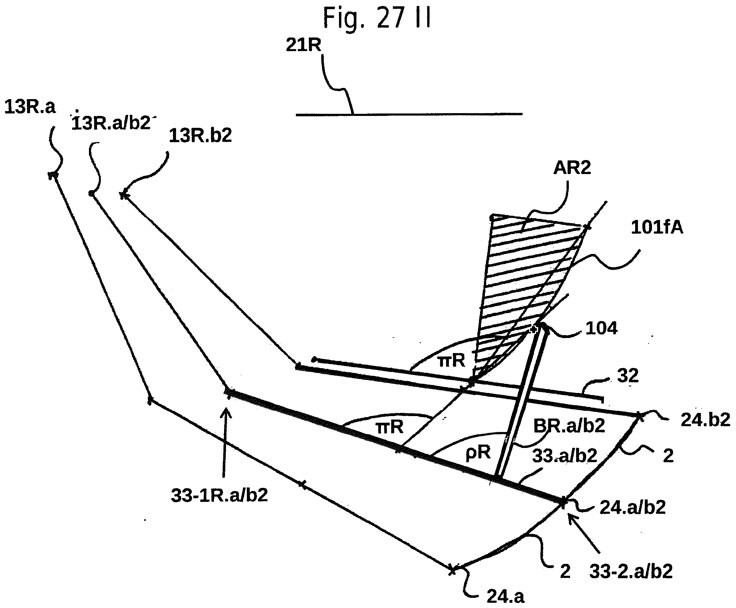

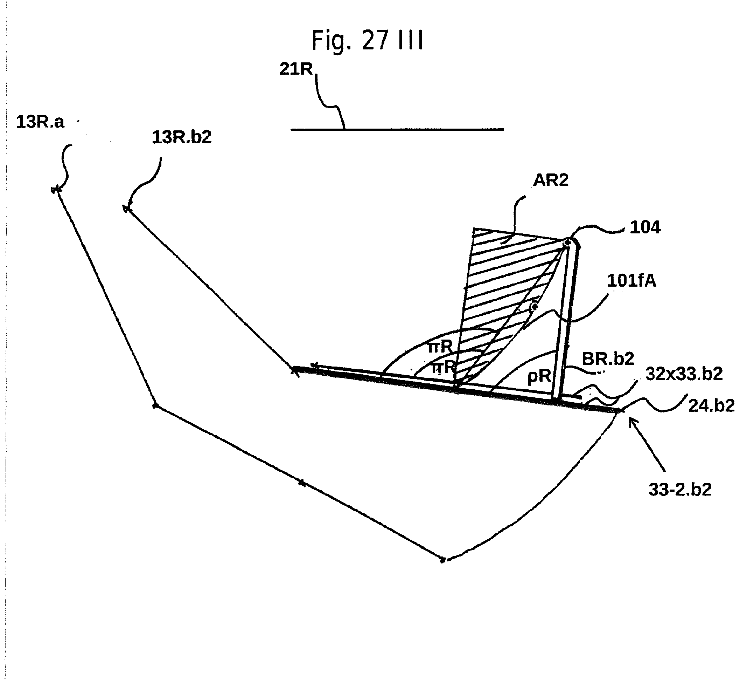

- Fig. 27 I - III: Extrem schematisierte Darstellungen des Kiefergelenkkopfzentrums, der Unterkieferschienenbasis, der Oberkieferschienenbasis und der Eingriffselemente in Positionen bei unterschiedlicher Unterkieferöffnung und Protrusion gemäß Fig. 3 II - IV, wobei das vordere Eingriffselement auf einen Abtaststifte reduziert ist, welcher die Führungsfläche des hinteren Eingriffselementes abtastet, dargestellt in habitueller Unterkieferöffnung (Fig. 27 I), in therapeutischer Protrusion (Fig. 27 III) und in der Mitte zwischen diesen beiden Unterkieferpositionen (Fig. 27 II).

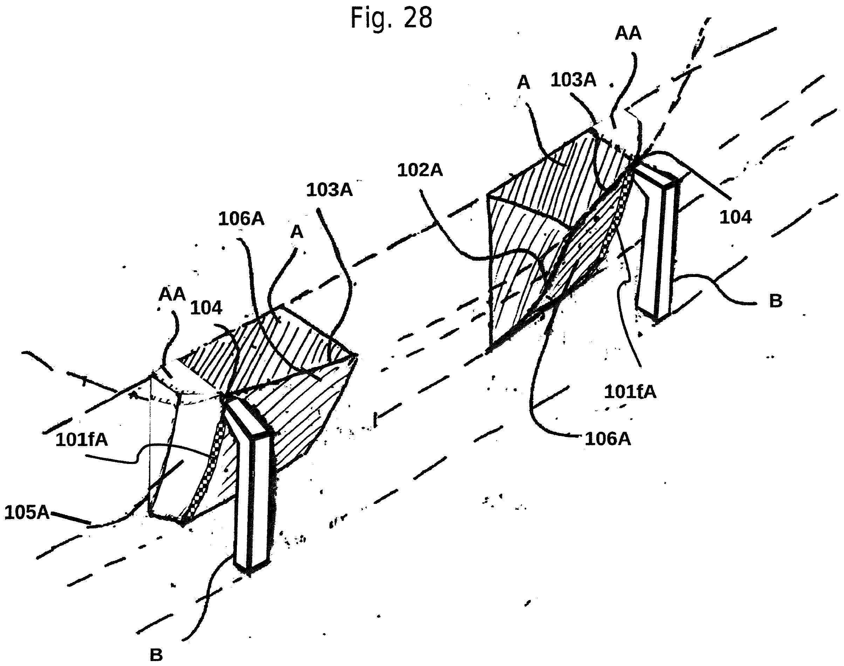

- Fig. 28: Ein Eingriffselementepaar gemäß Fig. 12 jedoch mit protrusionssteuernder Fläche, wobei die vorderen Eingriffselemente auf Abtaststifte reduziert sind, welche die Führungsflächen der hinteren Eingriffselemente abtasten, in der Darstellung unter Abtastung protrusionsteuernder Flächen der hinteren Eingriffselemente in therapeutischer Protrusionsstellung.

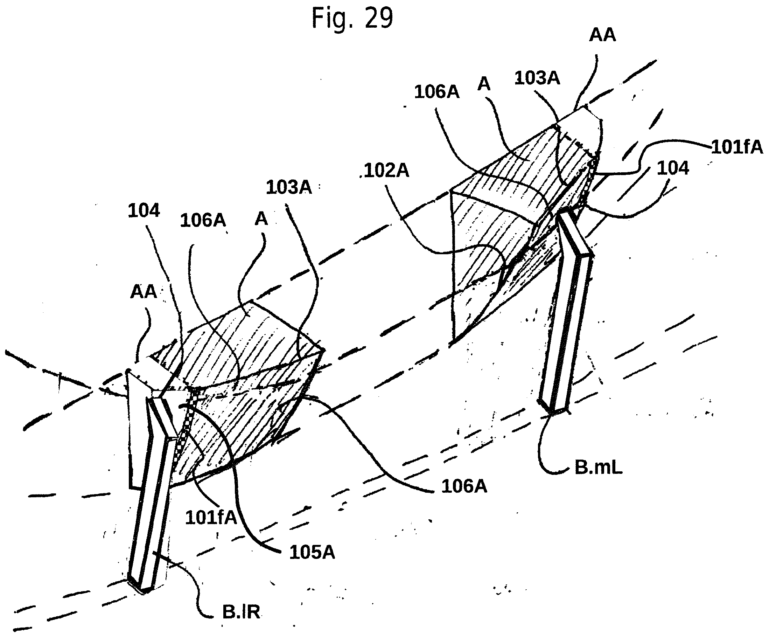

- Fig. 29: Ein Eingriffselementepaar gemäß Fig. 13, jedoch mit protrusionssteuernder Fläche, wobei die vorderen Eingriffselemente auf Abtaststifte reduziert sind, welche die Führungsflächen der hinteren Eingriffselemente abtasten, in der Darstellung unter Abtastung mediotrusionssteuernder Flächen auf der linken Kieferseite bzw. laterotrusionssteuernder Flächen auf der rechten Kieferseite der hinteren Eingriffselemente.

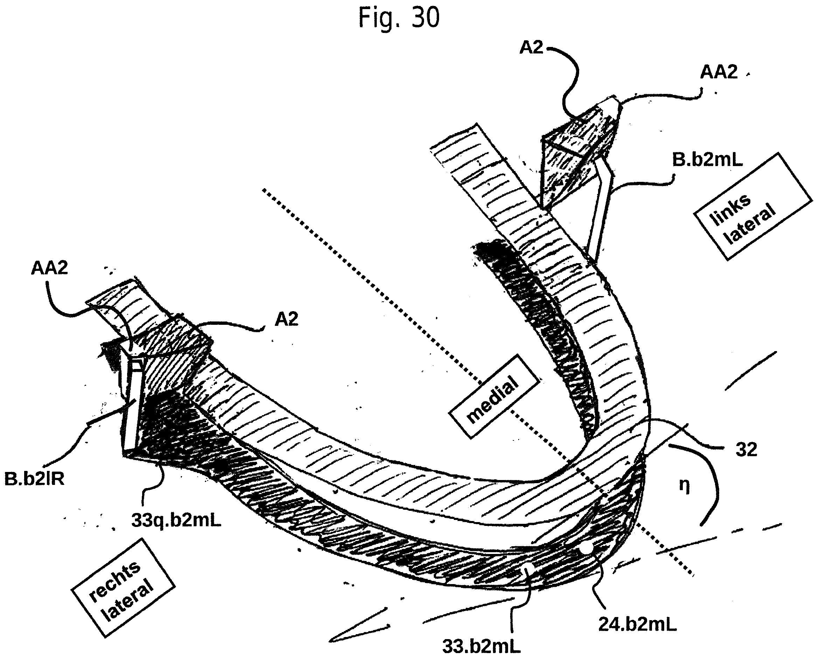

- Fig. 30: Eingriffselementpaare gemäß Fig. 29 bei Anordnung an den Schienen der UPS gemäß Fig. 14.

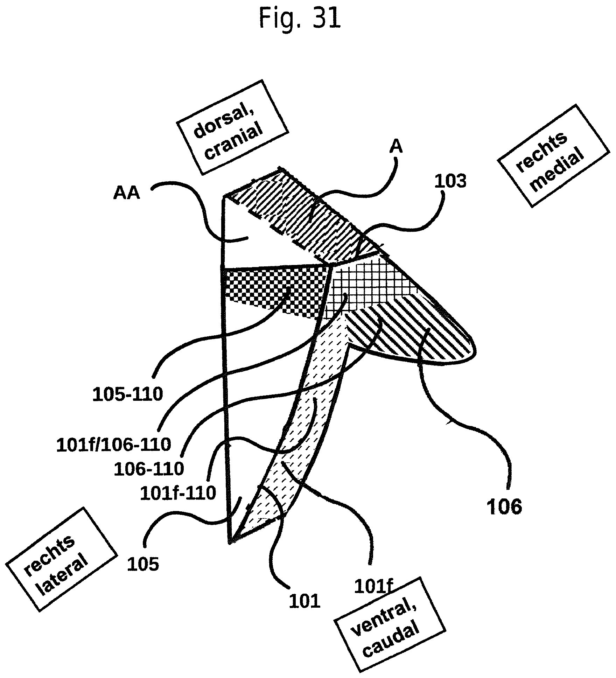

- Fig. 31: Eine Ausführungsform eines hinteren rechten Eingriffselementes gemäß Fig. 23 mit Reduzierung der medialen und laterodorsalen Anteile dieses Eingriffselementkörpers.
- Fig. 32: Eine seitenunabhängige Ausführungsform eines als Abtaststift ausgebildeten vorderen Eingriffselementes.

Die hier verwendeten Bezugszeichen unterliegen einer Systematik, mittels welcher in üblicher Art Elemente und Komponenten generell bezeichnet werden, zusätzlich aber durch Bezugszeichenzusätze bestimmte räumliche Positionen an solchen Elementen und Komponenten, Zuordnungen zu bestimmten Bewegungsbahnen, Bahnpunkten und Positionspunkten sowie Positionsbewegungen gekennzeichnet werden. So werden beispielsweise Elemente und Komponenten, die kieferseitig links oder rechts liegen, mit dem Bezugszeichenzusatz R bzw. L gekennzeichnet. Positionen, die sich ergeben, wenn sich der Unterkiefer im Inzisalpunkt 24 auf einer habituellen Bewegungsbahn a befindet, werden mit einem Bezugszeichenzusatz .a gekennzeichnet. Liegt der Inzisalpunkt 24 des Unterkiefers auf einer von mehreren möglichen Protrusionsbahnen 1, 2 oder 3 (nachfolgend näher erläutert) wird das mit einem entsprechenden Bezugszeichenzusatz .b1, bzw..b2 bzw..b3 gekennzeichnet. Eine bestimmte räumliche Position des Unterkiefers oder einer damit verbundenen Komponente wird mit einem Bezugszeichenzusatz "." und nach diesem Punkt mit der Angabe der räumlichen Position des Inzisalpunktes des Unterkiefers gekennzeichnet. Alle räumlichen Positionen der Bezeichnungen werden also nach der räumlichen Position des Inzisalpunktes gekennzeichnet. Und das Zurücklegen einer bestimmten Strecke zwischen zwei räumlichen Positionen wird mit dem Bezugszeichenzusatz "/" und der Angabe der Ausgangsposition vor "/" und der Angabe der Endposition nach "/" gekennzeichnet. Komponenten, die sich zwischen einem dorsalen Endpunkt und einem ventralen Endpunkt erstrecken, werden mit einem einstelligen Zusatz "-1" bzw. "-2" gekennzeichnet. Komponenten, die eine Oberflächenkonditionierung aufweisen, werden mit einem dreistelligen Zustaz "-110" gekennzeichnet. Einige der in der Beschreibung erwähnten Bezugszeichen sind in den Zeichnungen nur mit den Bezugszeichenzusätzen entsprechend der jeweils gezeigten Position, Bahn etc. angegeben.

Zusätzliche Information hierzu ist angegeben in einer tabellarischen "Bezugszeichen-Systematik", die am Ende der Beschreibung einer Bezugszeichenliste nachgestellt ist.

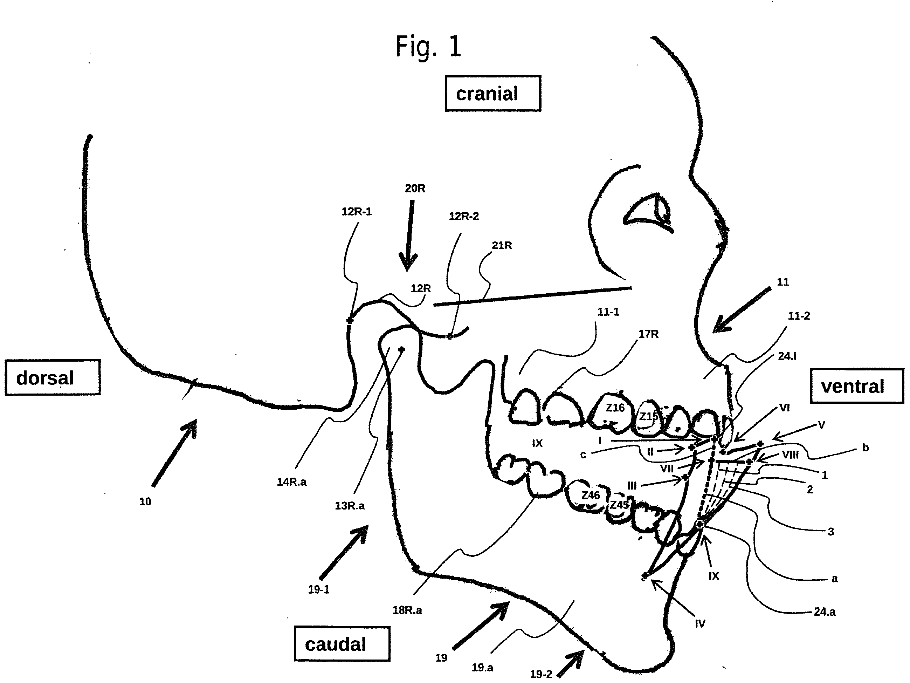

Fig. 1 zeigt eine Seitenansicht eines Schädels 10 in einer Sagittalebene, und zwar mit Blick auf dessen rechte Seite, mit einem Oberkiefer 11 und einem geöffneten Unterkiefer 19 in einer habituellen Unterkieferöffnungsposition 19.a, sowie oberkieferseitige obere Zähnen 17R mit den Zähnen Z15 und Z16 und unterkieferseitige unteren Zähne 18R mit den Zähnen Z45 und Z46, ferner ein rechtes Kiefergelenk 20R mit einem rechten Kiefergelenkkopf (Kondylus) 14R in einer Position 14R.a bei habitueller (entspannter) Unterkieferoffenstellung und einer mit diesem zusammenwirkenden rechten schädelseitigen Kiefergelenkbegrenzung 12R, verlaufend von einem hinteren Ende 12R-1 zu einem vorderen Ende 12R-2.

Ein Blick auf die (nicht gezeigte) linke Schädelseite sähe genauso aus, lediglich gespiegelt derart, dass das Auge links und der Hinterkopf rechts lägen, mit Darstellung der linken Kiefergelenkteile. Aufgrund der bei praktisch jedem Patienten bestehenden Kieferseitenasymmetrie hätten der Kiefergelenkkopf und die Kiefergelenkbegrenzung der linken Schädelseite allerdings eine von der rechten Schädelseite abweichende Form.

Dargestellt ist auch der Inzisalpunkt 24, an dem sich die Schneidekanten der beiden unteren mittleren Schneidezähne berühren, und zwar in einer Position 24.1 bei geschlossenem Unterkiefer mit maximalem Kontakt zwischen Ober- und Unterkieferzähnen und in einer auf einer Bahn a befindlichen Position 24.a bei habituell geöffnetem Unterkiefer.

In Fig. 1 sind verschiedene Bewegungsbahnen und Positionen I bis IX des Inzisalpunktes 24 dargestellt. Gezeigt sind sich ohne eingesetzte UPS ergebende Grenzbahnpositionen I bis VI, eine sich bei eingesetzten Schienen, aber ohne Eingriffselemente A, B ergebende habituelle Unterkieferöffnungsbahn a (punktierte Linie) und sich bei eingesetzter UPS, also mit Schienen und mit Eingriffselementen A, B ergebende therapeutische Protrusionsbahnen 1 bis 3 (gestrichelte Linien). Mit b ist eine therapeutische Protrusionsebene gekennzeichnet, in der der Inzisalpunkt 24 bei eingesetzten Schienen (ohne Eingriffselemente) und Kontakt zwischen den Schienen von einer habituellen Unterkieferposition VII auf der habituellen Unterkieferöffnungsbahn in die maximal mögliche therapeutische Protrusion VIII auf der Protrusionsgrenzbahn gelangt. c beschreibt eine habituelle Inzisalpunktbewegung von der maximalen Intercuspidation (maximaler Oberkiefer-Unterkiefer-Zahnkontakt) bis zu der von VII nach VIII verlaufenden therapeutischen Protrusionsebene b.

Folgende Inzisalpunktpositionen (24) werden bei folgenden Unterkieferstellungen erreicht:
I bei maximaler Intercuspidation (maximaler Kontakt zwischen Ober- und Unterkieferzähnen);
II bei maximaler Retrusion (maximale Rückstellung) des Unterkiefers unter (reduziertem) Ober- und Unterkieferzahnkontakt;
III nach überwiegender Rotationsbewegung des Unterkiefers in maximaler Retrusionsstellung des Kiefergelenks;
IV bei maximaler Unterkieferöffnung in maximaler Retrusionsstellung;
V bei maximaler Protrusion (Unterkiefervorschub) und (reduziertem) Kontakt zwischen den Unterkieferzähnen und den Oberkieferzähnen;
VI beim Kante-Kante-Kontakt der oberen und der unteren Frontzähne (Kopfbiss);
VII dorsaler Endpunkt der therapeutischen Protrusionsebene b auf der habituellen Unterkieferöffnungsbahn a gelegen;
VIII ventraler Endpunkt der therapeutischen Protrusionsebene b im Schnittpunkt mit der Grenzbahn I bis VI gelegen, bei maximaler Protrusion bei eingesetzter Oberkieferschiene und Unterkieferschiene;
IX Schnittpunkt zwischen der Protrusionsgrenzbahn (ab IV bis V) und der habituellen Unterkieferöffnungsbahn (a).

Wird der Unterkiefer aus einer habituellen Kieferöffnungsbahn a heraus, z.B. vom Inzisalpunktpunkt IX oder von dessen Nähe aus, in eine therapeutische Protrusion hinein (mittels einer in Fig. 1 noch nicht gezeigten UPS) geschlossen, führt das den Inzisalpunkt 24 auf einer von der gewählten therapeutischen Protrusion abhängigen Protrusionsbahn, von der in Fig. 1 drei Beispiele 1, 2 und 3 mit gestrichelten Linien dargestellt sind, zu einem Endpunkt auf der therapeutischen Protrusionsebene b.

Von II bis III führt der Kiefergelenkkopf 14R ganz überwiegend eine Rotation um einen Drehpunkt 13R ohne vergleichsweise nennenswerte Translationsbewegung (Gleitbewegung) für eine Protrusion und von III bis IV eine kombinierte Dreh- und Gleitbewegung durch. Wie noch näher erläutert werden wird, wird die UPS aufgrund der Dimension der die therapeutische Protrusion steuernden Eingriffselemente bei einer Unterkieferöffnung auf die Zähne aufgesetzt, bei welcher die Eingriffselemente noch nicht in Eingriff miteinander sind, also bei einer habituellen Unterkieferöffnung bei dem Schnittpunkt IX der habituellen Unterkieferöffnungsbahn a und der Grenzbahn (I-VI) oder in der Nähe dieses Schnittpunktes IX auf der habituellen Unterkieferöffnungsbahn a. Beim darauffolgenden Schließen des Mundes kommt es, sobald die Eingriffselemente bei einer Inzisalpunktbewegung auf der habituellen Unterkieferöffnungsbahn a aufeinander treffen, zum Beginn eines protrusionssteuernden Eingriffs der Eingriffselemente miteinander. Die Bahnen 1, 2, und 3 stellen mögliche therapeutische Protrusionsbahnen von der habituellen Unterkieferöffnungsbahn a aus in die therapeutische Protrusionebene b dar.

Auch weiterhin werden zur sprachlich kürzeren Darstellbarkeit eine Bewegung in eine therapeutische Protrusion als therapeutische Protrusionsbewegung und eine in eine therapeutische Protrusion führende Bewegungsbahn als therapeutische Bewegungsbahn bezeichnet, wobei noch einmal zur Klarstellung darauf hingewiesen sei, dass der therapeutische Effekt in der über die Protrusionsbewegung bzw. die Protrusionsbewegungsbahn erreichten Protrusion liegt und nicht in der Bewegung in die therapeutische Protrusion hinein.

Fig. 2 zeigt eine Schädel-Seitenansicht wie Fig. 1, jedoch zusätzlich mit dem Unterkiefer 19 in einer Schließstellung in der therapeutischen Protrusionsposition 19.b2 (also in einer Position des Unterkiefers, bei welcher der Inzisalpunkt 24 im Schnittpunkt der Bahn 2 mit der therapeutischen Protrusionebene b liegt) und mit einer UPS 29 mit einem auf die Zahnkronen oberer Zähne 17 aufgesetzten Oberkieferschienenkörper 30 und einem auf die Zahnkronen unterer Zähne 18 aufgesetzten Unterkieferschienenkörper 31. Seitlich neben den Kieferseiten sind am Oberkieferschienenkörper 30 obere hintere Eingriffselemente AR2 und am Unterkieferschienenkörper 31 vordere rechte Eingriffselemente BR2 angeordnet, die zusammen ein Eingriffselementepaar AR2, BR2 bilden. An ihren einander zugewandten Seiten sind die Eingriffselemente AR2 und BR2 (rechte Eingriffselemente AR und BR mit für die Bahn 2 gestalteten Führungsbahnen) mit später näher erläuterten Führungsbahnen versehen. Bei noch weiter geöffnetem Unterkiefer als in Fig. 2 dargestellt sind die vorderen Eingriffselemente BR2 außer Kontakt mit den hinteren Eingriffselementen AR2. Kommen die beiden Eingriffselemente AR2, BR2 in Kontakt miteinander, steuern sie von da ab die therapeutische Protrusionsbewegung in die therapeutische Protrusionsposition (mit einer Bewegung des Inzisalpunktes 24 von der Position 24.a in die Position 24.b2, bei einer Bewegung des vorderen rechten Eingriffselements von der Position BR2.a in die Position BR2.b2).

Die Führungsbahnen der Eingriffselemente sind derart gestaltet, dass bei dieser Schließbewegung der Unterkiefer 19 zunehmend nach vorne verschoben wird, bis er in der Unterkiefer-Schließposition 19.b2, in welcher sich das vordere Eingriffselement BR2 in der Position BR2.b2 befindet, die gewünschte Protrusionsposition erreicht hat. Der Inzisalpunkt 24 hat sich dabei von der Position 24.a zur Position 24.b2 bewegt, und zwar entsprechend der gewählten therapeutischen Protrusionsposition auf beispielsweise der Protrusionsbahn 2. Das Zentrum des rechten Kiefergelenkkopfzentrums 13R hat sich dabei von der Position 13R.a in die Position 13R.b2 bewegt. In dieser Protrusionsstellung befindet sich der Inzisalpunkt 24 in der Position 24.b2 und weist gegenüber den Schneidezähnen 17 des Oberkiefers 11 eine in Fig. 2 gezeigte Protrusion auf.

Diese Schließ- und Protrusionsvorgänge werden nun anhand der stark schematisierten Figuren 3 I bis 3 IV noch näher erläutert.

Wie anhand Fig. 3 I dargestellt, kommt es bei der habituellen Unterkieferöffnung durch die Rotation und Gleitbewegung im Kiefergelenk zu einer Translation des Kiefergelenkkopfzentrums in der Sagittalen (wie die sagittale Ebene auch genannt wird) von einer Position 13R.I nach ventral und caudal in eine Position 13R.a. Dabei wird der Inzisalpunkt 24 von der Position 24.1 nach 24.a verschoben und wandert in der Sagittalen nach dorsocaudal. Erreicht der Inzisalpunkt 24.a nicht die Grenzbahn im Punkt IX, gleitet das Kiefergelenkkopfzentrum in der therapeutischen Protrusionsbahn 1 in der Sagittalen nach dorsocranial und in der therapeutischen Protrusionsbahn 2 und 3 nach ventrocaudal. Bei einer reinen Rotation des Kiefergelenks in der habituellen Unterkieferöffnungsbahn a ergibt sich eine Kreisbahn Rad13R.a. Liegt die therapeutische Protrusionsbahn innerhalb der Kreisbahn, wie die therapeutische Protrusionsbahn 1, wandert das Kiefergelenkkopfzentrum in der Sagittalen nach dorsocranial in die Position 13R.b1. Liegt die therapeutische Protrusionsbahn vom Kiefergelenkkopfzentrum gesehen außerhalb (d. h. auf der ventralen Seite) der Kreisbahn Rad13R.a, wandert das Kiefergelenkkopfzentrum nach ventrocaudal in die Position 13R.b2 bzw. 13R.b3.

In den Fig. 3 II bis 3 IV sind drei verschiedene Unterkieferstellungen mit den zugehörigen Eingriffselementestellungen dargestellt, die den Fall betreffen, dass die therapeutische Protrusionsbahn außerhalb der Kreisbahn Rad13R.a liegt, also wie beispielhaft oben beschrieben auf der therapeutischen Protrusionsbahn 2 . Dabei ist bei der jeweils betrachteten Unterkieferschienenstellung die Basis 33 der Unterkieferschiene jeweils mit dickeren Linien gekennzeichnet. Da nur der Unterkiefer 19 beweglich ist, bleiben der Oberkieferschienenkörper 30 und das daran angeordnete hintere rechte Eingriffselement AR2 in unveränderter Position. Zusammen mit dem Unterkiefer beweglich ist nur das vordere rechte Eingriffselement BR2. Auch in diesen Figuren sind die Schienenbasen 33 und 32 des Unterkieferschienenkörpers 31 bzw. des Oberkieferschienenkörpers 30 der UPS 29 lediglich aus zeichnerischen Gründen in der therapeutischen Protrusionsposition b2 wieder mit einem kleinen Abstand voneinander dargestellt. Bei einer Bewegung der Unterkieferschienenbasis 33 aus der Lage 33.a bei habitueller Unterkieferöffnung mit der Inzisalpunktposition 24.a heraus in eine Lage 33.b2 mit einer Unterkieferstellung in der therapeutischer Protrusionsposition mit der Inzisalpunktposition 24.b2 findet die bereits erwähnte translatorische Bewegung des Kiefergelenckopf-Drehpunktes von 13R.a nach 13R.b2 statt.

Das hintere Eingriffselement AR2 weist auf seiner Vorderseite eine konvexe Führungsbahn 101fA auf und das vordere Eingriffselement BR2 weist auf seiner Rückseite eine konkave Führungsbahn 101fB auf, die komplementär zueinander in gleicher Weise gekrümmt sind. Gedachte Linien zwischen den beiden Endpunkten der jeweiligen Führungsbahn weisen gegenüber der Basis der je zugehörigen Schiene 32 bzw. 33 den gleichen Anstellwinkel π auf.

Die Krümmung der therapeutischen Protrusionsbahn für ein Hineinführen des Unterkiefers in eine therapeutische Protrusion ist nicht nur vom Ort der Messung entlang des Kiefers abhängig sondern ist auch von Patient zu Patient und für die rechte und linke Kieferseite verschieden. Daher wird für die Herstellung einer erfindungsgemäß ausgebildeten UPS beim jeweiligen Patienten an demjenigen Ort entlang des Kiefers, an dem sich später die Führungsbahnen der Eingriffselemente befinden sollen, die in die therapeutische Protrusion führende Protrusionsbahn für jede Kieferseite separat gemessen und werden die Krümmungen der Führungsbahnen 101fA und 101fB für jede Seite getrennt entsprechend dieser Messung gestaltet (hier vollfunktionelle endwertige Gestaltung genannt).

Orientiert man sich bei der Gestaltung der Krümmung der Führungsbahnen der Eingriffselemente, die vorzugsweise an der Stelle zwischen den Molaren (Z16, Z46) und Prämolaren (Z15, Z45) positioniert werden, an der therapeutischen Protrusionsbahn gemessen am Ort des Inzisalpunktes 24, bewirken (solange nicht die oben erwähnte Methode der halbfunktionellen Gestaltung angewendet wird) die Eingriffselementen AR, AB eine physiologisch ungünstige Unterkieferprotrusion. Gestaltet man die Führungsbahnkonturen der Eingriffselemente gar derart, dass sie parallel zur Protrusionsgrenzbahn verlaufen, entsprechend der Lehre der EP 1 094 761 B1 (Spalte 5, Zeilen 41-46; Spalte 6, Zeilen 54-57; Anspruch 1, Zeilen 31-37), steht dies den physiologischen Gegebenheiten des Kiefergelenks noch mehr entgegen.

Wie bereits erwähnt ist die physiologisch therapeutische Protrusionsbahn bei einer bestimmten therapeutischen Protrusion nicht nur patientenindividuell verschieden und hängt nicht nur vom entlang des Kiefers gewählten Ort ab sondern ist zudem für das rechte Kiefergelenk und das linke Kiefergelenk verschieden. Denn bedingt durch funktionelle Seitenunterschiede (z. B. neuronale Aktivierung der Muskulatur) und anatomische Seitenunterschiede (z. B. knöcherne Konfiguration der Kiefergelenke) ist der Verlauf der Vorschub-Gleit-Translationsbewegung im Raum nicht symmetrisch.

Zu physiologisch besonders guter Verträglichkeit einer UPS kommt man daher dann, wenn man die patientenindividuelle therapeutische Protrusionsbahnmessung am entlang des Kiefers für die Eingriffselemente gewählten Ort und für jede Kieferseite und damit für beide Kiefergelenke gesondert durchführt und die Führungsbahnen der rechten Eingriffselemente AR, BR entsprechend der Messung für das rechte Kiefergelenk und die Führungsbahnen der linken Eingriffselemente AL, BL entsprechend der Messung für das linke Kiefergelenk individuell gestaltet (vollfunktionelle endwertige Methode).

Fig. 3 II bezieht sich auf eine habituelle Unterkieferöffnung, bei welcher der caudale Endpunkt des hinteren Eingriffselementes AR und der craniale Oberpunkt 104 des vorderen Eingriffselementes BR beim Beginn des gegenseitigen Führungseingriffs in Berührung kommen. Zum Einsetzen der UPS 29 wird der Mund weiter als in Fig. 3 II dargestellt geöffnet, damit sich die vorderen und hinteren Eingriffselemente dabei nicht gegenseitig stören.

Beim Schließen des Unterkiefers aus der habituell geöffneten Stellung gemäß Fig. 3 II in die protrusionsgesteuerte Schließstellung der therapeutischen Position gemäß Fig. 3 IV findet - wie bei der in Fig. 3 III dargestellten Zwischenstellung 33.a/b2 der Unterkieferschiene - die Protrusionssteuerung nur durch eine Führungsberührung des cranialen Oberpunktes 104 des vorderen Eingriffselementes BR2 an der Führungsbahn 101fA des hinteren Eingriffselements AR2 statt. Dies ermöglicht eine Protrusionssteuerung mit geringer Gleitberührung. Dadurch wird ein protrusionsgesteuertes Schließen des Unterkiefers mit entsprechend geringer Reibung ermöglicht, also ein Schließen des Unterkiefers mit für den Patienten angenehm geringem Kraftaufwand. Dagegen liegen bei einer UPS gemäß EP 1 094 761 B1 die zusammenwirkenden Führungsflächen während des gesamten Schließvorgangs vollflächig aufeinander, mit entsprechend höherer Reibung beim Hineinführen in die Unterkieferprotrusion und mit entsprechend höherem Kraftbedarf beim Schließen des Unterkiefers.

Dies erklärt sich aus den unterschiedlichen Vorgaben für die Gestaltung der Führungsbahnen gemäß der vorliegenden Erfindung einerseits und gemäß EP 1 094 761 B1 andererseits. Im Fall der vorliegenden Erfindung werden die Führungsbahnen der Eingriffselemente entsprechend den Protrusionsbewegungsbahnen am Ort der Führungsbahnen gestaltet. Und diese beschreiben keinen Kreisabschnitt sondern einen Abschnitt aus einer asymmetrischen Ellipse, da der Kiefergelenkkopf beim Schließen des Unterkiefers aus der Position 13R.a heraus eine Rotationsbewegung und eine Translationsbewegung vollführt. Zwei spiegelsymmetrische Teile einer asymmetrischen Ellipse, wie sie durch die sich gegenüber liegenden Führungsflächen 101fA von AR2 und 101fB von BR2 gebildet werden, können nicht unter ständigem vollständigen Kontakt bis zum vollständigen Aufeinanderliegen verschoben werden. Dieser entsteht erst beim Erreichen der therapeutischen Protrusion, also bei vollständiger Überlappung von 101fA und 101fB (101fAx101fB). Im Fall der EP 1 094 761 B1 hingegen sollen die Führungsbahnen der Eingriffselemente parallel zur inzisalen Protrusionsgrenzbahn verlaufen. Diese wird als kreisförmig dargestellt, wohl unter der Annahme, dass bei einer Unterkieferschließbewegung unter maximaler Protrusion entlang der Protrusionsgrenzbahn nur noch eine reine Rotationsbewegung ohne Translation des Kiefergelenkkopfzentrums auftritt. Deshalb haben die zusammenwirkenden Führungsbahnen der beiden Eingriffselemente je die Form eines Kreisabschnittes und gleiten daher während der gesamten Unterkieferschließbewegung in dem sich jeweils überlappenden Ausmaß immer vollflächig aneinander.

Erst im Wesentlichen bei der in Fig. 3 IV dargestellten Unterkieferschienenschließstellung in der therapeutischen Protrusionsposition b2 der erfindungsgemäßen UPS 29, in der sich die Schienenbasen flächig berühren (32x33.b2), liegen die Führungsflächen 101fA und 101fB der beiden Eingriffselemente AR2 und BR2 vollflächig aneinander (101fAx101fB). Erst ab da tritt eine entsprechend hohe Reibung zwischen den beiden Führungsflächen auf, mit entsprechend starker mechanischer Stabilisierung der Endposition der therapeutischen Unterkieferprotrusion.

Fig. 3 V zeigt eine geometrische Darstellung im Zusammenhang mit der Bestimmung von Führungsbahnen der Eingriffselemente mittels Errechnung aus Bewegungsbahnmessungen am Inzisalpunkt 24 und an mindestens einem der Kiefergelenkkopfzentren 13, jeweils aus der habituellen Unterkieferöffnung heraus in die therapeutische Protrusion, hier halbfunktionelle Methode genannt.

Hierbei wird für die Gestaltung der Führungsflächen der Eingriffselemente aus den therapeutischen Protrusionsbahnen, die während der therapeutischen Protrusionsbewegung einerseits am Ort des Inzisalpunktes 24 und andererseits am Punkt eines Kiefergelenkkopfzentrums 13 gemessen werden, die therapeutische Protrusionsführungsbahn am Ort des cranialen Oberpunktes 104 der jeweiligen Führungsfläche der Eingriffselemente errechnet. Da die Protrusionsbahn am Inzisalpunkt 24 aber nicht der Protrusionsbahn am Ort der Führungsflächen der Eingriffselemente entspricht, wird die Protrusionsbahn am Ort der Führungsflächen aus der Geometrie der Bewegungsbahn des Inzisalpunktes und der Bewegungsbahn mindestens eines der Kiefergelenkkopfzentren errechnet. Hierfür wird für einzelne zusammengehörende Punktepaare dieser beiden Bewegungsbahnen jeweils eine erste Strecke 22 zwischen dem in der medianen Sagittalebene liegenden Inzisalpunkt 24 und dem auf die mediane Sagittalebene projizierten Kiefergelenkkopfzentrum 13 ermittelt. Der Bezug zur Bewegungsbahn des cranialen Oberkantenpunktes 104 des vorderen Eingriffselementes wird hergestellt über den Winkel β zwischen der ersten Strecke 22 und einer zweiten Strecke 104x/24 zwischen dem Inzisalpunkt 24 und dem cranialen Oberkantenpunkt 104 und über den Winkel γ zwischen der ersten Strecke 22 und einer dritten Strecke 13/104x zwischen dem Mittelpunkt des jeweiligen Kiefergelenkkopfzentrums 13 und dem cranialen Oberkantenpunkt 104.

Soll die Kieferseitenasymmetrie nicht berücksichtigt werden, reicht es aus auf Basis einer solchen einseitigen Errechnung (halbfunktionelle endwertige links- oder rechtsseitige Methode) oder mittelwertigen Errechnung aus den beidseitiger Errechnungen (halbfunktionelle mittelwertige Methode) die Führungsflächen der beiden Eingriffselemente gleich jedoch spiegelbildlich zueinander zu gestalten. Soll auch die Kieferseitenasymmetrie berücksichtigt werden, errechnet man die Bewegungsbahn des cranialen Oberkantenpunktes 104 in der oben angegebenen Weise getrennt für jede Kieferseite, indem für die jeweilige Errechnung die Protrusionsbewegung des Kiefergelenkkopfzentrums 13 der linken Kieferseite bzw. der rechten Kieferseite berücksichtigt wird (halbfunktionelle endwertige seitenindividuelle Methode).

Alternativ hierzu sind den erläuterten geometrischen Berechnungen entsprechende Berechnungen auch an weiteren, von den Punkten 24 und 13 abweichenden Stellen am Unterkiefer möglich, die vom Punkt 104 distal in Richtung Kiefergelenkkopfzentrum 13 und vom Punkt 104 mesial in Richtung Inzisalpunkt 24 liegen, oder zwei Punkte, die vom Punkt 104 mesial liegen, oder zwei Punkte, die vom Punkt 104 distal liegen.

Fig. 4 zeigt eine Fig. 2 ähnliche Schädelansicht unter Weglassung der Eingriffselemente und mit Darstellung des Unterkiefers 19 lediglich in einer Schließstellung 19.b3 und mit gegenüber Fig. 2 stärkerer therapeutischer Unterkieferprotrusion, mit entsprechend weiterer Vorverlagerung des Inzisalpunktes von 24.b2 nach 24.b3 gegenüber den Schneidezähnen des Oberkiefers, gemäß Fig. 3 I.

Der Verlauf der Vorschubbewegung des Unterkiefers 19 unter Zahnkontakt wird normaler Weise anfänglich durch die Geometrie der Frontzähne (Frontzahnführung, von I nach VI in Fig. 1) und den Verlauf der Kiefergelenkbahnen bestimmt.

Nur spielt die Frontzahnführung dann keine Rolle mehr, wenn die Ober- und Unterkieferschienenbasen, wie bei 32 und 33.b2 in Fig. 2, plan aufeinander liegen und damit die Frontzahnführung bei Veränderung der Protrusion aufgehoben wird.

Mit einer zunehmenden therapeutischen Protrusion einher geht eine entsprechende Vorwärtsverschiebung (ventral) und Absenkung (caudal) des Kiefergelenkkopfzentrums 13 von 13.b2 nach 13.b3 gemäß Fig. 3I einher. Infolge der verstärkten Unterkieferprotrusion und des Verlaufs der oberen Begrenzung der Kiefergelenkbahn 12 und deren Neigung von 12R-1 nach 12R-2 gemäß Fig. 1 entsteht aus der Geometrie heraus ein keilförmiger Spalt 34 zwischen der unverändert positionierten Oberkieferschienenbasis 32 und der positionsveränderten Unterkieferschienenbasis 33.b3. Die Keilform dieses Spaltes 34 ist von der Stärke der gewählten therapeutischen Unterkieferprotrusion und von der Neigung der Kiefergelenkbahn abhängig.

Grundsätzlich gilt vereinfacht: Je steiler die Bahn des rechten und des linken Kiefergelenkkopfzentrums 13 ist, desto mehr nimmt der Abstand zwischen den Ober-und Unterkieferseitenzähnen von mesial nach distal hin während der Kieferkopfgleitbewegung zu (also von den vorderen Seitenzähnen zu den hinteren Seitenzähnen). Die Steilheit dieser Bahn ergibt sich aus deren Winkel zu einer Schädelbezugsebene 21, z. B. der Frankfurter Horizontalen.

Dieser Abstand zwischen den Ober- und Unterkieferseitenzähnen ist aufgrund der funktionellen und anatomischen Seitenunterschiede für die rechten und linken Zahnreihen allgemein und individuell bei den einzelnen Patienten nicht gleich sondern mehr oder weniger unterschiedlich.

Der Grad, mit dem der Kontakt aufgehoben wird, ist meistens während der Vorschubbewegung für beide Unterkieferseiten unterschiedlich. Bedingt ist dies durch den unterschiedlichen Verlauf der beiden Kiefergelenkkopfzentrumbahnen zueinander, wobei die von beiden steiler verlaufende Kiefergelenkkopfzentrumbahn den weiter distal gelegenen Anteil des Unterkiefers von der Vorschubbahn nach caudal abdrängt. Es handelt sich bei der Vorschubbewegung des Unterkiefers und damit seines Inzisalpunkts 24 von der Position 24.b2 in Fig. 2 in eine Position 24.b3 in Fig. 4 oder in eine Position auf der Strecke zwischen VII und VIII in Fig. 1 also räumlich gesehen grundsätzlich nicht um eine symmetrische Parallelverschiebung der beiden Schienen der UPS zueinander im Raum. Aus diesem Grunde wird auch der Verlauf von frei gewählten Punkten, die entlang der Oberfläche der Unterkieferschiene liegen, bei allen Unterkiefervorschubbewegungen seitenbezogen zueinander nie parallel bzw. symmetrisch erfolgen können. Bei den unterschiedlichen therapeutischen Vorschubpositionen sind die Eingriffselementepaare und zur mechanischen Entlastung der Eingriffselementepaare und zur gleichmäßigen Druckübertragung zwischen Oberkieferschienenbasis 32 und Unterkieferschienenbasis 33 bei Kieferpressbewegungen in der therapeutischen Protrusion die Kontaktfläche zwischen der Oberkieferschiene 30 und der Unterkieferschiene 31 für ein planes Aufeinanderliegen neu zu gestalten.

Es ist von Vorteil, wenn die Oberkieferschienenbasis 32 und die Unterkieferschienenbasis 33 ständig plan aufeinander liegen, auch wenn aus medizinischen Gründen eine Veränderung der Protrusion erforderlich ist. Treten hingegen aufgrund des keilförmigen Spaltes 34 Frühkontakte zwischen den vorderen Schienenteilen 30-2 und 31-2 der UPS 29 auf (Fig. 4), so kommt es durch den Zug der Kaumuskulatur zu einem Aneinanderpressen der kontaktfreien hinteren Schienenteile 30-1 und 31-1 und damit zu unphysiologischen Zugkräften auf das Kiefergelenk mit all ihren Nachteilen.

Gemäß einer vorteilhaften Ausführungsform der Erfindung wird ein ständiges plan Aufeinanderliegen der Schienenbasen 32 und 33 erreicht, indem der in Fig. 4 gezeigte keilförmige Spalt 34 mittels eines Distanzplättchens E kompensiert wird. Ein Ausführungsbeispiel dieser Ausführungsform wird mittels der stark schematisierten Fig. 5 und deren Ansichten A, B, C und D erläutert, wie bei den vorausgehenden Figuren wieder anhand einer Draufsicht auf das rechte Kiefergelenk.

In Ansicht A ist eine Situation bei Erreichen der therapeutischen Protrusion ohne Darstellung eines Spaltes zwischen Oberkieferschienenbasis 32 und Unterkieferschienenbasis 33.b2 gezeigt. Die protrusionsteuernd zusammen wirkenden Führungsbahnen des Eingriffselementepaares AR2 und BR2.b2 liegen vollständig aneinander (101fAx101fB). Diese Führungsbahnen sind in Fig. 5 zur einfacheren Darstellung mit linearen Anstiegen gezeigt und nicht mit ihren eigentlich gekrümmten Anstiegen, wie in den Figuren 2 bis 3 IV gezeigt.

Ansicht B zeigt den in Fig. 4 dargestellten nach distal hin zunehmenden keilförmigen Spalt 34 zwischen Oberkieferschienenbasis 32 und Unterkieferschienenbasis 33.b3 bei Weglassung der Eingriffselemente unter Veränderung der Neigung der Strecke von 13R.b2/24.b2 nach 13R.b3/24.b3 in Fig. 3I. Bei Bewegungen des Inzisalpunktes 24 auf therapeutischen Protrusionsbahnen 2 oder 3, die vom Kiefergelenkkopfzentrum aus gesehen außerhalb der Kreisbahn Rad13R.a liegen, ergibt sich der in Ansicht B dargestellte nach distal hin zunehmende keilförmige Spalt 34.

Ansicht C zeigt - wieder bei Darstellung des vollständigen Erreichens der therapeutischen Protrusionsposition - ein den Spalt 34 kompensierendes Distanzplättchen E. Das vordere Eingriffselement BR3.b3 ist dabei, wie BR2.b2 in Fig. 5A, an der Basis der Unterkieferschiene, hier 33.b3, angeordnet. Erwähnt sei hier, dass die Eingriffselemente für einen verstärkten mechanischen Verbund mit der Schiene nicht nur mit der Schienenbasis sondern auch mit dem Schienenkörper verbunden werden können und dies zur besseren Darstellung der Figuren nicht in allen Figuren ersichtlich ist. Die zuvor erwähnten unphysiologischen Zugkräfte auf das Kiefergelenk treten daher nicht mehr auf.

Zusätzlich zur Spaltauffüllung, bedingt durch die neue therapeutische Protrusionsposition b3, sind für die entsprechend neue therapeutische Protrusionsbahn 3 neue Eingriffselemente AR3 und BR3 zu gestalten.

Ansicht D zeigt eine Situation bei Bewegung des Inzisalpunktes auf einer therapeutischen Protrusionsbahn 1, die vom Kiefergelenkkopfzentrum aus gesehen innerhalb der Kreisbahn Rad13R.a liegt. In diesem Fall ergibt sich ein nach mesial hin zunehmender keilförmiger Spalt 34 zwischen Oberkieferschienenbasis 32 und Unterkieferschienenbasis 33.b1, bei Weglassung der Eingriffselemente unter Veränderung der Neigung der Strecke von 13R.b2/24.b2 nach 13R.b1/24.b1 in Fig. 3I. In diesem Fall wurde also nicht der Unterkiefer gemäß Ansicht A gemäß Ansicht B vorgeschoben, sondern der Unterkiefer gemäß Ansicht A wurde gemäß Ansicht D zurückgeschoben mit dem daraus in umgekehrter Richtung stark ausgeprägten Spalt 34. Für den Fall D nicht dargestellt ist die Situation C, wie sie für den Fall B in Ansicht C dargestellt ist.

Das Phänomen der nicht seitensymmetrischen Abstandsveränderung zwischen Ober-und Unterkiefer während der Vorschubbewegung tritt ebenso bei eingesetzten plan aufeinanderliegenden Ober- und Unterkieferschienenbasen zweiteiliger UPS auf, die den Biss so wenig wie möglich sperren, also das vollständige Zusammenbeißen der Zähne so wenig wie möglich blockieren (möglichst geringe Bisssperrung). Aufgrund der Asymmetrie zwischen rechtem und linkem Kiefergelenk kommt es auf beiden Kieferseiten zu unterschiedlichen Formen des Spaltes 34. Es ist daher von Vorteil, an den beiden Kieferseiten entsprechend unterschiedlich geformte Distanzplättchen E anzuordnen.

Fig. 6 I bis 6 III zeigen je zwei Eingriffselementepaare mit einem rechten Eingriffselementepaar A, B auf der rechten und einem linken Eingriffselementepaar A, B auf der linken Kieferseite, deren für die Protrusionsteuerung je zusammenwirkende Führungsflächen 101fAx101fB mit Krümmungen versehen sind entsprechend den patientenindividuell gemessenen therapeutischen Protrusionsbewegungen am Ort der Eingriffselemente, die durch die Kieferkopfzentrumsbewegung und die Kieferbewegung bedingt sind. In Transversalrichtung verlaufen die Führungsflächen 101fA bzw. 101fB seitengleich gestaltet eben und je nach spezieller Ausführungsform parallel oder nicht-parallel zu vorderen Stirnflächen der beiden vorderen Eingriffselemente B der rechten und linken Seite. Dargestellt sind die Eingriffselementepaare in einer Stellung, in welcher die je zusammen wirkenden Führungsflächen 101fA bzw. 101fB vollflächig aneinander liegen (101fAx101fB) und die therapeutische Protrusionsposition erreicht ist. Die transversale Gestaltung der Führungsflächen gemäß Fig. 6 kann allerdings Kieferseitbewegungen aus der therapeutischen Protrusion heraus nicht unterstützen.

Die Fig. 6 I bis 6 III unterscheiden sich hinsichtlich der Anstellwinkel der Trennlinien 103Ax103B am cranialen Ende und der Trennlinien 100Ax100B am caudalen Ende. Bei der Ausführungsform gemäß Fig. 6 I sind die Anstellwinkel an beiden Enden gleich null. Bei der Ausführungsform gemäß Fig. 6 II ist der Anstellwinkel am cranialen Ende ungleich null und am caudalen Ende gleich null. Im Fall der Ausführungsform gemäß Fig. 6 III sind die Anstellwinkel an beiden Enden ungleich null. Im Fall der Fig. 6I sind Seitbewegungen des Unterkiefers nicht möglich. Im Fall der Fig. 6 II und der Fig. 6 III sind solche Seitbewegungen nach Beginn eines Auseinandergleitens der Eingriffselemente möglich.

Fig. 7 zeigt die Eingriffselementepaare A und B gemäß Fig. 6 II bei Anordnung an der je zugehörigen Schiene. Die beiden vorderen Eingriffselemente B sind an den beiden seitlichen Außenseiten des Unterkieferschienenkörpers 31 angeordnet und die hinteren Eingriffselemente A sind an den beiden seitlichen Außenseiten der Oberkieferschienenkörper 30 angeordnet mit ihren jeweiligen Schienenbasen 33 bzw. 32 und beispielhaft für die therapeutische Protrusionsbahn 2 ausgeformt und damit als A2, B2.b2 bezeichnet (.b2 steht für die therapeutische Protrusion des vorderen Eingriffselementes in Position b2). Dadurch ist der Unterkieferschienenkörper 31 mit seiner Basis 33 gegenüber dem Oberkieferschienenkörper 30 mit seiner Basis 32 in eine Protrusion Δ24.VII/b2 gesteuert (die Protrusion entspricht der Inzisalpunktwanderung von Position VII in die Position b2 gemäß Fig. 1), bei welcher die Führungsflächen 101fA2 eines jeden Eingriffselementepaares bei reiner Protrusion ohne Seitverschiebung vollflächig aneinander liegen (101fA2x101fB2). Dies entspricht der Darstellung in Fig. 2 in der Unterkieferstelleung 19.b2 und Fig. 3 IV. Entsprechend der bei der Protrusionsbewegung auftretenden Inzisalpunktverlagerung von 24.a nach 24.b2 erfolgt eine Vorverlagerung der Unterkieferschiene mit ihrer Basis 33, mit entsprechender Vorverlagerung der Unterkieferschiene 31 und der weiteren in Fig. 7 dargestellten Elemente der Unterkieferschiene 31 und der mit ihr verbundenen vorderen Eingriffselemente B2 in die Position B2.b2.

Fig. 8 zeigt die Anordnung gemäß Fig. 7 bei habitueller Unterkieferöffnung entsprechend Fig. 2 mit der Unterkieferstellung 19.a und entsprechend Fig. 3 II, d. h. bei einer Unterkieferöffnung, bei welcher die caudalen Enden der hinteren Eingriffselemente A in eine Anfangsberührung mit den cranialen Ende der vorderen Eingriffselemente B kommen, und damit als A2, B2.a bezeichnet werden.

Im Zusammenhang mit den bisherigen Figuren ist nur eine sagittale Unterkieferprotrusion betrachtet worden. Wie aber weiter oben schon angesprochen wurde, kommt es auch im Schlaf zu seitlichen Mediotrusions- und Laterotrusionsbewegungen, auch bei eingesetzter UPS, sofern diese solche seitlichen Bewegungen nicht behindert, und sind die physiologischen Mediotrusions- und Laterotrusionsbahnen einerseits nicht linear sondern gekrümmt und andererseits voneinander verschieden. Im Zusammenhang mit folgenden Figuren werden Ausbildungen der Führungsflächen der Eingriffselemente betrachtet, welche solche seitlichen Mediotrusions- und Laterotrusionsbewegungen nicht nur nicht behindern sondern gezielt führen. Dafür werden zunächst Schädelfrontansichten mit eingesetzter UPS und geschlossenem Unterkiefer betrachtet, in Fig. 9 ohne Seitbewegung des Unterkiefers in der Unterkieferstellung 19.b2 in Fig. 2 und in Fig. 10 mit Seitbewegung des Unterkiefers nach rechts (wieder vom Patienten aus gesehen), aus dieser Unterkieferstellung 19.b2 heraus..

Bei der in Fig. 9 gezeigten Position 19.b2 des Unterkiefers ohne Seitverschiebung liegt der Inzisalpunkt 24.b2 mittig bezüglich des Oberkiefers 11 und sieht man nur die vorderen Eingriffselemente BR2.b2 und BL2.b2, da diese die Sicht auf die genau dahinter liegenden hinteren Eingriffselemente AR2 und AL2 verhindern. Auch die beiden Kieferkopfzentren 13R.b2 und 13L.b2 sind physiologisch mittig bezüglich des Oberkiefers 11 positioniert.

Wird der Unterkiefer 19 gegenüber dem Oberkiefer 11 seitlich in die in Fig. 10 dargestellte Position bewegt, geschieht dies mit einer Laterotrusionsbewegung IR der rechten Unterkieferseite von der Mundmitte weg und einer damit zwangsweise einhergehenden Mediotrusionsbewegung mL der linken Unterkieferseite in Richtung Mundmitte sowie einer Bewegung des Inzisalpunktes 24 von der Mitte nach lateral..

Da die Laterotrusion und die Mediotrusion auf unterschiedlich gekrümmten Bahnen verlaufen (die linke Unterkieferseite bewegt sich nach unten vorne in Richtung Mitte, die rechte Unterkieferseite von der Mitte weggerichtet nach hinten seitlich), kommt es auf der Mediotrusionsseite zu der dargestellten Spaltbildung 35 zwischen den Schienenbasen 33.b2mL und 32, die im Eckzahn-Prämolarenbereich der linken Kieferhälften am größten ist (gezeigt als schwarzer kreisförmiger Fleck auf weißem Grund). Die Seitverschiebung des Unterkiefers 19.b2mL hat zur Folge, dass auch die vorderen Eingriffselemente in gleicher Richtung gegenüber den hinteren Eingriffselementen verschoben werden, weswegen in Fig. 10 die beiden hinteren Einstellelemente AR2 und AL2 gegenüber den vorderen Einstellelementen BR2.b2mL und BL2.b2IR seitlich etwas vorstehen und daher teilweise sichtbar sind.

Fig. 11 zeigt für ein linkes Kiefergelenk eine sagittale Kiefergelenkkopfzentrumbahn 70 bei sagittaler Unterkieferprotrusion ohne Kieferseitbewegung, dargestellt in einem dreidimensionalen Koordinatenraum und unter Zugrundelegung einer therapeutischen Protrusionsbahn 2 in Fig. 2 in Verbindung mit Fig. 3 II - 3 IV. Ursprung des Koordinatensystems dieses Koordinatenraumes ist die Ruheposition 60L.I des linken Kiefergelenkkopfzentrums bei maximalem Kontakt der Oberkiefer- und Unterkieferzähne. Bei einer von der Ruheposition 60L.I ausgehenden habituellen Unterkieferöffnungsbewegung mit ventral gerichteter sagittaler Kiefergelenkkopfzentrumprotrusion, also ohne Seitbewegung des Unterkiefers, bewegt sich das Kiefergelenkkopfzentrum entlang der sagittalen Kiefergelenkkopfzentrumbahn 70 in die habituelle Unterkieferöffnungsposition 60L.a entsprechend Fig. 3 II, und auf dieser Bahn 70 solange weiter, bis die therapeutische Protrusionsposition b2 gemäß Fig. 3 IV erreicht ist und mit darüber hinaus ausgeübter Unterkieferprotrusion der Endpunkt 60L.max. mit maximaler Protrusion im Punkt VIII in Fig. 2 erreicht ist. Eine therapeutisch sinnvolle Protrusionsposition wird hier beispielsweise wieder bei ca. 70% der maximalen Protrusion angenommen und ist, wie in diesem Beispiel bereits genannt, bei Punkt 60L.b2 erreicht. Der genaue Verlauf der sagittalen Kiefergelenkkopfzentrumbahn 70 ist von Patient zu Patient und von Kieferseite zu Kieferseite verschieden, kann durch patientenindividuelle und kieferseitenindividuelle Messung ermittelt werden und bedingt zusammen mit der therapeutischen Protrusionsbewegung mit Positionsveränderung des Inzisalpunktes 24 den gekrümmten Verlauf der Führungsbahnen 101f und 101 der Eingriffselemente A und B. Da auch die Position 24 des Inzisalpunktes in therapeutisch optimaler Unterkieferprotrusionsposition von Patient zu Patient verschieden ist, ist auch die Position von 60L auf der Kiefergelenkkopfzentrumbahn 70, bei der die optimale Protrusion erreicht ist, patientenabhängig und kann im Rahmen einer Titration (schrittweisen Anpassung) der Protrusion von einem zunächst eingestellten Anfangswert (z. B. b1) bis zu einem für den jeweiligen Patienten optimalen Wert (z. B. über .b2 nach .b3) erreicht werden.

Des Weiteren zeigt Fig. 11 neben der sagittalen Kiefergelenkkopfzentrumbahn 70 Bewegungsbahnen, die durch zusätzliche Seitbewegungen des Unterkiefers bzw. des Inzisalpunktes 24 zustande kommen, nämlich bei sagittaler Unterkieferprotrusion und zusätzlicher Mediotrusionsbewegung des Unterkiefers bzw. des Inzisalpunkts 24. Außerdem sind zwischen solchen Bahnen auftretende Winkel gezeigt.

Wird vom Ruhepunkt 60L.I des Kiefergelenkkopfzentrums ausgehend nicht nur eine reine sagittale Protrusion ausgeführt sondern wird zusätzlich eine maximal mögliche Mediotrusion ausgeführt, also eine zur Mundmitte gerichtete zusätzliche Bewegung des Kiefergelenkkopfzentrums, kommt es zu einer in ventraler, medialer und caudaler Richtung spiralförmig gekrümmten Mediotrusionsbahn 71 (punktiert gezeichnet) des Kiefergelenkkopfzentrums, mit Verlagerung des Kiefergelenkkopfzentrums in einen Auslenkungsort 60L.mLmax. Kommt es erst nach dem Erreichen der therapeutischen Protrusion zu einer zusätzlichen seitlichen Mediotrusionsbewegung gemäß Fig. 9 nach Fig. 10, geschieht das auf einer spiralförmigen Kiefergelenkkopfzentrumbewegungsbahn 72 (gestrichelt gezeichnet), die bei dem Punkt 60L.b2 der sagittalen Kiefergelenkkopfzentrumbahn 70 beginnt (Stellung des linken Kiefergelenkkopfzentrums gemäß Fig. 9) und bei maximaler Mediotrusion in einem Punkt 60L.b2mL auf der Mediotrusionbahn 71 endet (Stellung des linken Kiefergelenkkopfzentrums gemäß Fig. 10). Die Achse der Spiralform ist gegenüber einer craniocaudalen Achse des Koordinatenraums der Fig. 11 nach ventral und medial hin geneigt.

Bei einer Kiefergelenkkopfzentrumbewegung mit Protrusion und Mediotrusion entsprechend der Mediotrusionsbahn 71 kommt es also zu einer Abweichung von der sagittalen Kiefergelenkkopfzentrumbahn 70. Der Grad dieser Abweichung, projiziert auf die durch den Ruhepunkt 60L.I gehende Horizontalebene, wird als Bennett-Winkel (ε) bezeichnet und beträgt bekanntlich ca. 15 - 20 Grad. Die Differenz zwischen der Neigung der sagittalen Kiefergelenkkopfzentrumbahn 70 und der Neigung der Mediotrusionsbahn 71, projiziert auf die durch den Ruhepunkt 60L.I gehende sagittale Ebene, wird als Fischer-Winkel (ζ) bezeichnet und beträgt bekanntlich ca. 5 - 10 Grad.

In Fig. 11 sind Endpunkte der Kiefergelenkkopfzentrumbahn 70 mittels einer strichpunktiert gezeichneten Geraden 80 verbunden, die sich in der durch den Ruhepunkt 60L.i gehenden Sagittalebene erstreckt, und sind auf die Sagittale projizierte Endpunkte der Mediotrusionsbahn 71 mittels einer strichpunktiert gezeichneten Geraden 81 verbunden. Des Weiteren sind auf die durch den Ruhepunkt 60L.I gehende Horizontalebe projizierte Endpunkte der Mediotrusionsbahn 71 mittels einer in dieser Horizontaleben liegenden, strichdoppelpunktiert gezeichneten Geraden 82 verbunden. Der Bennet-Winkel ε bildet den Winkel zwischen der Geraden 82 und der durch den Ruhepunkt 60L.i gehenden Sagittalebene. Der Fischer-Winkel ζ bildet den Winkel zwischen den Geraden 80 und 81.

Die Formen der in Fig. 11 gezeigten Bewegungsbahnen und deren Winkel sind alle abhängig nicht nur von der Physiologie des jeweiligen Patienten sondern auch von der betrachteten Kiefergelenkseite und sind aus patientenindividuellen und kieferseitenindividuellen Messungen zu ermitteln.

Anhand der Figuren 6 bis 8 wurden Ausführungsformen von Eingriffselementen mit Führung in der Sagittalebene erläutert, bei denen die protrusionssteuernden Führungsbahnen der Eingriffselemente entsprechend der bei der therapeutischen Protrusionsbewegung an der Position der Führungsbahnen der Eingriffselemente patientenindividuell messbaren sagittalen Protrusionsbewegungsbahn gestaltet sind, ohne auf physiologische seitliche Kiefer- und Kiefergelenkbewegungen einzugehen. Anhand der folgenden Figuren werden nun Ausführungsformen der Erfindung betrachtet, bei denen die Führungsbahnen der Eingriffselemente zusätzlich auf physiologische Kieferseitbewegungen, insbesondere aus der Kieferstellung bei einer therapeutischen Protrusion heraus, eingehen.

Fig. 12 zeigt - unter Weglassung von Oberkieferschiene und Unterkieferschiene - ein rechtes und ein linkes EingriffselementepaarA, B. Die hinteren Eingriffselemente A weisen einen Hauptkörperteil A und einen diesen Hauptkörperteil A auf seiner lateralen Seite verlängernden Körperteil AA auf. Zur besseren grafischen Unterscheidbarkeit sind die vorderen Eingriffselemente B vollflächig schwarz dargestellt und erscheinen die hinteren Eingriffselemente A als weiß, wobei der verlängernde Körperteil AA vollflächig weiß und der Hauptkörperteil A weiß mit schwarzer Schraffur dargestellt ist, da nur deren Konturen gezeichnet sind. Dargestellt sind Positionen der Eingriffselemente in einer therapeutischen sagittalen Protrusionsposition ohne Seitverschiebung des Unterkiefers,

Anders als bei den in den Figuren 6 bis 8 betrachteten Ausführungsformen der Erfindung sind die zusammenwirkenden Führungsflächen des jeweiligen Eingriffselementepaares nicht nur für die Steuerung in eine sagittale Protrusion gestaltet sondern zusätzlich für eine Mediotrusions- und Laterotrusionssteuerung. Dafür besitzen die Führungsflächen neben die sagittale Protrusion führenden Führungsflächenkomponenten mit Krümmung in cranialer Richtung zusätzlich Führungsflächenkomponenten zur Mediotrusionsführung und Führungsflächenkomponenten zur Laterotrusionsführung. Deshalb liegen die je zueinander weisenden, zusammenwirkenden Führungsflächen der Eingriffselementepaare beim Erreichen der therapeutischen Protrusionsposition nicht vollflächig aneinander sondern berühren sich weitgehend nur an gegenüberliegenden Kanten oder Teilflächen der jeweiligen Eingriffselemente, die an lateralen und cranialen Trennlinien zwischen den Führungsflächen der Eingriffselementen des jeweiligen Eingriffselementepaares gebildet sind. Die laterale Trennlinie 101A, 101B ist in Fig. 12 nur zwischen den beiden rechten Eingriffselementepaaren A, AA und B zusehen und ist auf der linken Seite ansichtsbedingt verdeckt. Die cranialen Trennlinien 103A, 103B der rechten Seite und der linken Seite verlaufen auf einer in Fig. 12 gestrichelt dargestellten cranialen Krümmungsbahn 200. Die lateralen Trennlinien 101B und die cranialen Trennlinien 103B treffen sich je in einem cranialen Endpunkt 104.

In Fig. 12 ist bei der ventralen Führungsfläche des linken hinteren Eingriffselementes A ein vom gegenüberliegenden vorderen Eingriffselement B weg gekrümmter Führungsflächenbereich 106A mit einer medialen Kante 102A dargestellt, welcher für eine Führung einer medialen Seitbewegung des linken vorderen Eingriffselementes B aus der in Fig. 12 dargestellten therapeutischen sagittalen Protrusion heraus in eine in Fig 13 dargestellte Mediotrusion gestaltet ist. Gleichermaßen, wenn auch in Fig 12 nicht zu sehen, sind am hinteren rechten Eingriffselement A ein entsprechender medialer Führungsflächenbereich 106 und eine medialen Kante 102A vorgesehen. Die Krümmung der Führungsflächenbereiche 106 bedingen zwei Anteile: Die Kiefergelenkkopfzentrumbewegung im Kiefergelenk (für die Mediotrusion gemäß 72 in Fig. 11) einerseits und die Unterkieferseitbewegung unter Ober- und Unterkieferschienenkontakt (gemäß 19.b2 Fig 9 nach 19.b2mL Fig. 10) andererseits. Die Krümmung des Führungsflächenbereichs 106 folgt der patientenindividuellen Kiefergelenkkopfzentrumbewegungsbahn 72 in Fig. 11 und der Unterkieferbewegung bei eingesetzter Ober- und Unterkieferschiene, um die Mediotrusion physioligisch richtig zu führen. Aus physiologischer Sicht ist es auch hier wieder vorteilhaft, die Asymmetrie zwischen dem Bewegungsverhalten von rechtem Kiefergelenkkopfzentrum und linkem Kiefergelenkkopfzentrum durch separate Messungen an den die Eingriffselemente aufnehmenden Kieferstellen für die beiden Kieferseiten und durch entsprechende individuelle Gestaltung der rechten und linken Führungsflächenbereiche 106 zu berücksichtigen.

Hier gilt wieder, dass für die Erstellung der Führungsbahnen der Eingriffselemente anstatt direkte Messungen am für die Eingriffselemente beabsichtigten Ort (vollfunktionelle Methode) auch eine Errechnung dieser Führungsbahnen aus bezüglich der Eingriffselemente ortsfern vorgenommenen Messungen (halbfunktionelle Methode) zugrunde gelegt werden kann.

Wie bereits insbesondere im Zusammenhang mit Fig. 10 erläutert, führt eine Mediotrusion auf einer Kieferseite zu einer Laterotrusion auf der anderen Kieferseite. So führt aus einer in Fig. 12 dargestellten sagittalen therapeutischen Protrusion heraus eine Mediotrusion beispielsweise auf der linken Kiefergelenkseite zu einer Laterotrusion auf der rechten Kiefergelenkseite, wie in Fig. 13 dargestellt. Bei Kieferseitbewegungen aus der therapeutischen Protrusion heraus ist durch den Kontakt zwischen Oberkieferschienenbasis 32 und Unterkieferschienenbasis 33 die caudocraniale und craniocaudale Bewegung des Unterkiefers auf der Laterotrusionsseite blockiert, und nur noch eine craniocaudale Bewegung auf der Mediotrusionsseite, bedingt durch die craniomediocaudale Bewegungskomponente des Kiefergelenkkopfzentrums auf der Mediotrusionsseite, möglich. Dies führt zu einer Spaltbildung zwischen Ober- und Unterkieferschiene (35L in Fig. 10).

Aufgrund der die Mediotrusionsbewegung mitbestimmenden Kiefergelenkkopfzentrumbewegungsbahn 72 in Fig. 11 gleitet das Eingriffselement B mit einer entsprechend ventromediocaudal gerichteten spiralförmigen Bewegung entlang dem Führungsflächenbereich 106 des hinteren Eingriffselementes A. Bedingt hierdurch gleitet gleichzeitig das vordere Eingriffselement B auf einem eine Laterotrusion führenden Bereich 105A des hinteren Eingriffselementes A, die auf der ventralen Seite eines das jeweilige hintere Eingriffselement A in lateraler Richtung verlängernden Körperteils AA verläuft.

Da, wie bereits erwähnt, die Mediotrusionsbahn und die Laterotrusionsbahn verschiedene Krümmungen und Steigungen aufweisen, sind auch die mediotrusionsführenden Bereiche und die laterotrusionsführenden Bereiche der Führungsflächen der Eingriffselemente entsprechend unterschiedlich zu gestalten. Und dies wieder patientenindividuell und am Besten auch kieferseitenindividuell (nach halbfunktionell endwertiger Methode oder vollfunktionell endwertiger Methode).

Die Form der Mediotrusions- und Laterotrusionsbahn der Kiefergelenkkopfzentrumbewegung unter Blockierung der caudocranialen Bewegung des Unterkiefers durch die in Kontakt stehenden Ober- und Unterkieferschienenbasen 32 bzw. 33 führt zu der in Fig. 13 dargestellten caudalen Absenkung der cranialen Trennlinien 103 gegenüber der cranialen Krümmungsbahn 200, jedoch auf der mediotrusionsbewegten linken Kieferseite stärker als auf der laterotrusionsbewegten rechten Kieferseite, resultierend in dem bereits im Zusammenhang mit Fig. 10 betrachteten Spalt 35L, der von der linken Kieferseite zur rechten Kieferseite zunimmt.

Würde man bei der Gestaltung der Führungsflächen 106 für eine Zulassung und Führung von Kieferseitbewegungen nur die Mediotrusionbahn berücksichtigen, nicht aber die davon abweichende Laterotrusionbahn, würde das dazu führen, dass zwar eine Mediotrusionsbewegung der einen Kieferseite möglich wäre, nicht aber die damit zwangsläufig einhergehende Laterotrusionsbewegung auf der anderen Kieferseite, was wiederum zur Blockade der Mediotrusionsbewegung der einen Kieferseite führte.

Die beiden in Fig. 14 bei Seitbewegung des protrudierten Unterkiefers dargestellten Eingriffselementepaare sind in dieser Figur im Verbund mit der Oberkieferschiene und ihrer Oberkieferschienenbasis 32 und der dieser gegenüber protrudierten Unterkieferschiene mit ihrer Unterkieferschienenbasis 33.b2 gezeigt. Bei einer Kieferseitbewegung bleiben die an der Oberkieferschiene angebrachten hinteren Eingriffselemente A2R und A2L in unveränderter Position. Infolge der dargestellten Seitbewegung des Unterkiefers wird der linke Teil der linken Unterkieferschienenbasis 33.b2 nach rechts, vorne und unten in die Position 33.b2mL bewegt und nimmt das an ihr angeordnete vordere linke Eingriffselement B2.b2 in eine Mediotrusion mL in die Position B2.b2mL und das an dem linken Teil der rechten Unterkieferschienenbasis angeordnete vordere rechte Eingriffselement B2.b2 in eine Laterotrusion IR in die Position B2.b2IR mit.

Es werden nun anhand von 15 bis 24 noch weitere Betrachtungen zur Formgebung der Eingriffselemente, insbesondere deren Führungsflächen, angestellt.

Fig. 15 zeigt einen Kubus, der entlang einer gekrümmten Trennfläche 106 in zwei Teile geteilt ist, die - beim Erreichen der therapeutischen Protrusion auf der therapeutischen Protrusionsebene b - als vollflächig aneinander liegende linke Eingriffselemente A und B mit den Flächen 106A und 106B der dargestellten Konfiguration in vollflächigem Kontakt zueinander betrachtet werden können (106Ax106B). Die Trennfläche 106 ist gebildet zwischen vier Trennlinien 100, 101, 102 und 103. Die Trennlinie 100 verläuft an dem distocaudalen Ende des Kubuskörpers von einem Endpunkt auf der medialen Kubuskante zu einem Endpunkt auf der lateralen Kubuskante und weist einen Anstellwinkel zu dieser Kubuskante auf, der in Fig. 15 null ist, jedoch auch ungleich null sein kann, wie im Zusammenhang mit den Fig. 6 I bis 6 III erläutert.

Die Trennlinie 103 verläuft zwischen einem Endpunkt 104, einem cranialen Oberkantenpunkt auf der craniolateralen Kubuskante des Körpers B.b, und einem Endpunkt auf der craniomedialen Kubuskante mit einem Winkel zu diesen Kubuskanten. Die Trennlinie 101 verläuft gekrümmt zwischen den lateralen Endpunkten der Trennlinie 100 und dem cranialen Oberpunkt 104. Die Trennlinie 102 verläuft gekrümmt zwischen den medialen Endpunkten der Trennlinien 100 und 103. Die gekrümmte Trennlinie 101 verläuft steiler als die gekrümmte Trennlinie 102. Die genaue Gestaltung der Trennflächen 106 und der sie begrenzenden Trennlinien hängt von der patientenindividuellen Physiologie der Mediotrusionsbewegung des Unterkiefers ab. Dazu gehört auch, dass aufgrund der Seitenasymmetrie der Bewegungsbahnen der beiden Kiefergelenkkopfzentren die Gestaltung der Trennfläche 106 und der sie begrenzenden Trennlinien in vorteilhafter und empfehlenswerter Weise entsprechend kieferseitenindividuellen Messungen bei dem jeweiligen Patienten erfolgt.

Fig. 16 zeigt das Einstellelementepaar gemäß Fig. 15, wobei das vordere Eingriffselement B parallel zu seinen in der Frontalebene verlaufenden Seitenwänden in caudaler Richtung entlang der protrusionssteuernd wirkenden Führungskante 101A des hinteren Eingriffselementes A unter ständigem Kontakt mit dem cranialen Oberpunkt 104 des Eingriffselements B verschoben wird, bis dann unter Aufhebung des flächigen Kontakts zwischen der Führungsfläche 106A des hinteren Eingriffselementes A und der Führungsfläche 106B des vorderen Eingriffselementes B beide Eingriffselemente A und B nur noch in dem cranialen Oberpunkt 104 in Kontakt stehen und sich ansonsten ein Spalt S106 zwischen den Führungsflächen 106A und 106B gebildet hat.

Werden ausgehend von der in Fig. 16 dargestellten relativen Positionierung der beiden Eingriffselemente A und B neben einer Medialbewegung auch Drehbewegungen des vorderen Eingriffselementes B in der Frontal- und Sagittalebene, von ventrocraniomedial gesehen im Uhrzeigersinn, ausgeführt, so kommt es zu der in Fig. 17 gezeigten relativen Positionierung der beiden Eingriffselemente A und B.mL, wobei es unter Schließung des Spaltes S106 wieder zu einem flächigen Aneinanderliegen der beiden Führungsflächen 106A und 106B.bmL kommt, allerdings nicht vollflächig sondern nur bezüglich eines Teils dieser Führungsflächen, mit einem resultierenden Versatz der beiden Eingriffselemente A und B.bmL zueinander.

Fig. 18 zeigt das Eingriffselementepaar A, B.bmL mit der Positionierung gemäß Fig. 17, jedoch mit am vorderen Eingriffselement B.bmL vorgenommenen Reduzierungen, um einerseits eine Laterotrusionsführungsfläche 105B oder eine Laterotrusionsführungskante 105kB für einen laterotrusionssteuernden Eingriff zu formen und andererseits die Form des Körpers des vorderen Eingriffselementes B raumsparend zu gestalten (105rkB), womit die Konfiguration der Führungsflächen und Führungskanten abgeschlossen ist.

Die Form der Laterotrusionsführungsfläche 105B oder der Laterotrusionsführungskante 105kB wird entsprechend einer patientenindividuellen, vorteilhafter Weise auch entsprechend einer kieferseitenindividuellen Messung der Kieferbewegungsbahnen gestaltet (halbfunktionelle endwertige Methode oder vollfunktionelle endwertige Methode). Die Reduzierung besteht in einer Volumenreduktion des dorsocaudomedialen Körperanteils von B, wodurch neue Kanten 102r und 100r des Körpers B entstehen..

Zur Verdeutlichung ist in Fig. 19 das vordere linke Eingriffselement B mit seiner gemäß Fig. 18 erreichten Gestalt nach Drehung um etwa 45° gegen den Gegenuhrzeigersinn (von cranial gesehen) gesondert dargestellt. Die distolaterale Kante 101 stellt das protrusionssteuernd wirksame Element des Eingriffselementes B dar. Der in dorsale Richtung weisende, vollflächig schwarz gezeichnete Flächenteil 106 der Führungsfläche von B dient der Mediotrusionssteuerung. Der kreuzschraffierte Flächenteil 105 bzw. die Kante 105k dient der Laterotrusionssteuerung. Schraffiert ist der gegenüber der Reduktion 105 weiter reduzierte Teil 105r des Eingriffselementes B ohne Steuerungsfunktion.

Bei Fig. 20 handelt es sich um das in Fig. 19 gezeigte Eingriffselement B mit denselben Schraffuren der Flächen, jedoch nach einer 90°-Drehung gegen den Gegenuhrzeigersinn (von cranial gesehen) um eine von caudal nach cranial verlaufende Drehachse. Damit sollen in Fig 19 nicht so gut sichtbare Bereiche besser erkennbar gemacht werden. In seitlicher Draufsicht sieht man die Führungsfläche mit einem schwarz dargestellten Mediotrusionsführungsflächenteil 106 und einem kreuzschraffierten Laterotrusionsführungsflächenteil 105 bzw. 105k, sowie den einfachschraffierten angrenzenden reduzierten Blockteil 105r des Eingriffselementes B ohne Steuerungsfunktion. Der Mediotrusionsführungsflächenteil 106 wird an seiner cranialen Seite von einer der Trennlinie 103 entsprechenden Kante mit dem cranialen Oberpunkt 104, an seiner medialen Seite von einer medialen Kante 105K und auf seiner lateralen Seite von einer lateralen protrusionsführenden Kante 101 begrenzt. Der Laterotrusionsflächenteil 105 hat mit dem Mediotrusionsflächenteil 106 eine gemeinsame Begrenzungskante 105k.

Die Protrusionsführungskante 101 dient der Steuerung des vorderen Eingriffselementes B und damit der es tragenden Unterkieferschiene 31 aus der habituellen Unterkieferöffnung heraus in die therapeutische Protrusionsposition hinein. Die Krümmung der Protrusionsführungskante 101 ist entsprechend der patientenindividuell gemessenen sagittalen Unterkieferprotrusionbahn, bestehend aus der Kiefergelenkkopfzentrumbahn 70 in Fig. 11, und der Unterkieferschließbewegung gemäß 19.a nach 19.b Fig 2 gestaltet, im Hinblick auf die bereits erwähnte Kieferseiten-Asymmetrie vorzugsweise für beide Kieferseiten getrennt entsprechend den für jede Kieferseite individuell gemessenen Protrusionbahnen. Die Krümmung des Mediotrusionsführungsflächenteils 106 entspricht der patientenindividuell, vorzugsweise wieder kieferseitenindividuell gemessenen Unterkiefermediotrusionsbahn, bestehend aus der Kiefergelenkkopfzentrumbahn 72 Fig. 11 und der Unterkiefermediotrusionsbewegung gemäß 19.b2 Fig. 9 nach 19.b2mL Fig 10.

Fig. 21 zeigt ein linkes Eingriffselementepaar mit einem hinteren Eingriffselement A und einem in Laterotrusionsstellung befindlichen vorderen Eingriffselement B.IL. Das Eingriffselement A aus Fig. 15 bis 18 unterscheidet sich von der Ausführungsform des Eingriffselements A gemäß Fig. 21 dadurch, dass letzteres mit einem das Eingriffselement A in lateraler Richtung verlängernden Körperteil AA versehen ist. Dadurch wird eine Laterotrusionsführungsfläche 105A gebildet, über welche der Laterotrusionsführungsflächenteil 105B bzw. die Laterotrusionsführungsflächenkante 105kB bei einer Laterotrusionsbewegung gleitet und besser geführt wird. Ist das verlängernde Körperteil AA nicht vorhanden, gleiten bei der Laterotrusion die Kante 105kB bzw. die Fläche 105B des vorderen Eingriffselementes B über die Kante 101A bzw. Fläche 101fA und werden weniger geführt.

Fig. 22 zeigt eine mögliche Ausprägungsform des vorderen Eingriffselementes B gemäß Fig. 19 , wobei der Eingriffselementekörper weiter reduziert wurde, und zwar dergestalt, dass nur noch zwei dorsale Flächen 106 (schwarz) und 105r (schraffiert) verbleiben und ihre medialen Endpunkte die mediale Begrenzung des Körpers bilden.

Es werden nun noch einige mit Ausführungsformen der Erfindung erreichbare Verbesserungen und deren Vorteile erläutert.

Eine erste Verbesserung erreicht man mittels eines bereits im Zusammenhang mit den Fig. 4 und 5 erläuterten Ausgleichs des sich bei einer bestimmten Protrusion einstellenden keilförmigen Spaltes 34 (Fig. 4 und 5) zwischen den oberen und den unteren Zahnreihen mittels Distanzplättchen E (Fig. 5).

Eine zweite Verbesserung erreicht man durch eine Oberflächenkonditionierung bestimmter Gleitführungsflächen oder Gleitführungskanten zur Veränderung von deren Gleitfähigkeit. Bei den in den Figuren 23, 24, 25 und 31 gezeigten Veränderungen des hinteren Eingriffselementes A bzw. des vorderen Eingriffselementes B sind solche oberflächenkonditionierte Flächen als kreuzschraffierte craniale Anteile der Flächen 106 in Fig. 23, 24, 25 und 31 und Anteile der Flächen 101f, 105, 106 und der Kante 101 markiert, mit -110 hinter diesen Bezeichnungen bezeichnet, und werden hier Retentionselemente genannt. Diese können durch eine lokale Erhöhung des Reibungswiderstandes im kreuzschraffierten Bereich erreicht werden oder durch eine beliebige andere Ausgestaltung. Beispielsweise können sie gebildet sein durch Nuten und/oder Kerben und/oder Aufrauungen und/oder Mulden an den Führungsflächen, solange sie die Positionen, welche die beiden Eingriffselemente eines jeden Eingriffselementepaares bei der therapeutischen Protrusionsposition erreicht haben, in sagittaler Richtung stabilisieren, auch bei einer danach erfolgenden Seitbewegung des Unterkiefers.

Eine dritte Verbesserung erreicht man durch eine Feinjustierbarkeit der Positionen der Körper A und B zueinander durch distanzverändernde Mechanismen gemäß Fig. 25 (Eingriffselement A) und/oder gemäß Fig. 26 (Eingriffselement B). Mit dem Mechanismus gemäß Fig. 25 lässt sich das an der Oberkieferschiene 30 angeordnete hintere Eingriffselement A, hier mit seiner lateralen Erweiterung AA, gemäß dem in dieser Figur gezeigten Doppelpfeil in sagittaler Richtung verschieben. Mit dem Mechanismus gemäß Fig. 26 lässt sich das an der Unterkieferschiene 31 angeordnete vordere Eingriffselement B gemäß dem in dieser Figur gezeigten Doppelpfeil in transversaler Richtung verschieben. Dies kann man beispielsweise dadurch realisieren, dass das Eingriffselement A mittels eines Verstellbarkeit in sagittaler Richtung zulassenden Schraubmechanismus an einem seitlich von der Außenseite der Oberkieferschiene 30 abstehenden Halteelement C befestigt ist und dass das Eingriffselements B mittels eines Verstellbarkeit in transversaler Richtung zulassenden Schraubmechanismus an einem seitlich von der Außenseite der Oberkieferschiene 31 abstehenden Halteelement D befestigt ist. Nicht dargestellt, aber auch möglich, ist eine Verschiebung des hinteren Eingriffselements Ain transversaler Richtung und des vorderen Eingriffselements B in sagittaler Richtung mit entsprechend ausgebildeten Mechanismen. Eine derartige Feinjustierung ist besonders dann von Vorteil, wenn sie therapeutisch geboten ist, so z. B. wenn nach Anfertigung der Bewegungsbahnen auf Basis der patientenindividuellen Messwerte bei krankhaft veränderter Unterkieferbewegung beim Gebrauch der UPS eine Feinjustierung zur Optimierung der Unterkieferbewegung erfolgen soll. Aber auch bei verfahrens- oder herstellungsbedingten Ungenauigkeiten der Bewegungsbahn kann eine Feinjustierung zur Kompensation solcher Ungenauigkeiten dienlich sein.

Eine vierte Verbesserung ist die reversible Befestigung der Eingriffselemente an den Ober- und Unterkieferschienen durch Steck-, Schraub- oder Schiebeverbindungen. Diese sind nützlich, um die für jede der voneinander unterschiedlichen therapeutischen Protrusionseinstellungen und Protrusionsbahnen hergestellten Ober- und Unterkiefereingriffselemente austauschen zu können. Die Schienen werden weiter verwendet, die Eingriffselemente werden ausgetauscht.

Eine fünfte Verbesserung wird durch eine maximale Reduzierung der vorderen Eingriffselemente auf Abtaststifte erreicht, welche dann nur noch in dem cranialen Oberpunkt 104 die Führungsflächen des zugehörigen hinteren Eingriffselements abfahren, wodurch das Austauschen der vorderen Eingriffselemente B bei unterschiedlichen therapeutischen Protrusionen entfällt und entsprechend nur die hinteren Eingriffselemente ausgetauscht werden müssen. Außerdem wird hierdurch die Führung über Führungsflächen möglich, die nicht harmonisch geformt sind, also Wellen bzw. Stufen aufweisen, so wie es oftmals bei Patienten mit Kiefergelenks- oder Unterkieferbewegungsstörungen auftritt. Durch die Reduzierung auf einen Abtaststift wird mehr Platz im Mundraum geschaffen und der Tragekomfort, der wesentlich für eine positive Wirkung der UPS ist, deutlich erhöht.

Bei einer in den Fig. 27 bis 30 dargestellten Ausführungsform der Erfindung sind die vorderen Eingriffselemente B reduziert auf Abtaststifte mit einem möglichen Anstellwinkel ρ der gedachten Strecke zwischen dem cranialen Oberpunkt 104 und der Unterkieferschienenbasis 32. Mit den Abtaststiften werden bei einer Bewegung des (nicht dargestellten) Unterkiefers die Führungsflächen der hinteren Eingriffselemente A abgefahren oder abgetastet.

Fig. 27 I entspricht Fig. 3 II, Fig. 27 II entspricht Fig. 3 III und Fig. 27 III entspricht Fig. 3 IV mit dem Unterschied, dass bei den Ausführungsformen der je zuerst genannten Figur das Eingriffselement B im Vergleich zu den Ausführungsformen der je zuletzt genannten Figur durch Abtaststifte ausgetauscht wurden.

Fig. 28 zeigt eine Situation vergleichbar Fig. 12, wobei in Fig. 28 die vorderen Eingriffselemente B als Abtaststifte ausgeformt und zum Erreichen einer therapeutischen sagittalen Protrusion in die Position B.b2 gesteuert worden sind, indem die Abtaststifte B von der protrusionssteuernden Fläche 101fA geführt wurden.

Es gibt zudem die Möglichkeit, dass die Eingriffselemente A, B und/oder die Distanzplättchen E nicht lösbar mit der Unterkieferprotrusionseinrichtung verbunden sind, sondern fest, und für jede therapeutische Unterkieferprotrusionsposition gesonderte Einrichtungen hergestellt werden. Auch ist es möglich, z.B., die Unterkieferschiene der Unterkieferprotrusionseinrichtung für die unterschiedlichen therapeutischen Protrusionspositionen unverändert zu lassen und nur die Oberkieferschiene der Protrusionseinrichtung für die unterschiedlichen therapeutischen Protrusionspositionen gesondert herzustellen. Dies gelingt insbesondere unter Verwendung des Eingriffselements B der Unterkieferschiene, welches gleich bleibt, wobei dieses das Eingriffselement Ader Oberkieferschiene, welches für die unterschiedlichen therapeutischen Unterkieferprotrusionspositionen unterschiedliche gestaltet wird, in nur einem Punkt 104 berührt (siehe Fig. 28). Auch der umgekehrte Fall, das Eingriffselement A gleich zu behalten und unterschiedliche Eingriffselement B herzustellen, ist denkbar.

Fig. 29 zeigt eine Situation, in welcher nach Erreichen einer in Fig. 28 dargestellten therapeutischen Protrusion eine nach rechts gerichtete seitliche Unterkieferbewegung ausgeführt worden ist, infolge welcher es auf der linken Unterkieferschienenseite zu einer Mediotrusion und auf der rechten Unterkieferschienenseite zu einer Laterotrusion gekommen ist. Während dieser seitlichen Unterkieferbewegung sind der linke Abtaststift B entlang der linken Mediotrusionsführungsfläche 106A in Medianrichtung in Position B.mL und der rechte Abtaststift B entlang der Laterotrusionführungsfläche 105A in Lateralrichtung in Position B.IR geführt worden. Durch eine Reduzierung der Eingriffselemente B auf Abtaststifte ist nicht nur eine Materialeinsparung erreicht worden, sondern auch eine deutliche Verringerung der Reibungswiderstände die bei einer Unterkieferbewegung überwunden werden müssen. Außerdem gibt sich die oben erwähnte fünfte Verbesserung hieraus.

Fig. 30 zeigt die Eingriffselemente, vergleichbar Fig. 14, in einer Ausführungsform mit Abtaststiften und ihren Positionen wie in Fig. 29 und zusätzlich zeigt sie die sie haltende Oberkieferschiene bzw. Unterkieferschiene mit ihren Schienenbasen 32 und 33. Die Oberkieferschienenbasis 32 und die daran angeordneten hinteren Eingriffselemente A2 sind durch schwache Schraffierung hell, die nicht schraffierten lateralen Erweiterungen AA2 weiß, und die Unterkieferschienenbasis 33.b2 und die daran angeordneten vorderen Eingriffselemente B.b2 in Form von Abtaststiften sind dunkel dargestellt. Um in die in Fig. 30 gezeigte Positionierung von Schienen und Eingriffselementen zu gelangen, wurde zunächst aus einer habituellen Unterkieferöffnung heraus die Unterkieferschiene 31 mit ihrer Schienenbasis 33 infolge der abtaststiftförmigen Eingriffselemente B aus der Position B.a gemäß Fig. 8 entlang der protrusionssteuernden Flächen 101fA2 in eine sagittale Protrusion gemäß Fig. 7 in die Position B.b2 verschoben (Fig. 28). Dadurch ist es zu einer Vorverlagerung Δ24.VII/b2 gemäß Fig. 7 der Unterkieferschiene und damit des Unterkiefers gekommen und zu einer Positionierung der abtaststiftförmigen Eingriffselemente B.b2 gemäß Fig. 28. Nach einer aus der sagittalen Protrusionsposition erfolgten seitlichen Unterkieferbewegung nach rechts (wieder vom Patienten aus gesehen) ist es zu einer in den Fig. 29 und 30 dargestellten Mediotrusion mL auf der linken Unterkieferseite und einer Laterotrusion IR auf der rechten Unterkieferseite gekommen, und zwar unter Führung der abtaststiftförmigen Eingriffselemente B entlang der Mediotrusionsführungsfläche 106A Fig. 29 auf der Mediotrusionsseite bzw. entlang der Laterotrusionsführungsfläche 105A Fig. 29 auf der Laterotrusionsseite des Unterkiefers. Da die Mediotrusionssteuerung und die Laterotrusionsteuerung, wie bereits erläutert, aufgrund der Physiologie des Kiefergelenkmechanismus auf gekrümmten Bahnen ablaufen, kommt es zu einer Drehung des Unterkiefers und somit der Unterkieferschienenbasis von der Position 33.b2 in die Position 33.b2mL, was durch eine Drehung um einen Winkel η Fig. 30 angedeutet ist. In dieser Situation sind die Eingriffselemente in den Fig. 29 und 30 dargestellt.

Fig. 31 zeigt eine Ausführungsform eines hinteren rechten Eingriffselementes A mit einem gegenüber den zuvor anhand der Fig. 12 - 14, 23, 25, 28 - 30 betrachteten Eingriffselementen A reduzierten Körper. Der Körper ist auf ein minimales Maß für die Stabilität des Körpers und die Steuerung des Abtaststiftes über die Führungsflächen 101f, 105 und 106 reduziert. Die Reduzierung ist am caudalen und cranialen Teil des die Mediotrusionssteuerfläche 106A tragenden Körperteils des Eingriffselementes A vorgenommen worden. Weiterhin vorhanden sind der lateral verlängernde Körperteil AA (ebenfalls reduziert) mit der laterotrusionsteuernden Fläche 105, die protrusionssteuernde Fläche 101f und ein mittlerer Restbereich der Mediotrusionssteuerfläche 106. Deren kreuzschraffierter cranialer Endbereich ist bei der dargestellten Ausführung mit einem Retentionselement 110A versehen, zur mechanischen Stabilisierung des Abtaststiftes in der therapeutischen Protrusionsstellung. Die weiteren schraffierten Bereiche der Führungsflächen 101f, 105 und 106 kennzeichnen eine durch Mulden oder Rinnen gebildete Oberflächenkonditionierung zur besseren Führung des Abtaststiftes, so dass der das vordere Eingriffselement bildende, in Fig. 32 gezeigte Abtaststift mit seinem cranialen Oberpunkt 104 in den Retentions-Mulden oder -Rinnen des hinteren Eingriffselements gemäß Fig. 31 über die Trusionen (Protrusion bzw. Mediotrusion bzw. Laterotrusion) hinweg stabilisiert verlaufen kann.

Fig. 32 ist eine detailliertere Darstellung des schon in den Fig. 27 - 30 gezeigten abtaststiftförmigen vorderen Eingriffselements B. Dieses besteht in dieser reduzierten Form nur noch aus einem von caudal nach cranial gerichteten Winkeloder Bogenstift (letzterer nicht abgebildet), dessen caudaler Endbereich an der Seite der Unterkieferschiene angeordnet wird, und einem dorsal gerichteten Ansatz mit einem freien abgerundeten Endbereich 104, der sich je nach Unterkieferbewegung und -position auf einer der Führungsflächen bzw. -kanten 101fA, 101A, 105A, 106A befindet und diese in die Unterkieferschiene und damit den Unterkiefer steuernder Weise abtastet.

Betrachtungen, die vorausgehend in Figuren und Beschreibungsteilen für Eingriffselemente nur einer Kieferseite vorgenommen worden sind, gelten gleichermaßen für die Eingriffselemente auf der anderen Kieferseite, gegebenenfalls unter Berücksichtigung einer gespiegelten Form.

Die Herstellung der UPS mit ihren Eingriffselementen und Distanzplättchen erfolgt konventionell in analoger Weise labortechnisch nach analogen Abdrucknahmen mittels Abdrucklöffel und Bissgabeln und fakultativ nach analoger Axiografie (Vermessung der Kiefergelenksbewegungen). Diese analogen Verfahren können teilweise oder vollständig durch digitale Verfahren ersetzt werden. Die digitale Axiografie oder der digitale dynamische Oberflächenvideoscan der Oberkieferzahnreihen und der Unterkieferzahnreihen bei Unterkieferbewegungen können die analoge Axiografie ersetzen, digitale statische Oberflächenfotoscans der Zahnreihen können die analoge Abdrucknahme ersetzen

Oberflächenscans der Zähne können die analoge Abdrucknahme ersetzen, das CAD kann die analoge labortechnische Modellierung der Bauteile ersetzen und das CAM kann die analoge labortechnische Herstellung der Bauteile ganz oder teilweise ersetzen. Die digitale Volumentomographie (DVT), ein dreidimensionales, bildgebendes Röntgentomographie-Verfahren, kann bei der Axiografie und / oder CAD zusätzlich zum Einsatz kommen.

Die therapeutischen Protrusionsbahnen und Protrusionsseitbahnen für die rechte Kieferseite und die linke Kieferseite an einer Stelle entlang des Unterkiefers, die dem Punkt 104 in den Figuren entspricht, werden analog und / oder mittels solcher digitaler Verfahren ermittelt.

Hinsichtlich der dem Fachmann geläufigen analogen Abdrucktechnik mit Bissgabel, auch Bissplatte genannt, wird beispielsweise hingewiesen auf DE 102014102770 A1, wo eine Bissgabel, ein Bissnahme-Set und ein System zur Darstellung eines Gebisses beschrieben sind. Hinsichtlich der dem Fachmann geläufigen digitalen Technik wird beispielsweise hingewiesen auf DE 10218435 A1, wo ein Verfahren und eine Vorrichtung zur 3-dimensionalen Bewegungsanalyse von Zahnoberflächen des Oberkiefers in Relation zum Unterkiefer bestehend aus einem Messsystem zur Bestimmung aller Bewegungsfreiheitsgrade mittels elektronischer Messsensoren beschrieben sind.

Im Rahmen des Einsatzes analoger labortechnischer Methoden können Einzelregistrierungen durch voneinander getrennten Bissplatten für den Ober- und Unterkiefer erfolgen, die einerseits mit Abdruckmaterial mit den Zähnen und andererseits miteinander durch ein an inzisaler Stelle an den Bissplatten angreifendes Gestänge verbunden sind. So können bei im Patientenstuhl sitzenden Patienten und auf den Zähnen fixierten Bissplatten mit an den Bissplatten inzisal angeschraubtem oder gestecktem Gestänge jeweils unterschiedliche Raumpositionen von Ober- und Unterkiefer registriert werden, die im Verlauf der therapeutischen Protrusionsbewegung auftreten. Für jede zu messende Kieferrelation kommt entweder jeweils ein neues Gestänge zum Einsatz oder werden die Winkelgrade an diesem Gestänge notiert, so dass die Bissplatten während der Messungen unterschiedlicher Kieferrelationen nicht entfernt werden müssen, woraus sich Ungenauigkeiten ergeben würden. Nach diesen Messungen mit diesem Gestänge und den Bissplatten, und zwar mit einem Messpunkt in habitueller Unterkieferöffnung, mindestens zwei Messpunkten zwischen habitueller Unterkieferöffnung und therapeutischer Protrusionsposition und mindestens zwei Messpunkten für eine Laterotrusion und Mediotrusion auf jeder der beiden Kieferseiten, und der herkömmlichen Bissnahme in therapeutischer Protrusionsposition werden die Bissplatten von den Zähnen weg auf Gipsmodelle hin aufgesetzt und werden die Kieferrelationen mit den unterschiedlichen Gestängen bzw. entsprechend notierten Winkelgraden mit einem Gestänge auf das Modell vorzugsweise in einen Artikulator übertragen.

Die digitalen Herstellungsverfahren bieten die Möglichkeit der "Schnellen Produktentwicklung" (SPE) oder englisch "Rapid Product Development" (RPD).

Im Rahmen des Einsatzes digitaler Techniken kann zwischen registrierenden (Digitalisierung), designenden (3D-CAD) und herstellenden (CAM) Systemen unterschieden werden. Mit registrierenden Systeme können sowohl Strukturen wie z. B. Zahnkronenoberflächen incl. ihrer Unterschnitte oder Zahnkronenpositionen von Ober- und Unterkiefer zueinander, wie z. B. Zahnkronenflächen im Ober- und Unterkiefer in der therapeutischen Protrusionsposition, als auch Abläufe, wie z. B. die Registrierung der Kiefergelenkkopfzentrumbewegung oder z. B. Unterkieferbewegungen bei der Protrusionsbewegung am Ort der Eingriffselemente, registriert werden. Die digitalen Daten fließen in ein Konstruktionsprogramm zum manuellen und / oder automatischen Design der Bauteile der UPS ein. Der so geschaffenen 3D-CAD Datensatz (z. B. STL Datensatz als Industriestandart) fließt in das generative Fertigungsverfahren, auch additives Verfahren genannt (AM - additive Manufacturing), oder in das subtraktive Fertigungsverfahren ein. Letzteres hat gegenüber ersterem den Nachteil der reduzierten Präzision und der begrenzten Möglichkeit der Herstellung von 3 D-Körpern: die Herstellung von bestimmten Unterschnitten oder Hohlkörpern ist nicht möglich und daher nur wenig für die Herstellung der UPS geeignet. Denn Werkzeuge, die bei der subtraktiven Fertigung einen Materialkörper solange abtragen, bis die endgültige Form erreicht ist, können nur bedingt jede gewünschte 3D-Form herstellen. Mit den Additiven Verfahren hingegen, bei denen ein Substrat mittels Licht punktweise ausgehärtet oder mittels Aufspritzen (jetting) punktweise aufgetragen wird bis die endgültige Form erreicht ist, lässt sich jede nur denkbar mögliche 3D-Form herstellen. Die Präzision der so hergestellten Bauteile ist aber nicht nur von dem jeweiligen generativen Fertigungsverfahren abhängig, sondern auch von den Eigenschaften des bei diesem Fertigungsverfahren angewendeten Ausgangssubstrats.

An dieser Stelle seien nur einige, zukunftsweisende generative Fertigungsverfahren beispielhaft genannt:
Maskenbelichtung durch Projektor (DLP - Digital Light Processing, Texas Instruments in
Dallas, Texas, USA)
NanoParticle Jetting^{™} (NPJ, Xjet in Rehovot, Israel)) http://xjet3d.com/ LCM-Verfahren (Lithoz in Wien, Österreich)
Hot Lithography (Cubicure in Wien, Österreich)

Ein Beispiel eines digitalen und kombiniert digitalen-analogen Arbeitsablaufs bietet z. B. Optisleep der Firma SICAT (https://www.sicat.de/media/wysiwyg/pdfSicat/SiCAT-Air_Broschuere_DE.pdf).

Ein Beispiel der digital/analogen Herstellung einer UPS bietet Narval der Firma Resmed (https://www.resmed.com/de-de/consumer/products/dental-series/big-ideas-in-a-small-device.html).

In den Fig. 7 - 10, 14 und 30 sind die Ober- und Unterkieferschienenbasen jeweils als durchgehende U-förmige Gebilde dargestellt. Für den Patienten angenehmer, weil insbesondere im Zungenspitzenbereich weniger störend, ist eine Ausbildung der Oberkieferschienenbasis und/oder der Unterkieferschienenbasis in der in der DE 102 16 242 C1 des Anmelders näher dargestellten Weise. Dabei weisen die Oberkieferschiene und die Unterkieferschiene je nur auf rechte bzw. linke Backenzähne aufzusetzende Schienenteile aus Kunststoff auf, die im vorderen Zahnbogenbereich je über einen schlanken Verbindungsbügel aus Metall verbunden sind. Ausführungsformen darüber hinaus können auch noch weitere Zähne mit einfassen, so dass dann nur noch im Oberkiefer die ersten Frontzähne oder die ersten und zweiten Frontzähne und /oder im Unterkiefer die ersten beiden Frontzähne oder die ersten und zweiten Frontzähne frei belassen und / oder diese nur in ihren Schneidezahnkanten und / oder in einem Teil ihrer Zahnkronen mit eingefasst sind. Die in DE 102 16 242 C1 für die Ober- und Unterkieferbasen und für die Verbindungsbügel angegebenen Materialien eignen sich auch für die UPS der vorliegenden Erfindung. Des Weiteren eignen sich auch keramische Substrate.

Die Führungsbahnen der Eingriffselemente A, B können durch Führungsflächen, Führungskanten oder beides gebildet werden. In den Patentansprüchen wird meist der Begriff Führungsbahnen verwendet, der beides umfassen soll.

### Bezugszeichenliste

### Bezeichnungen und Unterbezeichnungen

### Hauptbezeichnungen

A oberes, hinteres Eingriffselement
B unteres, vorderes Eingriffselement
C einstellbares Halteelement zwischen Oberkieferschiene und Eingriffselement A
D einstellbares Halteelement zwischen Unterkieferschiene und Eingriffselement B
E Spalt 34 ausfüllende Distanzplättchen

### Seitenbezeichnung

- L: linke Seite
- R: rechte Seite

### Bezeichnungen der Inzisalpunktpositionen 24

I Inzisalpunktposition 24 bei maximalem Kontakt zwischen Ober- und Unterkieferzähnen
II Inzisalpunktposition 24 in retrudierter Zahnkontaktposition
II/III überwiegende Rotationsbewegung des Unterkiefers
III Inzisalpunktposition 24 am Endpunkt der überwiegenden Rotationsbewegung des Unterkiefers
IV Inzisalpunktposition 24 in maximaler Unterkieferöffnung
V Inzisalpunktposition 24 in maximal protrudierter Zahnkontaktposition
VI Inzisalpunktposition 24 in Kopfbissstellung (Schneidezahnkantenkontakt)
VII Inzisalpunktposition 24 im dorsalen Endpunkt der therapeutischen Protrusionsebene b, cranialer Punkt der Strecke a
VIII Inzisalpunktposition 24 im ventralen Endpunkt der therapeutischen Protrusionsebene b, auf der Protrusionsgrenzbahn liegend
IX Inzisalpunktposition 24 im caudalen Endpunkt der habituellen Mundöffnung, auf der Protrusionsgrenzbahn liegend
I/IX Inzisalpunktpositionen 24 bei habitueller Unterkieferöffnung ohne eingesetzte UPS

### Bezeichnungen der Bahn- bzw. Ebenenpositionen des Inzisalpunkts 24

a habituelle Mundöffnungsbahn von 24 bei eingesetzter UPS ohne Eingriffselemente A und B
b therapeutische Protrusionsebene von 24 (zwischen VII und VIII), bei eingesetzter UPS ohne Eingriffselemente A und B
c habituelle Mundöffnungsbahn von 24 in der Verlängerung von a bis zum maximalen Zahnkontakt von Oberkiefer und Unterkiefer im Punkt I

### Bezeichnungen der Protrusionsgleitbahnen des Inzisalpunkts 24

1 Unterkieferprotrusionsgleitbahn 1 von einer habituellen Unterkieferöffnung a in eine therapeutischen Unterkieferprotrusionsposition b1
2 Unterkieferprotrusionsgleitbahn 1 von einer habituellen Unterkieferöffnung a in eine therapeutischen Unterkieferprotrusionsposition b2
3 Unterkieferprotrusionsgleitbahn 1 von einer habituellen Unterkieferöffnung a in eine therapeutischen Unterkieferprotrusionsposition b3

### Bezeichnungen der anatomischen Strukturen

- 10: Schädel
- 11: Oberkiefer
- 11-1: dorsaler Oberkieferbereich
- 11-2: ventraler Oberkieferbereich
- 12: Schädelseitige Begrenzung des Kiefergelenks
- 12-1: dorsaler Punkt von 12
- 12-2: ventraler Punkt von 12
- 13: Rotationszentrum des Kiefergelenkkopfs, Kiefergelenkkopfzentrum
- 14: Kiefergelenkkopf
- 17: Oberkieferzähne
- 18: Unterkieferzähne
- 19: Unterkiefer
- 19-1: hinterer Unterkieferbereich mit seinem linken und rechten aufsteigenden

### Unterkieferast mit rechtem und linken Kiefergelenkkopf

- 19-2: vorderer Teil des Unterkiefers mit dem Unterkieferkinn
- 20: Kiefergelenk
- 21: Frankfurter Horizontale
- 22: Verbindungsstrecke zwischen 13 und 24
- 24: Inzisalpunkt: der mesiale (der Zahnbogenmitte zugewandte Gebissteil-) Berührungspunkt der Zahnkronen der mittig angeordneten Schneidezähne des Unterkiefers, der bei nichtabradiertem Gebiss ca. 1 mm unterhalb der Schneidezahnkanten liegt

### Bezeichnung der UPS

- 29: Oberkieferschiene und Unterkieferschiene (mit Eingriffselementen A und B) Hauptbezeichnungen der beiden Schienen der UPS
- 30: Oberkieferschienenkörper der UPS (ohne Eingriffselemente)
- 30-1: distaler Endpunkt des rechten und linken Oberkieferschienenanteils der UPS mit oder ohne Eingriffselemente
- 30-2: mesialer Endpunkt des Oberkieferschienenanteils der UPS mit oder ohne

### Eingriffselemente

- 31: Unterkieferschienenkörper der UPS (ohne Eingriffselemente)
- 31-1: distaler Anteil des rechten und linken Unterkieferschienenanteils der UPS mit oder ohne Eingriffselemente
- 31-2: mesialer Anteil des Unterkieferschienenanteils der UPS mit oder ohne Eingriffselemente

### Unterbezeichnungen der Hauptbezeichnungen der beiden Schienen der UPS

- 32: Schienenbasis der Oberkieferschiene der UPS
- 33: Schienenbasis der Unterkieferschiene der UPS
- 34: Spalt zwischen 32 und 33
- 35: Spalt zwischen 32 und 33 bei Seitwärtsbewegung des Unterkiefers

### Bezeichnung der Kiefergelenkkopfzentrumpositionen

- 60: Kiefergelenkkopfzentrumposition bei maximalem Kontakt von Ober- und Unterkieferzähnen

### Bezeichnungen der Kiefergelenkkopfzentrumbahnen

- 70: sagittale Kiefergelenkkopfzentrumbahn
- 71: mediale Kiefergelenkkopfzentrumbahn (Mediotrusionsbahn)
- 72: Mediotrusionsbahn aus einer therapeutischen Protrusionsposition heraus

### Bezeichnungen der Neigungsstrecken der Kiefergelenkkopfzentrumbahnen

- 80: Neigungsstrecke der Kiefergelenkkopzentrumbahnen 70 in der Sagittalebene
- 81: Neigungsstrecke der Kiefergelenkkopzentrumbahnen 71 auf die Sagittalebene projiziert
- 82: Neigungsstrecke der Kiefergelenkkopfzentrumbahn 71 auf die Horizontalebene projiziert

### Unterbezeichnungen an den Hauptbezeichnungen A und/oder B

### Trennlinien bzw. Kanten

- 100: dorsocaudale
- 101: laterale
- 102: mesiale
- 103: craniale
- 105k: zwischen 106 und 105 oder zwischen 106 und 105r

### Kontaktpunkt

- 104: cranialer Oberpunkt oder Oberkantenpunkt, lateraler Endpunkt von 103 des Eingriffselements B

### Führungsflächen bzw. Kanten

- 101: protrusionssteuernde/führende Kante
- 101f: protrusionssteuernde/führende Fläche
- 105: laterotrusionssteuernde/führende Fläche
- 105k: laterotrusionssteuernde/führende Kante
- 106: mediotrusionssteuernde/führende Fläche

### Retentionselemente

- 110: Retentionselemente der steuernden Flächen 101f, 105 und 106

### Bezeichnungen der Krümmungsbahnen

- 200: Krümmungsbahn von 103

### Bezeichnungen der Winkel

| | |
|---|---|
| π (pi) | Anstellwinkel der gedachten Strecke zwischen den Endpunkten der sagittalen Führungsfläche 101f bzw. Kante 101 und der Oberkieferschienenbasis 32 bzw. Unterkieferschienenbasis 33 (Fig. 3 II - 3 IV) |
| ρ (rho) | Anstellwinkel des Abtaststifts (Fig. 27) zur Schienenbasis, der zwischen der gedachten Strecke, die zwischen dem cranialen Endpunkt 104 und 100r (Fig. 32) verläuft, und der Unterkieferschienenbasis 32 liegt |
| α (alpha) | Winkel zwischen 13/104 und 22 im Punkt 13 (Fig. 3V) |
| β (beta) | Winkel zwischen 104/24 und 22 im Punkt 24 (Fig. 3V) |
| γ (gamma) | Winkel zwischen 13/104 und 104/24 im Punkt 104 (Fig. 3V) |
| η (eta) | Winkel zwischen der Frontalebene vor und nach Mediotrusion in der Horizontalen gemessen (Fig. 14) |
| ε (epsilon) | Bennet-Winkel (Fig. 11) |
| ζ (zeta) | Fischer-Winkel (Fig. 11) |

### Bezeichnungen der Streckenveränderungen

- Δ (delta): Strecke, die sich bei Positionsveränderung eines Bezugspunktes durch Protrusion ergibt (Fig. 7)

### Bezeichnungen von Zähnen

| | |
|---|---|
| Z15 | Zahn 15 |
| Z16 | Zahn 16 |
| Z45 | Zahn 45 |
| Z46 | Zahn 46 |

### Bezeichnungen Kanten und Flächen

- f: Kante als Fläche ausgeprägt
- k: Fläche als Kante ausgeprägt

### Bezeichnungen von Trusionen

- I: Laterotrusion
- m: Mediotrusion

### Bezeichnungen für Reduktion, Spalt/Kontakt, Radius

- r: Anteil eines Körpers entstanden nach körperlicher Reduktion
- s: Spalt zwischen zwei Flächen, zwei Kanten oder Kantenflächen
- x: Kontakt, gegebenenfalls flächiger Art, zwischen zwei Körpern. Beispiel: A104xB103, 104 von A steht mit 103 B in Kontakt
- rad: Radius um 13

### Bezeichnung von Zeichen

| | |
|---|---|
| (Punkt) | räumliche Position einer Bezeichnung. Vor dem Punkt: Bezeichnung; hinter dem Punkt: Position von 24. Beispiel: 19.b1: Unterkiefer 19 befindet sich in der Position mit dem Inzisalpunkt 24 im Schnittpunkt von b und 1. |
| - (Bindestrich) | vor dem Bindestrich: Bezeichnung; hinter dem Bindestrich 1 dorsaler und 2 mesialer Punkt einer Bezeichnung. Beispiel: 31-1: dorsaler Unterkieferschienenanteil. |
| / (Schrägstrich) | Strecke zwischen zwei Bezeichnungen. Beispiel: VII/VIII = b |

### Bezeichnungsregeln

Die räumlichen Positionen aller Bezeichnungen des Unterkiefers und des Unterkieferanteils der UPS tragen in einer bestimmten Position die räumliche Positionsbezeichnung des Inzisalpunktes 24 in dieser bestimmten Position.
Die Reihenfolge der Schriftweise der Unterbezeichnungen: Unterbezeichnung (s. B. 103), Bezeichnung (beispielsweise B), Seitenbezeichnung (beispielsweise R), . (Punkt), räumliche Position (beispielsweise b3): 103BR.b3: Die obere Trennlinie des Eingriffselements B der rechten Seite befindet sich am Unterkiefer mit der räumlichen Position seines Inzialpunkts im Schnittpunkt der Bahn B mit der Bahn 3.
Unterbezeichnungen können verkürzt geschrieben werden, wenn sie sich in einer Figur auf eine Hauptbezeichnung mit rechts / links und / oder räumlicher Bezeichnung beziehen: z. B. 103B anstatt 103BR.b3.
Die Bezeichnung der Ausformung der Führungsflächen bzw. Kanten der Eingriffselemente werden, entsprechend den zugehörigen Führungsbahnen des Inzisalpunktes den Bezeichnungen A bzw. B nachgestellt. Beispiel: AR2: rechtes Eingriffselement mit der Führungsfläche / Kante entsprechend des Inzisalpunktes 24 mit der Protrusionsgleitbahn 2 .
Die Seitenbezeichnung (R/ L)wird der Bezeichnung nachgestellt. Beispiel: Rechtes Kiefergelenkkopfzentrum: 13R

## Patentansprüche

1. Unterkieferprotrusionseinrichtung für eine therapeutische Unterkieferprotrusion bei einem zu therapierenden Patienten, aufweisend einen auf die Zahnkronen mindestens eines Teils der seitlichen Oberkieferzähne (17) aufsetzbaren, deren Zahnkronen ganz oder teilweise umfassenden Oberkieferschienenkörper (30), einen auf die Zahnkronen mindestens eines Teils der seitlichen Unterkieferzähne (18) aufsetzbaren, deren Zahnkronen ganz oder teilweise umfassende Unterkieferschienenkörper (31), eine an diesen beiden Schienenkörpern (30, 31) angeordnete, den therapeutischen Unterkiefervorschub bewirkende Vorschubeinrichtung mit in den beiden Seitenbereichen, vorzugsweise an den beiden Außenseiten, des Unterkieferschienenkörpers (31) angeordneten vorderen Eingriffselementen (B) und in den beiden Seitenbereichen, vorzugsweise an den beiden Außenseiten, des Oberkieferschienenkörpers (30) angeordneten hinteren Eingriffselementen (A), die auf jeder Kieferseite ein durch Mundschließbewegung in einen protrusionssteuernden Eingriff miteinander bringbares Eingriffselementepaar (A, B) bilden mit vorderseitigen Führungsbahnen (101A, 101fA) an den hinteren Eingriffselementen (A), die protrusionssteuernde Führungsbahnen bilden,
**dadurch gekennzeichnet, dass** die vorderen Eingriffselemente (B) als Abtaststifte ausgebildet sind mit an freien Stiftenden vorgesehenen Abtastspitzen (104) zum Abtasten der Führungsbahnen der je zugehörigen hinteren Eingriffselemente (A).

2. Unterkieferprotrusionseinrichtung nach Anspruch 1,
wobei die hinteren Eingriffselemente (A) mit Seitbewegungen des Unterkiefers (19) führenden gekrümmten Transversalbahnen (105, 105k, 106) versehen sind, die entsprechend den Seitbewegungsbahnen des Unterkiefers gekrümmt sind.

3. Unterkieferprotrusionseinrichtung nach mindestens einem der vorausgehenden Ansprüche,
wobei die Eingriffselemente (A, B) an den Schienenkörpern (30, 31) angeordnet sind, insbesondere in Bereichen entsprechend dem patientenindividuell messbaren Kaudruckzentrum von Oberkiefer (11) und Unterkiefer (19), und vorzugsweise in Bereichen zwischen dem zweiten Prämolaren (Z15, Z45) und dem ersten Molaren (Z16, Z46) angeordnet sind.

4. Unterkieferprotrusionseinrichtung nach mindestens einem der vorausgehenden Ansprüche,
wobei die beiden Eingriffselemente (A, B) jedes Eingriffselementepaars (AR, BR, AL, BL) in der therapeutischen Position mechanisch stabilisiert sind mittels einer an mindestens einem Teil der Führungsbahnen (101, 101fA) der Eingriffselemente vorgesehenen Oberflächenkonditionierung, vorzugsweise mittels Retentionselementen (110) in Form von Nuten und/ oder Kerben und/oder Aufrauungen und/oder Mulden und/oder Rinnen und/oder Beschichtungen an den Führungsbahnen (101, 101fA) der Eingriffselemente (A, B), insbesondere in ihren laterocranialen und cranialen Bereichen.

5. Unterkieferprotrusionseinrichtung nach mindestens einem der vorausgehenden Ansprüche,
wobei zum Ausgleich eines keilförmigen Spaltes (34) mit sich in distaler oder mesialer Richtung ändernder Dicke, der sich bei einer Unterkieferprotrusion zwischen den oberen und den unteren seitlichen Zahnreihen bildet, den Eingriffselementen (A, B) am Oberkieferschienenkörper (30) und/oder am Unterkieferschienenkörper (31) keilförmige Distanzplättchen (E) entsprechend der Dicke des keilförmigen Spaltes (34) mindestens am Ort der Einstellelemente (A, B) zugeordnet sind und vorzugsweise in muldenförmigen Aussparungen in den Schienenkörpern (30, 31) fixiert aufliegen.

6. Unterkieferprotrusionseinrichtung nach mindestens einem der Ansprüche 1 bis 4, gegebenenfals auch nach Anspruch 5,
wobei alle oder ein Teil der Eingriffselemente (A, B) und/oder deren Führungsbahnen (101, 101fA) und/oder gegebenenfalls die Distanzplättchen (E) mit den Schienenkörpern (30, 31) durch eine lösbare Schiebe- oder Steck- oder Schraubverbindung oder durch eine Klebe- oder Laserschweißverbindung verbunden sind.

7. Unterkieferprotrusionseinrichtung nach mindestens einem der Ansprüche 1 bis 4, gegebenenfalls auch nach mindestens einem der Ansprüche 5 und 6, wobei der Oberkieferschienenkörper und/oder der Unterkieferschienenkörper je zwei nur auf rechte bzw. linke Molaren, Prämolaren und Eckzähne oder je zwei nur auf rechte bzw. linke Molaren, Prämolaren, Eckzähne und einen Teil der Ober- und/oder Unterkieferfrontzähne aufsetzbare Schienenseitenteile aufweisen, die im Bereich der oberen und/oder unteren Schneidezähne je mittels eines Metall- und/oder Kunststoff- und/oder Keramikbügels miteinander verbunden sind,
und wobei die Eingriffselemente (A, B) und/oder deren Führungsbahnen (101, 101fA) und/oder gegebenenfalls die Distanzplättchen (E) mit den Metallbügeln verbunden sind über eine Klebe- und/oder Steck- und/oder Schiebe- und/oder Laserschweißverbindung.

8. Unterkieferprotrusionseinrichtung nach mindestens einem der vorausgehenden Ansprüche,
wobei mindestens ein Teil der Eingriffselemente (A, B) mit dem je zugehörigen Schienenkörper (30, 31) mittels einer in dorsaler und/oder ventraler Richtung und/oder mittels einer in lateraler und/oder medialer Richtung und/oder mittels einer in kreisförmiger Richtung verstellbaren Justiereinrichtung (C, D) verbunden ist.

9. Unterkieferprotrusionseinrichtung nach mindestens einem der vorausgehenden Ansprüche,
wobei die Eingriffselemente (A, B) und/oder die Ausformung von deren Führungsbahnen (101, 101f) und/oder deren Positionierung am jeweiligen Schienenkörper (30, 31) und/oder die Distanzplättchen (E) und/oder deren Positionierung an dem jeweiligen Schienenkörper (30, 31) und/oder deren muldenförmige Aussparungen an den Schienenkörpern (30, 31) und/oder die Oberflächenkonditionierungen (110) gestaltet sind mittels CAD und/ oder CAM Technik, insbesondere nach Maßgabe von mittels analoger und/oder digitaler Axiografie und/oder digitaler statischer Oberflächenscans und/oder dynamischen Oberflächenenvideoscans der Zähne und/ oder Schleimhäute, und/oder digitaler Röntgentechniken (OPTG, DVT, CT) und/oder MRT, und/oder DVT und/oder Oberflächenscans der Zähne erhaltener digitaler Daten, oder unter Einsatz von Abdrucktechnik nach Maßgabe von Abdrücken und damit hergestellten Modellen beispielsweise aus Gips, Kunststoff und/oder Metall gestaltet sind, insbesondere unter Einsatz einer Protrusionsbissgabel und/oder einer Mundöffnungsbissgabel.

10. Unterkieferprotrusionseinrichtung nach mindestens einem der vorausgehenden Ansprüche,
wobei bei Patienten mit infolge Kiefergelenkmissbildungen und/oder Kiefergelenkschadens und/oder Unterkieferbewegungsschadens (CMD, craniomandibuläre Dysfunktion) physiologisch abnormalem Bewegungsverlauf der Kiefergelenkbewegung die Führungsbahnen für eine therapeutische Protrusion und/oder eine Kieferseitbewegungsführung nicht entsprechend dem patientenindividuell gemessenen abnormalen Bewegungsverlauf gestaltet sind sondern gemäß einem Bewegungsverlauf, der einem physiologisch korrekten Verlauf entsprechend korrigiert ist.

11. Verfahren zur Herstellung einer Unterkieferprotrusionseinrichtung für eine therapeutische Unterkieferprotrusion bei einem zu therapierenden Patienten, umfassend die Bereitstellung von einem auf die Zahnkronen mindestens eines Teils der seitlichen Oberkieferzähne (17) aufsetzbaren, deren Zahnkronen ganz oder teilweise umfassenden Oberkieferschienenkörper (30), einen auf die Zahnkronen mindestens eines Teils der seitlichen Unterkieferzähne (18) aufsetzbaren, deren Zahnkronen ganz oder teilweise umfassende Unterkieferschienenkörper (31), eine an diesen beiden Schienenkörpern (30, 31) angeordnete, den therapeutischen Unterkiefervorschub bewirkende Vorschubeinrichtung mit in den beiden Seitenbereichen, vorzugsweise an den beiden Außenseiten, des Unterkieferschienenkörpers (31) angeordneten vorderen Eingriffselementen (B) und in den beiden Seitenbereichen, vorzugsweise an den beiden Außenseiten, des Oberkieferschienenkörpers (30) angeordneten hinteren Eingriffselementen (A), die auf jeder Kieferseite ein durch Mundschließbewegung in einen protrusionssteuernden Eingriff miteinander bringbares Eingriffselementepaar (A, B) bilden mit protrusionssteuernde Führungsbahnen bildenden vorderseitigen Führungsbahnen (101A) an den hinteren Eingriffselementen (A), **dadurch gekennzeichnet, dass** die vorderen Eingriffselemente (B) als Abtaststifte ausgebildet sind mit an freien Stiftenden vorgesehenen Abtastspitzen (104) zum Abtasten der Führungsbahnen der je zugehörigen hinteren Eingriffselemente (A), wobei bei dem jeweiligen Patienten an der Position entlang der Unterkiefererstreckung, an welcher die Anordnung der vorderseitigen Führungsbahnen (101A) der hinteren Eingriffselemente (A) beabsichtigt ist, eine in die therapeutische Protrusion führende Protrusionsbahn ermittelt wird und die Führungsbahnen entsprechend dem Verlauf der ermittelten Protrusionsbahn gestaltet werden,
wobei vorzugsweise die Protrusionsbahn für beide Kiefergelenkseiten individuell ermittelt und die Führungsbahnen kieferseitenindividuell gestaltet werden.

12. Verfahren nach Anspruch 11,
wobei der patientenindividuellen Bestimmung der Protrusionsbewegung in die therapeutische Protrusion an der Position der Führungsbahnen (101, 101f) der Eingriffselementepaare (A, B) zugrunde gelegt werden:
- entweder Protrusionsbewegungsmessungen direkt an den Positionen der Führungsbahnen (101, 101f) (vollfunktionelle Methode)
- oder Protrusionsbewegungen an den Positionen der Führungsbahnen (101, 101f), die errechnet sind aus Protrusionsbewegungsmessungen an anderen Positionen des Unterkiefers als denen der Führungsbahnen (101, 101f), wobei diese anderen Positionen ausgewählt sind aus einerseits einer auf mesialer Seite des Eingriffselementepaars am Unterkiefer, vorzugsweise am Inzisalpunkt, befindlichen Position und andererseits einer auf distaler Seite des Eingriffselementepaars am Unterkiefer, vorzugsweise im Kiefergelenkkopfzentrum, befindlichen Position oder aus zwei auf mesialer oder zwei auf distaler Seite des Eingriffselementepaars am Unterkiefer befindlichen Positionen (halbfunktionelle Methode) wobei die Führungsbahnen (101, 101f)
- entweder auf der Grundlage der an den Positionen der Führungsbahnen (101, 101f) gemessenen oder für diese Positionen errechneten Protrusionsbewegungen kieferseitenindividuell gestaltet werden (endwertige Methode)
- oder auf der Grundlage einer auf den kieferseitenindividuellen Messungen oder Berechnungen beruhenden Mittelwertbildung spiegelsymmetrisch gleich gestaltet werden (mittelwertige Methode)
- oder auf der Grundlage einer auf den kieferseitenindividuellen Messungen oder Berechnungen einer Seite beruhenden Bildung spiegelsymmetrisch gleich gestaltet werden (links- oder rechtsseitige Methode).

13. Verfahren nach mindestens einem der Ansprüche 11 bis 12,
wobei für jede der unterschiedlichen therapeutischen Protrusionspositionen mit gesonderten Eingriffselementen (A, B) und/oder Ausformungen von deren Führungsbahnen und/oder deren Positionierungen an der jeweiligen Schiene (29) und/oder mit Distanzplättchen (E) und/oder deren Positionierungen an der jeweiligen Schiene (29) und/oder deren muldenförmige Aussparungen an den Schienen (29) jeweils eine der Oberkieferschiene und Unterkieferschiene hergestellt wird oder eine der Oberkieferschiene oder Unterkieferschiene hierbei unverändert bleibt.

## Claims

1. A mandibular protrusion device for a therapeutic mandibular protrusion in a patient undergoing therapy, comprising a maxillary splint body (30), which can be placed on the tooth crowns of at least part of the lateral maxillary teeth (17) and completely or partially covers the tooth crowns thereof, a mandibular splint body (31), which can be placed on the tooth crowns of at least part of the lateral mandibular teeth (18) and completely or partially covers the tooth crowns thereof, an advancement means, which is arranged on said two splint bodies (30, 31) and causes the therapeutic mandibular advancement, having front engagement elements (B) arranged in the two lateral areas, preferably on the two outer sides, of the mandibular splint body (31), and rear engagement elements (A) arranged in the two lateral areas, preferably on the two outer sides, of the maxillary splint body (30), which, on each jaw side, form a pair of engagement elements (A, B), which can be brought into protrusion-controlling engagement with one another by a mouth closing movement, having front-side guide tracks (101A, 101fA), which form protrusion-controlling guide tracks, on the rear engagement elements (A), **characterized in that** the front engagement elements (B) are designed as scanning pins with scanning tips (104) provided at free pin ends for scanning the guide tracks of the respective associated rear engagement elements (A).

2. The mandibular protrusion device according to claim 1,
wherein the rear engagement elements (A) are provided with curved transversal paths (105, 105k, 106) guiding lateral movements of the mandible (19), which are curved according to the lateral movement paths of the mandible.

3. The mandibular protrusion device according to at least one of the preceding claims,
wherein the engagement elements (A, B) are arranged on the splint bodies (30, 31), in particular in areas in accordance with the chewing pressure center of the maxilla (11) and the mandible (19), which can be measured individually for each patient, and are preferably arranged in areas between the second premolar (Z15, Z45) and the first molar (Z16, Z46).

4. The mandibular protrusion device according to at least one of the preceding claims,
wherein the two engagement elements (A, B) of each pair of engagement elements (AR, BR, AL, BL) are mechanically stabilized in the therapeutic position by means of surface conditioning provided on at least a part of the guide tracks (101, 101fA) of the engagement elements, preferably by means of retention elements (110) in the form of grooves and/or notches and/or roughenings and/or depressions and/or trenches and/or coatings on the guide tracks (101, 101fA) of the engagement elements (A, B), in particular in the laterocranial and cranial areas thereof.

5. The mandibular protrusion device according to at least one of the preceding claims,
wherein, for the compensation of a wedge-shaped gap (34) of changing thickness in the distal or mesial direction, which is formed between the upper and the lower lateral rows of teeth during a mandibular protrusion, wedge-shaped spacer plates (E) in accordance with the thickness of the wedge-shaped gap (34) are associated to the engagement elements (A, B) on the maxillary splint body (30) and/or on the mandibular splint body (31) at least at the location of the adjustment elements (A, B) and preferably rest in depression-shaped recesses in the splint bodies (30, 31) in a fixed manner.

6. The mandibular protrusion device according to at least one of claims 1 to 4, also according to claim 5, if applicable,
wherein all or part of the engagement elements (A, B) and/or the guide tracks (101, 101fA) thereof and/or the spacer plates (E), if applicable, are connected to the splint bodies (30, 31) by means of a detachable sliding or plug or screw connection or by means of an adhesive or laser welded connection.

7. Mandibular protrusion device according to at least one of claims 1 to 4, also according to at least one of claims 5 and 6, if applicable,
wherein the maxillary splint body and/or the mandibular splint body each comprise two splint side parts which can be placed only on right and left molars, premolars and canines, or two splint side parts which can be placed only on right and left molars, premolars, canines and part of the maxillary and/or mandibular front teeth, which are, in the area of the upper and/or lower incisors, each connected to one another by means of a metal and/or plastic and/or ceramic bracket, and
wherein the engagement elements (A, B) and/or the guide tracks (101, 101fA) thereof and/or the spacer plates (E), if applicable, are connected to the metal brackets by means of an adhesive and/or plug and/or sliding and/or laser welded connection.

8. The mandibular protrusion device according to at least one of the preceding claims,
wherein at least part of the engagement elements (A, B) is connected to the respective associated splint body (30, 31) by means of an adjusting device (C, D) which can be adjusted in the dorsal and/or ventral direction, and/or an adjusting device (C, D) which can be adjusted in the lateral and/or medial direction, and/or an adjusting device (C, D) which can be adjusted in the circular direction.

9. The mandibular protrusion device according to at least one of the preceding claims,
wherein the engagement elements (A, B) and/or the shape of the guide tracks (101, 101f) thereof and/or the positioning thereof on the respective splint body (30, 31) and/or the spacer plates (E) and/or the positioning thereof on the respective splint body (30, 31) and/or the depression-shaped recesses on the splint bodies (30, 31) thereof and/or the surface conditionings (110) are designed by means of CAD and/or CAM technology, in particular in accordance with digital data obtained by means of analog and/or digital axiography and/or digital static surface scans and/or dynamic surface video scans of the teeth and/or mucous membranes, and/or digital X-ray techniques (OPTG, DVT, CT) and/or MRT, and/or DVT and/or surface scans of the teeth, or using impression taking techniques in accordance with impressions and models manufactured therewith, for example made of plaster, plastic and/or metal, in particular using a protrusion bite fork and/or a mouth opening bite fork.

10. The mandibular protrusion device according to at least one of the preceding claims,
wherein in patients with a physiologically abnormal course of movement of the temporomandibular joint as a result of temporomandibular joint malformations and/or temporomandibular joint damage and/or a mandibular movement disorder (CMD, craniomandibular dysfunction), the guide tracks for a therapeutic protrusion and/or for guiding a lateral jaw movement are not designed according to the abnormal course of movement measured individually for the patient, but according to a course of movement that is corrected according to a physiologically correct course.

11. A method for manufacturing a mandibular protrusion device for a therapeutic mandibular protrusion in a patient undergoing therapy, comprising the provision of a maxillary splint body (30), which can be placed on the tooth crowns of at least part of the lateral maxillary teeth (17) and completely or partially covers the tooth crowns thereof, a mandibular splint body (31), which can be placed on the tooth crowns of at least part of the lateral mandibular teeth (18) and completely or partially covers the tooth crowns thereof, an advancement means, which is arranged on said two splint bodies (30, 31) and causes the therapeutic mandibular advancement, having front engagement elements (B) arranged in the two lateral areas, preferably on the two outer sides, of the mandibular splint body (31), and rear engagement elements (A) arranged in the two lateral areas, preferably on the two outer sides, of the maxillary splint body (30), which, on each jaw side, form a pair of engagement elements (A, B), which can be brought into protrusion-controlling engagement with one another by a mouth closing movement, having front-side guide tracks (101A), which form protrusion-controlling guide tracks, on the rear engagement elements (A), **characterized in that** the front engagement elements (B) are designed as scanning pins with scanning tips (104) provided at free pin ends for scanning the guide tracks of the respective associated rear engagement elements (A),
wherein, in the respective patient, at the position along the extension of the mandible where the front-side guide tracks (101A) of the rear engagement elements (A) are to be arranged, a protrusion path guiding into the therapeutic protrusion is identified, and the guide tracks are designed in accordance with the course of the identified protrusion path,
wherein the protrusion path is preferably identified individually for both temporomandibular joint sides, and the guide tracks are designed individually for each side of the jaw.

12. The method according to claim 11,
wherein the patient-specific determination of the protrusion movement into the therapeutic protrusion at the position of the guide tracks (101, 101f) of the pairs of engagement elements (A, B) is based on:
- either protrusion movement measurements taken directly at the positions of the guide tracks (101, 101f) (fully functional method)
- or protrusion movements at the positions of the guide tracks (101, 101f) calculated from protrusion movement measurements taken at positions of the mandible other than those of the guide tracks (101, 101f), wherein these other positions are selected from a position located on the mesial side of the pair of engagement elements on the mandible, preferably at the incisal point, on the one hand, and a position located on the distal side of the pair of engagement elements on the mandible, preferably in the mandibular condyle center, on the other hand, or from two positions located on the mesial side or two positions located on the distal side of the pair of engagement elements on the mandible (semi-functional method), wherein the guide tracks (101, 101f) are
- either designed individually for each jaw-side on the basis of the protrusion movements measured at the positions of the guide tracks (101, 101f) or calculated for these positions (final-value method)
- or designed mirror-symmetrically identical on the basis of averaging based on the jaw-side-specific measurements or calculations (mean-value method)
- or designed mirror-symmetrically identical on the basis of a formation based on the jaw side-specific measurements or calculations of one side (left or right side method).

13. The method according to at least one of claims 11 to 12,
wherein for each of the different therapeutic protrusion positions, one of the maxillary splint and of the mandibular splint each is manufactured with separate engagement elements (A, B) and/or shapes of the guide tracks thereof and/or positioning thereof on the respective splint (29) and/or spacer plates (E) and/or positioning thereof on the respective splint (29) and/or depression-shaped recesses thereof on the splints (29), or one of the maxillary splint or the mandibular splint remains unchanged.

## Revendications

1. Orthèse de protrusion mandibulaire pour une protrusion mandibulaire thérapeutique chez un patient qui doit être soumis à une thérapie, qui présente un corps de rail maxillaire (30) qui peut être monté sur les couronnes d'au moins une partie des dents maxillaires supérieures latérales (17), en comprenant en tout ou en partie les couronnes dentaires de celles-ci, un corps de rail mandibulaire (31) qui peut être monté sur les couronnes d'au moins une partie des dents inférieures latérales (18), en comprenant en tout ou en partie les couronnes dentaires de celles-ci, une orthèse d'avance disposée contre ces deux corps de rail (30, 31), mettant en oeuvre l'avance mandibulaire thérapeutique, qui comporte des éléments d'engrènement avant (B) disposés dans les deux zones latérales, de préférence contre les deux côtés externes du corps de rail mandibulaire (31) et des éléments d'engrènement arrière (A) disposés dans les deux zones latérales, de préférence contre les deux côtés externes du corps de rail maxillaire (30), qui, de chaque côté de la mâchoire, forment une paire d'éléments d'engrènement (A, B) pouvant être réunis dans un engrènement qui commande la protrusion par un mouvement de fermeture de la bouche, qui comprennent des glissières de guidage avant (101A, 101fA) contre les éléments d'engrènement arrière (A), qui forment les glissières de guidage qui commandent la protrusion, **caractérisée en ce que** les éléments d'engrènement avant (B) sont réalisés sous la forme de stylets de balayage munis de pointes de balayage (104) prévues aux extrémités libres des stylets pour le balayage des glissières de guidage des éléments d'engrènement arrière (A) respectivement correspondants.

2. Orthèse de protrusion mandibulaire selon la revendication 1, dans laquelle les éléments d'engrènement arrière (A) sont munis de glissières transversales (105, 105k, 106) de forme courbe qui guident les mouvements latéraux de la mâchoire inférieure (19), qui sont courbées en épousant la forme des glissières de mouvements latéraux de la mâchoire inférieure.

3. Orthèse de protrusion mandibulaire selon au moins l'une quelconque des revendications précédentes, dans laquelle les éléments d'engrènement (A, B) sont disposés contre les corps de rail (30, 31), en particulier dans des zones qui correspondent au centre de pression masticatoire de la mâchoire supérieure (11) et de la mâchoire inférieure (19), qui peut être mesuré pour chaque patient individuel, et de préférence sont disposés dans des zones situées entre la deuxième prémolaire (Z15, 245) et la première molaire (Z16, Z46).

4. Orthèse de protrusion mandibulaire selon au moins une des revendications précédentes, dans laquelle, dans laquelle les deux éléments d'engrènement (A, B) de chaque paire d'éléments d'engrènement (AR, BR, AL, BL) sont stabilisés mécaniquement dans la position thérapeutique au moyen d'un conditionnement de surface prévu sur au moins une partie des glissières de guidage (101, 101fA) des éléments d'engrènement, de préférence au moyen d'éléments de rétention (110) sous la forme de rainures et/ ou d'encoches et/ou de rugosités et/ou de creux et/ou de goulottes et/ou de revêtements sur les glissières de guidage (101, 101fA) des éléments d'engrènement (A, B), en particulier dans leurs zones latérocrâniale et crâniale.

5. Orthèse de protrusion mandibulaire selon au moins une des revendications précédentes, dans laquelle, pour compenser un interstice cunéiforme (34) d'épaisseur variable dans la direction distale ou mésiale, qui se forme dans le cas d'une protrusion mandibulaire entre les rangées de dents latérales supérieures et inférieures, des plaquettes d'écartement cunéiformes (E) sont attribuées aux éléments d'engrènement (A, B) sur le corps de rail maxillaire (30) et/ou sur le corps de rail mandibulaire (31) de manière correspondante à l'épaisseur de l'interstice cunéiforme (34), au moins à l'endroit des éléments de réglage (A, B), et de préférence viennent s'appliquer dans des évidements en forme de creux dans les corps de rail (30, 31).

6. Orthèse de protrusion mandibulaire selon au moins une des revendications 1 à 4, le cas échéant également selon la revendication 5, dans laquelle la totalité ou une partie des éléments d'engrènement (A, B) et/ou de leurs glissières de guidage (101, 101fA) et/ou le cas échéant les plaquettes d'écartement (E) sont reliés aux corps de rail (30, 31) par l'intermédiaire d'une liaison amovible par coulissement, par enfichage ou par vissage ou par l'intermédiaire d'une liaison par collage ou par soudage au laser.

7. Orthèse de protrusion mandibulaire selon au moins une des revendications 1 à 4, le cas échéant également selon au moins une des revendications 5 et 6, dans laquelle le corps de rail maxillaire et/ou le corps de rail mandibulaire présente(nt) respectivement deux parties latérales de rails qui peuvent venir se disposer seulement sur des molaires, prémolaires et canines droites, respectivement gauches, ou seulement deux parties latérales de rails qui peuvent venir se disposer seulement sur des molaires, prémolaires et canines droites, et sur une partie des dents avant de la mâchoire supérieure et/ou de la mâchoire inférieure, qui sont reliées entre elles dans la zone des incisives supérieures et/ou inférieures, respectivement au moyen d'un étrier métallique et/ou en matière synthétique et/ou en céramique, et dans laquelle les éléments d'engrènement (A, B) et/ ou leurs glissières de guidage (101, 101fA) et/ou, le cas échéant, les plaquettes d'écartement (E) sont reliés aux étriers métalliques par l'intermédiaire d'une liaison par adhésif et/ou par enfichage et/ou par coulissement et/ou par soudage au laser.

8. Orthèse de protrusion mandibulaire selon au moins une des revendications précédentes, dans laquelle au moins une partie des éléments d'engrènement (A, B) sont reliés au corps de rail respectivement correspondant (30, 31) au moyen d'un mécanisme d'ajustement (C, D) qui peut être réglé dans la direction dorsale et/ou ventrale et/ou au moyen d'un mécanisme d'ajustement (C, D) qui peut être réglé dans la direction latérale et/ou médiale et/ou au moyen d'un mécanisme d'ajustement (C, D) qui peut être réglé dans la direction circulaire.

9. Orthèse de protrusion mandibulaire selon au moins une des revendications précédentes, dans laquelle les éléments d'engrènement (A, B) et/ou la conformation de leurs glissières de guidage (101, 101f) et/ou leur positionnement contre le corps de rail respectif (30, 31) et/ou les plaquettes d'écartement (E) et/ou leur positionnement contre le corps de rail respectif (30, 31) et/ou leurs évidements en forme de creux contre les corps de rail (30, 31) et/ou les conditionnements de surface (110) sont réalisés au moyen de la technique CAD et/ou CAM, en particulier en conformité avec des données numériques que l'on obtient au moyen d'une axiographie analogique et/ou numérique et/ou d'un balayage de surface statique numérique et/ou d'un balayage vidéo de surface dynamique des dents et/ou des muqueuses, et/ou de techniques radiographiques numériques (OPTG, DVT, CT) et/ou d'une MRT, et/ou d'une DVT et/ou de balayages de surface des dents, ou utilisant une technique de moulage en conformité avec des empreintes et des modèles fabriqués avec celles-ci, par exemple en plâtre, en matière plastique et/ou en métal, en particulier en utilisant une fourchette occlusale de protrusion et/ou une fourchette occlusale pour l'ouverture de la bouche.

10. Orthèse de protrusion mandibulaire selon au moins une des revendications précédentes, dans laquelle, chez des patients présentant une trajectoire de mouvement anormale du point de vue physiologique de l'articulation temporo-mandibulaire suite à des malformations de l'articulation temporo-mandibulaire et/ou à des lésions de l'articulation temporo-mandibulaire et/ou à des lésions du mouvement de la mâchoire inférieure (CMD, dysfonctionnement crâniomandibulaire), les glissières de guidage pour une protrusion thérapeutique et/ou un guidage du mouvement latéral de la mâchoire ne sont pas conçues en fonction de la trajectoire anormale du mouvement mesurée individuellement pour chaque patient, mais en conformité avec une trajectoire du mouvement, qui a été corrigée conformément à une trajectoire correcte du point de vue physiologique.

11. Procédé destiné à la fabrication d'une orthèse de protrusion mandibulaire pour une protrusion mandibulaire thérapeutique chez un patient qui doit être soumis à une thérapie, comprenant la mise à disposition d'un corps de rail maxillaire (30) qui peut être monté sur les couronnes d'au moins une partie des dents maxillaires supérieures latérales (17), en comprenant en tout ou en partie les couronnes dentaires de celles-ci, d'un corps de rail mandibulaire (31) qui peut être monté sur les couronnes d'au moins une partie des dents inférieures latérales (18), en comprenant en tout ou en partie les couronnes dentaires de celles-ci, d'une orthèse d'avance disposée contre ces deux corps de rail (30, 31), mettant en oeuvre l'avance mandibulaire thérapeutique, qui comporte des éléments d'engrènement avant (B) disposés dans les deux zones latérales, de préférence contre les deux côtés externes du corps de rail mandibulaire (31) et des éléments d'engrènement arrière (A) disposés dans les deux zones latérales, de préférence contre les deux côtés externes du corps de rail maxillaire (30), qui, de chaque côté de la mâchoire, forment une paire d'éléments d'engrènement (A, B) pouvant être réunis dans un engrènement qui commande la protrusion par un mouvement de fermeture de la bouche, qui comprennent des glissières de guidage avant (101A, 101fA) contre les éléments d'engrènement arrière (A), qui forment les glissières de guidage qui commandent la protrusion, **caractérisé en ce que** les éléments d'engrènement avant (B) sont réalisés sous la forme de stylets de balayage munis de pointes de balayage (104) prévues aux extrémités libres des stylets pour le balayage des glissières de guidage des éléments d'engrènement arrière (A) respectivement correspondants à laquelle la disposition des glissières de guidage avant (101A), dans lequel, chez le patient respectif, à l'endroit situé le long de l'étendue de la mâchoire inférieure, où l'on envisage l'agencement des glissières de guidage avant (101A) des éléments d'engrènement arrière (A), on détermine une glissière de protrusion qui guide la protrusion thérapeutique et on conçoit les glissières de guidage sont conçues de manière correspondante à la trajectoire de la glissière de protrusion déterminée, dans lequel, de préférence, on détermine de manière individuelle la glissière de protrusion pour les deux côtés de l'articulation temporo-mandibulaire et on conçoit les glissières de guidage en fonction des côtés individuels de la mâchoire.

12. Procédé selon la revendication 11, dans lequel, servent de base à la détermination propre au patient de la trajectoire de protrusion dans la protrusion thérapeutique à la position des glissières de guidage (101, 101f) des paires d'éléments d'engrènement (A, B) :
- soit des mesures du mouvement de protrusion effectuées directement aux positions des glissières de guidage (101, 101f) (procédé entièrement fonctionnel) ;
- soit des mouvements de protrusion aux positions des glissières de guidage (101, 101f), qui sont calculés à partir de mesures de mouvement de protrusion à des positions de la mâchoire inférieure autres que celles occupées par les glissières de guidage (101, 101f), dans lesquels ces autres positions sont choisies parmi d'une part une position qui se trouve du côté mésial de la paire d'éléments d'engrènement sur la mâchoire inférieure, de préférence au point incisai, et d'autre part une position qui se trouve du côté distal de la paire d'éléments d'engrènement sur la mâchoire inférieure, de préférence au centre de la tête de l'articulation temporo-mandibulaire ou à partir de deux positions sur le côté mésial ou de deux positions qui se trouvent sur le côté mésial et de deux positions qui se trouvent sur le côté distal de la paire d'éléments d'engrènement sur la mâchoire inférieure (procédé semi-fonctionnel) ;
dans lequel les glissières de guidage (101, 101f)
- soit sont conçues d'une manière individuelle en ce qui concerne les côtés de la mâchoire, sur la base des mouvements de protrusion qui ont été mesurés aux positions des glissières de guidage (101, 101f) ou qui ont été calculés pour ces positions (méthode de finition) ;
- soit sont conçues de manière égale par l'intermédiaire d'une symétrie spéculaire, sur la base d'une formation d'une valeur moyenne qui s'appuie sur les calculs ou sur les mesures qui ont été effectués d'une manière individuelle en ce qui concerne les côtés de la mâchoire, (procédé basé sur la moyenne) ;
- soit sont conçues de manière égale par l'intermédiaire d'une symétrie spéculaire, sur la base d'une élaboration qui s'appuie sur les calculs ou les mesures d'un côté, qui ont été effectués d'une manière individuelle en ce qui concerne les côtés de la mâchoire (procédé basé sur le côté droit ou gauche).

13. Procédé selon au moins une des revendications 11 à 12, dans lequel, pour chacune des différentes positions thérapeutiques de protrusion, qui comprennent des éléments d'engrènement particuliers (A, B) et/ou des conformations particulières de leurs glissières de guidage et/ou de leurs positionnements sur le rail respectif (29) et/ou qui comprennent des plaquettes d'écartement (E) et/ou leurs positionnements sur le rail respectif (29) et/ou leurs évidements en forme de creux sur les rails (29), on fabrique respectivement un des rails maxillaires et des rails mandibulaires ou bien un des rails maxillaires ou des rails mandibulaires reste en l'occurrence inchangé.
